# EUROPÄISCHE PATENTSCHRIFT

(11) **EP 4 422 728 B1**
(45) Veröffentlichungstag und Bekanntmachung des Hinweises auf die Patenterteilung: **14.10.2026**
(21) Anmeldenummer: 22808646.8
(22) Anmeldetag: 18.10.2022
(51) Int. Cl.: A61M 16/20, A61M 16/00, A61B 5/08, A61B 5/00

(54) **VORRICHTUNG ZUR EINSTELLUNG EINES DURCH EINE BEATMUNGSVORRICHTUNG VORGEGEBENEN BEATMUNGSPARAMETERS UND/ODER ZUR AUTOMATISIERTEN DARSTELLUNG VON FÜR MASCHINELLE BEATMUNG RELEVANTER INFORMATION AUF GRUNDLAGE EINES ÖSOPHAGUSBALLONDRUCKS**
DEVICE FOR ADJUSTING A VENTILATION PARAMETER SPECIFIED BY A VENTILATION DEVICE AND/OR FOR AUTOMATICALLY DISPLAYING INFORMATION WHICH IS RELEVANT TO A MECHANICAL VENTILATION ON THE BASIS OF AN ESOPHAGUS BALLOON PRESSURE
DISPOSITIF POUR LE RÉGLAGE D'UN PARAMÈTRE D'ASSISTANCE RESPIRATOIRE PRÉDÉFINI PAR UN DISPOSITIF D'ASSISTANCE RESPIRATOIRE ET/OU POUR LA REPRÉSENTATION AUTOMATISÉE D'INFORMATIONS RELEVANT DE LA RESPIRATION ARTIFICIELLE SUR LA BASE D'UNE PRESSION DE BALLONNET OESOPHAGIEN

(30) Priorität: 29.10.2021 DE 102021128269
(43) Veröffentlichungstag der Anmeldung: 04.09.2024
(73) Patentinhaber: Hamilton Medical AG, 7402 Bonaduz (CH)
(72) Erfinder: SCHWENNINGER, David, 7012 Felsberg (CH)
(74) Vertreter: Schmitt-Nilson Schraud Waibel Wohlfrom Patentanwälte Partnerschaft mbB
(86) Internationale Anmeldenummer: PCT/EP2022/078957
(87) Internationale Veröffentlichungsnummer: WO 2023/072676

(56) Entgegenhaltungen:
- WO-A1-2020/171746
- WO-A1-2022/184471
- DE-A1- 102012 215 662
- US-A1- 2019 231 202
- US-A1- 2021 016 035
- YAN-LIN YANG ET AL: "Optimal esophageal balloon volume for accurate estimation of pleural pressure at end-expiration and end-inspiration: an in vitro bench experiment", INTENSIVE CARE MEDICINE EXPERIMENTAL, BIOMED CENTRAL LTD, LONDON, UK, vol. 5, no. 1, 2 August 2017 (2017-08-02), pages 1 - 12, XP021247648, DOI: 10.1186/S40635-017-0148-Z
- FRANCESCO MOJOLI ET AL: "In vivo calibration of esophageal pressure in the mechanically ventilated patient makes measurements reliable", CRITICAL CARE, vol. 20, no. 1, 11 April 2016 (2016-04-11), XP055524430, DOI: 10.1186/s13054-016-1278-5
- CAMMAROTA GIANMARIA ET AL: "Oesophageal balloon calibration during pressure support ventilation: a proof of concept study", JOURNAL OF CLINICAL MONITORING AND COMPUTING, SPRINGER NETHERLANDS, DORDRECHT, vol. 34, no. 6, 2 December 2019 (2019-12-02), pages 1223 - 1231, XP037266872, ISSN: 1387-1307, [retrieved on 20191202], DOI: 10.1007/S10877-019-00436-3

## Beschreibung

Die vorliegende Erfindung betrifft eine Vorrichtung zur Einstellung eines durch eine Beatmungsvorrichtung vorgegebenen Beatmungsparameters und/oder zur automatisierten Darstellung von für maschinelle Beatmung relevanter Information, auf Grundlage eines Ösophagusballondrucks. Die vorliegende Erfindung betrifft darüber hinaus eine Beatmungsvorrichtung mit Ösophagusballonkatheter mit Ballonsonde zur Bestimmung des Ösophagusballondrucks.

Ösophagusballonkatheter mit Ballonsonde zur Bestimmung eines Ösophagusballondrucks werden insbesondere bei der maschinellen Beatmung eingesetzt, um den transpulmonalen Druck zu ermitteln.

Bei heute gängigen Formen der maschinellen Beatmung wird dem Patienten Atemgas mit Überdruck zugeführt. Deswegen ist bei der Beatmung der Atemwegsdruck bzw. alveoläre Druck zumindest während der Inspirationsphase größer als der Druck in dem die Lungenbläschen bzw. Alveolen umgebenden Pleuraspalt. Während der Exspirationsphase erfolgt keine Druckbeaufschlagung des Atemwegs durch die Beatmungsvorrichtung, mit der Folge, dass das Lungengewebe sich entspannt und der Atemwegsdruck bzw. der alveoläre Druck sinkt. Diese Art der Überdruckbeatmung kann unter bestimmten Umständen dazu führen, dass sich die Druckverhältnisse im Atemweg bzw. in den Alveolen am Ende der Exspirationsphase derart ungünstig einstellen, dass es zu einem Kollaps von Teilen der Alveolen kommt. Dann muss der kollabierte Teil des Lungenvolumens im nachfolgenden Atemzyklus erst wieder von Neuem entfaltet werden. Die funktionelle Residualkapazität der Lunge wird stark beeinträchtigt, so dass die Sauerstoffsättigung abnimmt, und auch das Lungengewebe nimmt dauerhaft Schaden.

Um einen Kollaps von Alveolen am Ende der Exspirationsphase zu verhindern, wird bei der maschinellen Überdruckbeatmung in der Regel ein sog. positiver endexpiratorischer Druck, in der Regel kurz als PEEP bezeichnet, eingestellt. Mit dieser Maßnahme kann in vielen Fällen eine Verbesserung der Sauerstoffsättigung erreicht werden.

Bei Beatmung mit PEEP beaufschlagt die Beatmungsvorrichtung den Atemweg dauerhaft - also sowohl während der Inspirationsphase als auch während der Exspirationsphase - mit einem vorbestimmten Überdruck, dem PEEP. Der PEEP liegt also auch nach dem Ende der Exspirationsphase noch an.

Idealerweise sollte der PEEP genügend groß eingestellt werden, so dass während der Exspirationsphase der alveoläre Druck nicht, oder jedenfalls nur so weit, unterhalb des Druckes im Pleuraspalt liegt, dass das alveoläre Gewebe unter der Wirkung des Drucks im Pleuraspalt nicht kollabiert. Mit anderen Worten: Der PEEP soll verhindern, dass der transpulmonale Druck - das ist die Druckdifferenz zwischen alveolärem Druck und Druck im Pleuraspalt - kleiner wird als Null bzw. einen unteren negativen Grenzwert unterschreitet, ab dem Teile der Alveolen zu kollabieren beginnen.

Andererseits kann sich ein zu hoher Wert des PEEP negativ auswirken, insbesondere während der Inspirationsphase. Denn das Lungengewebe kann bei sehr hohen Atemwegsdrücken während der Inspirationsphase überdehnt werden. Zahlreiche Studien deuten außerdem darauf hin, dass ein hoher Wert des PEEP den Rückfluss von venösem Blut zum Herzen behindern kann mit entsprechend negativen Auswirkungen auf das Herz-Kreislaufsystem.

An sich sollte der PEEP an den jeweils herrschenden transpulmonalen Druck angepasst sein. Der transpulmonale Druck bei einem beatmeten Patienten ist aber einer einfachen Bestimmung nicht zugänglich. Man behilft sich daher mit der Messung des Drucks in einer Ballonsonde, die mittels eines Ösophagusballonkatheters im Ösophagus eines zu beatmenden Patienten platziert wird. Bei geeigneter Positionierung und Konfiguration des Ballons lässt sich der im Ballon gemessene Druck näherungsweise zur Bestimmung des Drucks im Pleuraspalt heranziehen.

Die WO 2014/037175 A1 beschreibt eine automatisierte Einstellung eines durch eine Beatmungsvorrichtung vorgegebenen Drucks, insbesondere des positiven endexpiratorischen Drucks (PEEP) und des maximalen Atemwegsdrucks, auf Grundlage des Ösophagusballondrucks, der als Indikator für den transpulmonalen Druck, also die Druckdifferenz zwischen alveolärem Druck und Druck im Pleuraspalt, angesehen wird.

In der Praxis kann es vorkommen, dass die Beziehung zwischen dem Druck, der in einem in den Ösophagus eingeführten Ballonkatheter gemessen wird, und dem Druck im Pleuraspalt sich während der Beatmung verändert. Die Ursachen hierfür können mannigfaltig sein und sind in aller Regel nicht im Einzelnen ermittelbar.

Mojoli et al., Crit. Care (2016) 20:98 sowie Hotz et al., Respir. Care (2018) 63(2):177-186 empfehlen Prozeduren zur Kalibrierung eines zur Messung des ösophagealen Drucks vorgesehenen Ösophagusballonkatheters mit Ballonsonde. Ziel der Kalibrierung ist es, eine optimale Befüllung der Ballonsonde mit Luft zu erzielen, bei der die Ballonsonde möglichst empfindlich auf Änderungen des auf den Ösophagus einwirkenden Drucks im Pleuraspalt reagiert und den Druck im Pleuraspalt möglichst gut wiedergibt. Die für die Kalibrierung beschriebenen Messprozeduren sind allerdings vergleichsweise aufwändig und empfindlich, so dass in der Praxis, lediglich eine optimale Befüllung für die Ballonsonde vor Beginn einer Beatmung bestimmt und voreingestellt werden kann. Diese optimale Befüllung wird dann während der Beatmung beibehalten und nicht mehr verändert.

Der vorgegebene Beatmungsparameter kann insbesondere ein Druck sein, beispielsweise ein positiver endexpiratorischer Druck (PEEP) und/oder ein maximaler Atemwegsdruck (im Folgenden auch als Paw_max bezeichnet), also typische Beatmungsparameter für gängige Beatmungsmodi, insbesondere für solche Beatmungsmodi, die mit geschlossenen Regelkreisen arbeiten, um eine weitgehend automatisierte Beatmung zu realisieren. Als Beispiele für mit geschlossenen Regelkreisen arbeitende Beatmungsmodi seien die von der Anmelderin entwickelten Modi Adaptive Support Ventilation (ASV) - sowie INTELLiVENT-ASV genannt.

Die erfindungsgemäße Vorrichtung kann zusätzlich oder alternativ auch zur automatisierten Darstellung von für maschinelle Beatmung relevanter Information ausgebildet sein. Solche Information kann insbesondere eine für die Einstellung anderer Beatmungsparameter oder die Wahl eines Beatmungsmodus relevante Größe sein, beispielsweise ein Druck. Solche Informationen bzw. Größen sind oft selbst nicht unmittelbar einer Erfassung zugänglich, können aber mit Hilfe eines Ösophagusballonkatheters ermittelt werden. Ein Beispiel für solch eine Größe wäre etwa DE 10 2012 215 662 A1 offenbart ein System zur automatisierten Einstellung eines durch eine Beatmungseinrichtung vorgegebenen Drucks welches eine Druckermittlungsanordnung zum Ermitteln eines transpulmonalen Drucks am Ende einer Expirationsphase und/oder zum Ermitteln eines transpulmonalen Drucks am Ende einer Inspirationsphase umfasst. Das System umfasst auch eine Einrichtung zur automatisierten Einstellung des durch die Beatmungseinrichtung vorgegebenen Drucks auf Grundlage des ermittelten transpulmonalen Drucks am Ende der Expirationsphase und/oder des ermittelten transpulmonalen Drucks am Ende der Inspirationsphase.

US 2021/016035 A1 beschreibt ein klinisches Entscheidungsunterstützungssystem (CDS) zur Unterstützung bei der Speiseröhrendruckmessung. Das CDS für den Speiseröhrendruck würde den Arzt daran erinnern, bei jedem ersten Einführen des Ballons oder bei jeder drastischen Veränderung einen Okklusionstest durchzuführen. Es könnte den Okklusionstest überwachen und Feedback zur Durchführung und zum Erfolg des Okklusionstests geben. Änderungen beim Patienten oder bei den überwachten Daten können verfolgt werden, indem nach Änderungen beim Ballon-Basisdruck, Änderungen bei der Amplitude der Druckwellenform oder Änderungen beim Muster der Speiseröhrendruck-Wellenform gesucht wird. Durch die Informationen vom Beatmungsgerät kann das System außerdem besser feststellen, wann sich der Speiseröhrendruck unerwartet ändert.

WO 2020/171746 A1 beschreibt ein Verfahren zur automatischen Auswertung eines Füllvolumens eines Ösophagus-Ballonkatheters, der in einen mechanisch beatmeten Patienten eingeführt worden ist. Das Verfahren umfasst das Erhalten von Stichproben eines Atemwegsdrucks Paw und eines Speiseröhrendrucks Pes des Patienten während einer Okklusionsperiode, in der die Atmung des Patienten verhindert wird, das Auswerten des Füllvolumens des Ösophagus-Ballonkatheters durch Bestimmen eines Verhältnisses ΔPes/ΔPaw zwischen Pes und Paw mit Hilfe einer Regressionsanalyse der Pes- und Paw- Stichproben und das Mitteilen eines Ergebnisses der Auswertung an einen Benutzer.

US 2019/231202 A1 offenbart ein System zur Erfassung von Atemanstrengungen eines Patienten, wobei das System eine Druckermittlungsvorrichtung zum Ermitteln eines transpulmonalen Druckes zu dem Zeitpunkt der Atemanstrengung des Patienten umfasst. Die Erfindung betrifft zudem eine Einrichtung zur Erfassung des optimalen Füllvolumens des Ballons des Ösophaguskatheters.

WO 2022/184471 A1 offenbart ein Kalibriersystem zur automatisierten Einstellung einer betriebsgemäßen Befüllung eines in den Ösophagus einführbaren Ösophaguskatheters mit Ballonsonde zur Bestimmung eines ösophagealen Drucks. Das Kalibriersystem umfasst eine Anordnung zum Befüllen der Ballonsonde mit einem Messfluid nach dem Platzieren der Ballonsonde im Ösophagus, einen Drucksensor zum Erfassen des in der Ballonsonde herrschenden ösophagealen Drucks, sowie eine Kalibriersteuerung die derart ausgebildet ist, dass sie die Menge an Messfluid in der Ballonsonde schrittweise verändert. Die Kalibriersteuerung nimmt bei jeder der auf diese Weise schrittweise als Messpunkte eingestellten Mengen an Messfluid in der Ballonsonde einen von dem Drucksensor erfassten ösophagealen Druck auf und ordnet der jeweils eingestellten Menge an Messfluid in der Ballonsonde zu. Die Kalibriersteuerung ist derart ausgebildet, dass sie zum Anfahren der jeweiligen Messpunkte die Menge an Messfluid ausgehend von einem Startwert bis zum Erreichen eines Endwertes in mindestens zwei Schritten in monotoner Weise verändert.

Eine erfindungsgemäße Vorrichtung zur automatisierten Einstellung eines durch eine Beatmungsvorrichtung vorgegebenen Beatmungsparameters, insbesondere eines Drucks, insbesondere eines positiven endexpiratorischen Drucks (PEEP) und/oder eines maximalen Atemwegsdrucks (Paw_max) wird durch Patentanspruch 1 definiert.

Die Patentansprüche 2 bis 9 beschreiben vorteilhafte Ausgestaltungen einer erfindungsgemäßen Vorrichtung.

Ein weiteres Beispiel zur Darstellung von für die Beatmung relevanter Information sind Therapieempfehlungen an Bedienpersonal, insbesondere Therapieempfehlungen, die sich aus von dem Ösophagusballonkatheter gelieferten Informationen ableiten lassen. Die Therapieempfehlungen können im Hinblick auf eine angesichts des erfassten Beatmungszustands im Laufe einer Beatmung empfohlene Änderung oder Neueinstellung von bei der Beatmung angewendeten Beatmungsparametern wie positiver endexpiratorischer Druck (PEEP), maximaler Atemwegsdruck (Paw_max), Tidalvolumen, Tidaldruck, usw. relevant sein. Ggf. können die Therapieempfehlungen auch einen Wechsel des angewendeten Beatmungsmodus umfassen.

Um die Überwachung zu erleichtern, können solche relevanten Größen bzw. Informationen offenbarungsgemäß automatisiert dargestellt werden, falls gewünscht zusammen mit weiteren Informationen, wie Informationen, die angeben, wie zuverlässig die Bestimmung solcher Beatmungsparameter aktuell erfolgen kann.

Die offenbarungsgemäße Vorrichtung weist Folgendes auf:
- ein Druckermittlungssystem zum Ermitteln eines transpulmonalen Drucks, insbesondere eines transpulmonalen Drucks am Ende einer Exspirationsphase und/ oder eines transpulmonalen Drucks am Ende einer Inspirationsphase, auf Grundlage eines in den Ösophagus einführbaren Ösophagusballonkatheters mit Ballonsonde zur Bestimmung eines Ösophagusballondrucks;
- ein Ösophagusballonsteuersystem zur automatisierten Überwachung und/oder Einstellung einer betriebsgemäßen Befüllung der Ballonsonde des Ösophaqusballonkatheters in vivo; und
- eine Beatmungssteuerung.

Die Beatmungssteuerung wird normalerweise zur Steuerung/Regelung der Beatmung vorgesehen sein, insbesondere zur Steuerung/Regelung und/oder Überwachung des Ablaufs der Beatmung in einem jeweils gewählten Beatmungsmodus. ggf. auch zur Auswahl oder zum Wechsel von Beatmungsmodi, beispielsweise im Sinne von weitgehend automatisierten Beatmungsmodi auf Grundlage von geschlossenen Regelkreisen zur laufenden Anpassung wichtiger Beatmungsparameter während der Beatmung, wie es beispielsweise beit der Modi Adaptive Support Ventilation (ASV)-Beatmung und bei der INTELLiVENT-ASV der Fall ist. Die Beatmungssteuerung soll insbesondere zur Einstellung des durch die Beatmungsvorrichtung vorgegebenen Beatmungsparameters und/oder zur Darstellung der für die Beatmung relevanten Information auf Grundlage des durch das Druckermittlungssystem ermittelten transpulmonalen Drucks ausgebildet sein.

Das Druckermittlungssystem ist dazu konfiguriert, den im Ösophagusballon herrschenden Fluiddruck zu erfassen und Signale, die diesen Druck repräsentieren, zur Verfügung zu stellen. Diese Ösophagusballondrucksignale sollen grundsätzlich bei der Beatmung berücksichtigt werden, insbesondere im Hinblick darauf, bestimmte Beatmungsparameter wie den positiven endexpiratorischen Druck (PEEP) und den maximalen Atemwegsdruck (Paw_max) in jeweils an den physiologischen Zustand eines Patienten angepasster Weise einzustellen. Das Druckermittlungssystem ist demgemäß dazu ausgebildet, für den erfassten Ösophagusballondruck charakteristische Ösophagusballondrucksignale für die Beatmungssteuerung zur Verfügung zu stellen. Dementsprechend ist die Beatmungssteuerung dazu konfiguriert, solche Signale von dem Druckermittlungssytem zu empfangen.

Das Druckermittlungssystem bündelt Informationen, die von mindestens einem Sensor, häufig von einer Mehrzahl von unterschiedlichen Sensoren, in der Beatmungsvorrichtung und/oder im Ösophagusballonkatheter geliefert werden. Daher bietet gemäß Ausführungsformen der Erfindung das Druckermittlungssystem auch eine geeignete Grundlage für die Ansteuerung des Ösophagusballonsteuersystems.

Das Druckermittlungssystem kann, jedenfalls zum Teil, der Beatmungssteuerung zugeordnet sein. Das Druckermittlungssystem kann auch, ganz oder zum Teil, dem Ösophagusballonsteuersystem zugeordnet sein. Beispielsweise kann vorgesehen sein, dass die Komponenten des Druckermittlungssystems, die den Druck in der Ballonsonde des Ösophagusballonkatheters erfassen, dem Ösophagusballonsteuersystem zugeordnet sind. Weitere Komponenten, die zur Bestimmung des transpulmonalen Drucks anhand des in der Ballonsonde des Ösopaguskatheters erfassten Ösophagusballondrucks dienen, können dem Ösophagusballonsteuersystem oder der Beatmungssteuerung zugeordnet sein. Das Druckermittlungssystem kann auch als weitgehend eigenständiges System ausgebildet sein, wobei lediglich geeignete Schnittstellen zur Beatmungssteuerung und/oder zum Ösophagusballonsteuersystem vorgesehen sind. Ungeachtet der Zuordnung zur Beatmungssteuerung, dem Ösophagusballonsteuersystem oder als weitgehend eigenständiges System ist es zum Verständnis der vorliegenden Erfindung zweckmäßig, das Druckermittlungssystem als eigenes Teilsystem zu beschreiben.

Offenbarungsgemäß ist vorgesehen, dass die Beatmungssteuerung auch zur automatisierten Ansteuerung des Ösophagusballonsteuersystems ausgebildet ist. Zusätzlich oder alternativ kann vorgesehen sein, dass das Ösophagusballonsteuersystem auch zur automatisierten Ansteuerung der Beatmungssteuerung ausgebildet ist. Das Ösophagusballonsteuersystem kann insbesondere auch dazu ausgebildet sein, die Beatmungssteuerung so anzusteuern, dass die Beatmungssteuerung bestimmte vorgegebenen Manöver ausführt. Solche Manöver können z.B. sogenannte Hold-Manöver umfassen, die es ermöglichen, quasi-statische Werte für den endinspiratorsichen und endexspiratorischen Ösophagusdruck zu messen.

Insbesondere können die Beatmungssteuerung und das Ösophagusballonsteuersystem zu bidirektionaler Interaktion miteinander ausgebildet sein.

Mit der Formulierung, dass die Beatmungssteuerung auch zur automatisierten Ansteuerung des Ösophagusballonsteuersystems ausgebildet ist, soll zum Ausdruck gebracht werden, dass die Beatmungssteuerung neben ihrer bestimmungsgemäßen steuernden/regelnden Funktion hinsichtlich der Beatmungsvorrichtung an sich auch eine steuernde/regelnde Funktion hinsichtlich des Ösophagusballonkatheters, insbesondere für dessen Ballonsonde, aufweist. Dabei kann die Beatmungssteuerung das Ösophagusballonssteuersystem zum Start von vorbestimmten Prozeduren ansteuern, insbesondere zur Befüllung der Ballonsonde mit Messfluid, Kalibrierung der Ballonsonde und/oder laufenden Überwachung eines Füllungszustands der Ballonsonde. Zusätzlich oder alternativ kann die Beatmungssteuerung auf den Ablauf von solchen Prozeduren im Ösophagusballonssteuersystem steuernden/regelnden Einfluss nehmen. Ein solcher steuernder/regelnder Einfluss kann beispielsweise dadurch geschehen, dass die Beatmungssteuerung dem Ösophagusballonsteuersystem Werte von aktuell eingestellten Beatmungsparametern zur Verfügung stellt, auf deren Grundlage das Ösophagusballonsteuersystem wiederum vorbestimmte Steuerprozeduren (wie etwa die oben genannten) für den Ösophagusballon steuert/regelt. Auf diese Weise lässt sich ein optimales Zusammenspiel der beiden Steuersysteme Beatmungssteuerung und Ösophagusballonssteuersystem verwirklichen, so dass die von den beiden Systemen ausgeführten Prozeduren jeweils aufeinander abgestimmt und synchronisiert ablaufen.

Mit der Formulierung, dass das Ösophagusballonsteuersystem auch zur automatisierten Ansteuerung der Beatmungssteuerung ausgebildet ist, soll zum Ausdruck gebracht werden, dass das Ösophagusballonsteuersystem über die gegebenenfalls vorgesehene Übermittlung von Signalen, die den erfassten Ösophagusballondruck repräsentieren, hinaus eine steuernde/regelnde Funktion für auf die Beatmungssteuerung aufweist. Dabei kann das Ösophagusballonssteuersystem die Beatmungssteuerung zum Start von vorbestimmten Prozeduren ansteuern. Zusätzlich oder alternativ kann das Ösophagusballonssteuersystem den Ablauf von bestimmten Prozeduren in der Beatmungssteuerung steuernd/regelnd beeinflussen, beispielsweise dadurch, dass das Ösophagusballonsteuersystem Werte von aktuell eingestellten Beatmungsparametern "einfriert" oder festhält, d.h. für eine bestimmte Zeit verhindert, dass solche Beatmungsparameter durch die Beatmungssteuerung verändert werden. Auch auf diese Weise lässt sich ein optimales Zusammenspiel der beiden Steuersysteme Beatmungssteuerung und Ösophagusballonsteuersystem verwirklichen, so dass die von den beiden Systemen ausgeführten Prozeduren jeweils aufeinander abgestimmt und synchronisiert ablaufen.

Dabei kann die Beatmungssteuerung das Ösophagusballonssteuersystem zum Start von vorbestimmten Steuerprozeduren ansteuern. Beispiele für solche Steuerprozeduren können sein: Überprüfung einer korrekten Befüllung der Ballonsonde des Ösophagusballonkatheters mit Messfluid (z.B. Luft); Neubefüllung der Ballonsonde mit Messfluid, ggs. nach Entleerung derselben; vollständige Kalibrierung der Ballonsonde, um die für eine optimale Befüllung der Ballonsonde erforderliche Menge an Messfluid einzustellen. Zusätzlich oder alternativ kann die Beatmungssteuerung in den Ablauf von vorbestimmten Steuerprozeduren im Ösophagusbal-Ionssteuersystem eingreifen, bzw. die Kontrolle über den Ablauf solcher Steuerprozeduren sogar ganz selbst übernehmen. Auf diese Weise lässt sich erreichen, dass die von den beiden Steuersystemen Beatmungssteuerung und Ösophagusballonssteuersystem ausgeführten Prozeduren jeweils aufeinander abgestimmt und/oder zeitlich koordiniert oder synchronisiert ablaufen. Die Durchführung der genannten Steuerprozeduren für den Ösophagusballonkatheter kann auf diese Weise bei weiterlaufender Beatmung durchgeführt werden. Die Beatmung kann dabei während des Ablaufs einer Steuerprozedur für den Ösophagusballonkatheter in einer solchen Weise durchgeführt werden, dass die Steuerprozedur für den Ösophagusballonkatheter nicht gestört wird. Hierzu kann beispielsweise gehören, dass die Beatmungssteuerung eine Veränderung von bei der Beatmung angewendeten Beatmungsparametern unterdrückt, solange eine Steuerprozedur für den Ösophagusballonkatheter abläuft.

Insbesondere erlaubt die offenbarungsgemäße Vorrichtung eine genauere Berücksichtigung des im Pleuraspalt herrschenden Drucks durch den Ösophagusballonkatheter und flexiblere Anpassung von durch den Ösophagusballonkatheter gelieferten Messwerten auf sich verändernde Umgebungsbedingungen während andauernder Beatmung, insbesondere bei einer weitgehend automatisiert ablaufenden Beatmung. Wenn die zur Beatmungsvorrichtung gehörende Beatmungssteuerung und das zum Ösophagusballonkatheter gehörende Ösophagusballonsteuersystem zu einer bidirektionalen Kommunikation miteinander ausgelegt sind, wird eine dauernde gegenseitige Überwachung der beiden Steuerungen und Anpassung des Gesamtsystems an veränderte Umgebungsbedingungen während laufender Beatmung möglich, ohne dass manuelle Eingriffe erforderlich wären und/ oder die Beatmung unterbrochen werden muss, um bestimmte Funktionen neu zu kalibrieren oder neu zu starten. Insbesondere sind Anpassungen am Füllzustand der Ballonsonde mit Messfluid ohne Unterbrechung der laufenden Beatmung möglich. Die angesprochene Anpassung des Gesamtsystems schließt die Möglichkeit einer vollständigen (Neu-)Kalibrierung des Ösophagusballonkatheters ohne Unterbrechung der Beatmung ein. Dies gilt selbst dann, wenn die Beatmung mittels vollautomatischer Beatmungsmodi erfolgt, beispielsweise bei Beatmung mittels geschlossener Regelkreise, wie bei der von der Anmelderin entwickelten Adaptive Support Ventilation (ASV) und der INTELLiVENT-ASV.

Die Beatmungssteuerung kann auch Informationen zur Verfügung stellen, die für den Ablauf von bestimmten Steuerprozeduren für den Ösophagusballonkatheter von Bedeutung sind. Dies erlaubt es, Veränderungen von Beatmungsparametern oder erfassten Größen, die während der Beatmung auftreten, beim Ablauf von Steuerprozeduren im Ösophagusballonsteuersystem zu berücksichtigen. Als Beispiel sei erwähnt, dass bei manchen Beatmungsmodi die Beatmungssteuerung das im Laufe maschineller Beatmung applizierte Tidalvolumen von Atemzug zu Atemzug neu anpasst bzw. neu einstellt. In solchen Fällen ist das Tidalvolumen kein bei der Beatmung konstanter Beatmungsparameter, sondern eine von Atemzug zu Atemzug veränderliche Größe. Je nach Beatmungsmodus spricht man anstelle von Atemzug auch von Beatmungszug, Atemzyklus oder Beatmungszyklus. Im Folgenden werden die Begriffe Atemzug, Beatmungszug, Atemzyklus und Beatmungszyklus synonym verwendet. Das im jeweiligen Atemzug applizierte Tidalvolumen hat aber einen Einfluss auf andere Größen, etwa den Tidaldruck, der die Druckdifferenz in der Lunge zwischen dem Ende der Inspirationsphase und dem Ende der Exspirationsphase beschreibt. Es kann vorkommen, dass das jeweils applizierte Tidalvolumen auch einen Einfluss nimmt auf Steuerprozeduren im Ösophagusballonsteuersystem. Beispielsweise wird bei in vivo erfolgender Kalibrierung einer einzustellenden Menge an Messfluid in der Ballonsonde des Ösophagusballonkatheters vorausgesetzt, dass sich jedenfalls der Tidaldruck während der Kalibrierung nicht verändert. Damit sollte auch während der Kalibrierung das Atemzug für Atemzug applizierte Tidalvolumen möglichst konstant bleiben. Aus diesem Grund wäre an sich eine Kalibrierung der Ballonsonde des Ösophagusballonkatheters nur möglich in Beatmungsmodi, bei denen das applizierte Tidalvolumen konstant gehalten wird. Die erfindungsgemäß vorgeschlagene Vorrichtung erlaubt es nun, dass die Beatmungssteuerung das Atemzug für Atemzug applizierte Tidalvolumen an das Ösophagusballonsteuersystem übermittelt, so dass die Veränderung des der Ballonsonde jeweils applizierten Tidalvolumens bei der Kalibrierung des Ösophagusballonkatheters berücksichtigt werden kann. Beispielsweise kann dies dadurch geschehen, dass ein jeweils erfasster Wert für die Differenz zwischen dem Ösophagusballondruck am Ende der Inspirationsphase und dem Ösophausballondruck am Ende der Exspirationsphase auf das im jeweiligen Atemzug applizierte Tidalvolumen bezogen wird, etwa durch Bilden eines Quotienten mit dem Tidalvolumen im Nenner. Damit wird eine Kalibrierung der Ballonsonde auch während weiterlaufender Beatmung in Beatmungsmodi mit nicht fest vorgegebenem Tidalvolumen möglich.

Wie bereits angesprochen, kann neben der automatisierten Einstellung von Beatmungsparametern wie dem PEEP oder dem maximalen Atemwegsdruck auch vorgesehen sein, dass die Vorrichtung zur weitgehend automatisierten Darstellung von für die Beatmung relevanter Information ausgebildet ist. Die Darstellung solcher Information kann die Anzeige bestimmter Beatmungsparameter, wie den transpulmonalen Druck, auf einem Display der Beatmungsvorrichtung umfassen. In diesem Fall könnten noch weitere Informationen angezeigt werden, wie etwa die Information, ob der Ösophagusballonkatheter in einem Normalbetrieb arbeitet oder in einem Sondermodus wie einer Kalibrierprozedur oder Befüllungsprozedur.

Besondere Ausgestaltungen der hier vorgeschlagenen Vorrichtung können eines oder mehrere der nachfolgend dargelegten optionalen Merkmale aufweisen. Soweit Merkmale ausschließlich alternative Ausgestaltungen betreffen, ist dies im Folgenden ausdrücklich vermerkt. Es versteht sich daher, dass die nachfolgenden Merkmale in beliebiger Weise mit den jeweils vorangehend oder nachfolgend dargelegten Merkmalen kombinierbar sind, soweit dies nicht ausdrücklich ausgeschlossen ist.

Die Beatmungssteuerung kann zu steuernder/regelnder Interaktion mit dem Ösophagusballonsteuersystem ausgebildet sein, und zwar derart dass die Beatmungssteuerung das Ösophagusballonsteuersystem nach Maßgabe von durch das Druckermittlungssystem gelieferten Druckwerten steuert/regelt. Die Interaktion kann insbesondere bidirektional erfolgen. Dabei kann die Intelligenz des Gesamtsystems weitestgehend bei der Beatmungssteuerung liegen, die alle Steuer/Regelprozeduren durchführt, einschließlich der den Ösophagusballonkatheter betreffenden Steuer/Regelprozeduren wie Kalibrierung des Ösophagusballonkatheters, Überwachen der Fluidmenge im Ösophagusballon, Nachfüllen der Fluidmenge im Ösophagusballon. Das Ösophagusballonsteuersystem ist dann lediglich zum Ausführen von Steuer/Regelbefehlen ausgebildet, die von der Beatmungssteuerung geliefert werden. Bei Ausführungsformen, in denen das Druckermittlungssystem wenigstens teilweise dem Ösopophagusballonsteuersystem zugeordnet ist, kann das Ösophagusballonsteuersystem auch zum Liefern von Signalen ausgebildet sein, die den im Ösophagusballon erfassten Druck wiedergeben.

Es kann auch vorgesehen sein, dass das Ösophagusballonsteuersystem zu steuernder/regelnder Interaktion mit der Beatmungssteuerung ausgebildet ist, und zwar derart, dass das Ösophagusballonsteuersystem nach Maßgabe von durch die Beatmungssteuerung gelieferten Steuerbefehlen Ösophagusballonsteuerprozeduren abarbeitet. Auch diese Interaktion kann bidirektional erfolgen. Dabei kann das Ösophagusballonsteuersystem selbst jedenfalls soweit Intelligenz besitzen, dass es eigenständig Steuer/Regelprozeduren abarbeiten kann, die den Ösophagusballonkatheter betreffen, insbesondere Steuer/Regelprozeduren wie Prozeduren zur Kalibrierung des Ösophagusballonkatheters, Prozeduren zum Überwachen der Fluidmenge im Ösophagusballon oder Prozeduren zum Nachfüllen der Fluidmenge im Ösophagusballon. Die Beatmungssteuerung liefert dann lediglich Befehle zum Aktivieren solcher Prozeduren an das Ösophogasballonsteuersystem, welches diese Prozeduren aber eigenständig abarbeitet, d.h. steuert und/oder regelt. Daneben kann das Osophagusballonsteuersystem bei Ausführungsformen, in denen das Druckermittlungssystem wenigstens teilweise dem Ösopophagusballonsteuersystem zugeordnet ist, auch Signale liefern, die den im Ösophagusballon erfassten Ösophagusballondruck (dieser wird im Folgenden auch kurz als Peso bezeichnet) wiedergeben. Diese Messsignale können bereits weiterverarbeitet sein, beispielsweise so, dass die zur Beatmungssteuerung gesendeten Signale bereits den transpulmonalen Druck wiedergeben.

Zusätzlich oder alternativ zu den vorangehend beschriebenen Ausführungsformen kann das Ösophagusballonsteuersystem dazu ausgebildet sein, vorbestimmte Ösophagusballonsteuersignale an die Beatmungssteuerung zu senden. Dann kann die Beatmungssteuerung derart konfiguriert sein, dass sie vorbestimmte Beatmungsparameter und/oder Beatmungsmodi nach Maßgabe dieser Ösophagusballonsteuersignale einstellt, steuert/regelt und/oder anzeigt. In solchen Ausführungsformen können insbesondere das Ösophagusballonsteuersystem und die Beatmungssteuerung zu bidirektionaler Kommunikation miteinander ausgebildet sein. Mit den hier angesprochenen Ösophagusballonsteuersignalen sollen nicht von dem Ösophagusballon erfasste Druckwerten oder Signale, die repräsentativ für solche Druckwerte sind, gemeint sein. Bei Ösophagusballonsteuersignalen im hier angesprochenen Sinn handelt es sich vielmehr um Steuersignale, die zusätzlich zu dem Ösophagusballondruck zugeordneten Signalen (im Folgenden auch als Ösophagusballondrucksignale bezeichnet) von dem Ösophagusballonssteuersystem erzeugt werden und der Beatmungssteuerung zur Verfügung gestellt werden. Die Beatmungssteuerung nimmt anhand solcher Ösophagusballonsteuersignale steuernden/regelnden Einfluss auf bei der Beatmung ablaufende Beatmungsmodi und/oder nimmt steuernden/regelnden Einfluss auf bestimmte Beatmungsparameter. "Steuernd/regelnd Einfluss nehmen" soll dabei bedeuten, dass die Beatmungssteuerung Veränderungen an Beatmungsparametern herbeiführt oder auch, dass die Beatmungssteuerung solche Veränderungen für bestimmte

Beatmungsparameter jedenfalls für eine gewisse Zeit unterbindet. Dies kann beispielsweise in einer solchen Weise geschehen, dass die Beatmungssteuerung eine Veränderung von Beatmungsmodi unterbindet und/oder eine Veränderung von Beatmungsparametern blockiert, nachdem das Ösophagusballonsteuersystem ein Ösophagusballonsteuersignal gesendet hat, das anzeigt, dass eine Kalibrierprozedur für den Ösophagusballon begonnen worden ist. Ein derartiges "Einfrieren" von Beatmungsmodi bzw. Festhalten von Beatmungsparametern kann wieder aufgehoben werden, nachdem das Ösophagusballonsteuersystem ein (weiteres) Ösophagusballonsteuersignal gesendet hat, dass eine begonnene Kalibrierprozedur beendet worden ist oder abgebrochen worden ist. In gleicher Weise kann die Beatmungssteuerung anhand solcher Ösophagusballonsteuersignale steuernden/ regelnden Einfluss auf die Darstellung von Beatmungsparametern (z.B. auf einem Display der Beatmungsvorrichtung) nehmen, bzw. solche Beatmungsparameter überhaupt darstellen. Beispielsweise kann bei Vorliegen eines kritischen transpulmonalen Drucks oder eines außerhalb der erlaubten Spezifikationen liegenden Füllzustands der Ballonsonde des Ösophagusballonkatheters eine entsprechende Meldung angezeigt werden, ggf. zusammen mit dem erfassten transpulmonalen Druck oder Füllzustand der Ballonsonde. Zusätzlich oder alternativ könnten auch Therapie-Empfehlungen oder sonstige angezeigte Maßnahmen angezeigt werden, etwa wenn der anhand des Ösophagusballons erfasste transpulmonale Druck in einem kritischen Bereich liegt oder ein unzulässiger Füllzustand der Ballonsonde vorliegt. Außerdem ist denkbar, dass eine Warnung auf einem Display der Beatmungsvorrichtung angezeigt wird, sobald das Ösophagusballonsteuersystem ein Ösophagusballonsteuersignal sendet, das anzeigt, dass eine Kalibrierprozedur für den Ösophagusballon gerade aktiviert ist.

Die Beatmungssteuerung kann derart konfiguriert sein, dass sie nach Aktivierung oder Beginn einer Ösophagusballonsteuerprozedur für vorbestimmte Beatmungsparameter und/oder Beatmungsmodi solange keine Veränderung zulässt, bis die Ösophagusballonsteuerprozedur beendet ist. Insbesondere kann nach Maßgabe von Ösophagusballonsteuersignalen der Beatmungssteuerung angezeigt werden, dass die Ösophagusballonsteuerprozedur beendet ist, so dass die Beatmungssteuerung nach Empfang solcher Ösophagusballonsteuersignale wieder in den normalen Modus zurückkehrt.

Zwischen dem Ösophagusballonsteuersystem und der Beatmungssteuerung können auch Daten/Parameter übertragen werden, die nicht unmittelbar mit der Beatmung bzw. der Steuerung automatischer Beatmung in Verbindung stehen. So können z.B. die Umgebungstemperatur und/oder der Umgebungsdruck in einem von dem Ösophagusballonsteuersystem und der Beatmungssteuerung gemessen und an die Beatmungssteuerung bzw. an das Ösophagusballonsteuersystem übertragen werden, so dass ein zur Bestimmung des jeweiligen Parameters ausgebildeter Sensor nur einmal vorhanden sein muss.

Ferner kann die Beatmungssteuerung derart konfiguriert sein, dass sie ab einem vorbestimmten Zeitpunkt vor Aktivierung oder Beginn einer Ösophagusballonsteuerprozedur für vorbestimmte Beatmungsparameter und/oder Beatmungsmodi keine Veränderung zulässt, bis die Ösophagusballonsteuerprozedur beendet ist oder nicht innerhalb einer vorbestimmten Zeit begonnen hat. Dabei kann Beenden sowohl Abschließen einer Ösophagusballonsteuerprozedur als auch Abbrechen einer Ösophagusballonsteuerprozedur bedeuten. Mit dieser Ausgestaltung kann eine Synchronisierung zwischen Beatmungsparameteränderungen und/oder Beatmungsmoduswechseln einerseits und Ösophagusballonsteuerprozeduren (insbesondere Kalibrierprozeduren) andererseits erreicht werden, und zwar durch Warten mit einer jeweiligen Beatmungsparameteränderung und/oder Beatmungsmodusänderung, bis die anstehende Ösophagusballonsteuerprozedur erfolgreich oder nicht erfolgreich beendet worden ist oder nicht innerhalb einer vorbestimmten Wartezeit begonnen hat. Damit kann sichergestellt werden, dass eine jeweilige Ösophagusballonsteuerprozedur unter möglichst gleichbleibenden Beatmungsbedingungen abläuft, wobei dennoch die Beatmung während der Ösophagusballonsteuerprozedur weiterlaufen kann.

In weiteren Ausführungsformen kann die Beatmungssteuerung und/oder das Ösophagusballonsteuersystem derart konfiguriert sein, dass eine Warnung erzeugt wird, wenn ab einem vorbestimmten Zeitpunkt vor Beginn einer Ösophagusballonsteuerprozedur für vorbestimmte Beatmungsparameter und/oder Beatmungsmodi eine Veränderung erzwungen wird, insbesondere durch entsprechende Benutzereingaben. Die Warnung kann mit einer Suspendierung der Veränderung konfiguriert sein, so dass eine weitere Bestätigung in Reaktion auf die Warnung erforderlich ist, damit eine (z.B. durch Benutzereingabe) erzwungene Veränderung des vorbestimmten Beatmungsparameters/Beatmungsmodus wirksam wird. Damit kann erreicht werden, dass Bedienereingaben zumindest nicht ungewollt in Ösophagusballonsteuerprozeduren eingreifen und so deren korrekten Ablauf beeinträchtigen. Das Erzeugen einer solchen Warnung kann bis zum Ende oder Abbruch einer Ösophagusballonsteuerprozedur aktiviert sein.

Ferner kann die Beatmungssteuerung und/oder das Ösophagusballonsteuersystem derart konfiguriert sein, dass eine Ösophagusballonsteuerprozedur zu vorbestimmten Zeitpunkten gestartet wird, wobei dann wenn innerhalb einer vorstimmten Zeitdauer vor Beginn einer Ösophagusballonsteuerprozedur eine Änderung eines vorbestimmten Beatmungsparameters und/oder Beatmungsmodus erfolgen soll, das Ösophagusballonsteuersystem den Beginn der Ösophagusballonsteuerprozedur solange verschiebt, bis die Änderung des Beatmungsparameters und/ oder des Beatmungsmodus abgeschlossen ist. Auch mit dieser Maßnahme kann eine effektive Synchronisierung zwischen Änderung von Beatmungsparametern und/oder Beatmungsmodi bei der Beatmung einerseits, und Ösophagusballonsteuerprozeduren (z.B. Kalibrierprozeduren) andererseits erreicht werden. In diesem Fall erfolgt die Synchronisierung durch Aufschieben des Beginns der Ösophagusballonsteuerprozedur, bis die jeweilige Beatmungsparameteränderung oder der jeweilige Beatmungsmoduswechsel erfolgt ist. Auch auf diese Weise wird erreicht, dass bei weiterlaufender Beatmung während einer jeweiligen Ösophagusballonsteuerprozedur die Ösophagusballonsteuerprozedur dennoch unter gleichbleibenden Bedingungen abläuft, in diesem Fall gemäß dem neuen Beatmungsparameter und/oder dem neuen Beatmungsmodus.

In weiteren Ausführungsformen kann die Beatmungssteuerung und/oder das Ösophagusballonsteuersystem derart konfiguriert sein, dass eine Änderung eines vorbestimmten Beatmungsparameters und/oder Beatmungsmodus, die nach Aktivierung oder nach Beginn einer Ösophagusballonsteuerprozedur erfolgen soll, verschoben wird, bis die Ösophagusballonsteuerprozedur abgeschlossen ist. Es wird gewissermaßen die Priorisierung zwischen Beatmung und Ösophagusballonsteuerprozeduren derart festgelegt, dass jedenfalls nach dem Start einer Ösophogusballonsteuerprozedur bzw. während des Ablaufs einer Ösophagusballonsteuerprozedur die Ösophagusballonsteueruprozedur Priorität gegenüber der Beatmung, insbesondere gegenüber Änderungen von Beatmungsparametern wie PEEP oder Paw_max, oder gegenüber Änderungen des Beatmungsmodus hat. Man kann in diesem Sinne davon sprechen, dass die Beatmungssteuerung jeweils eingestellte Werte für Beatmungsparameter und/oder den Beatmungsmodus "einfriert", solange eine Ösophagusballonsteuerprozedur aktiv ist.

Sofern bestimmte Änderungen von Beatmungsparametern bzw. Beatmungsmodi sofort erfolgen sollen, kann vorgesehen sein, diese auszunehmen und das vorangehend beschriebene Aufschieben von Änderungen nur auf aufschiebbare Beatmungsparameter bzw. Beatmungsmodi anzuwenden.

In weiterer Ausgestaltung kann vorgesehen sein, dass die Beatmungssteuerung und/oder das Ösophagusballonsteuersystem derart konfiguriert ist, dass eine Ösophagusballonsteuerprozedur nach Maßgabe von vorbestimmten Zeitintervallen gestartet wird, wobei dann, wenn innerhalb einer vorbestimmten Zeitdauer vor Beginn einer Ösophagusballonsteuerprozedur eine aufschiebbare Änderung eines vorbestimmten Beatmungsparameters und/oder Beatmungsmodus erfolgen soll, die Beatmungssteuerung den Beginn der Änderung des Beatmungsparameters und/oder des Beatmungsmodus solange verschiebt, bis die Ösophagusballonsteuerprozedur abgeschlossen ist. In dieser Ausgestaltung findet eine Abstimmung zwischen der Beatmungssteuerung und dem Ösophagusballonsteuersystem bereits im Hinblick auf in der Zukunft liegende geplante Ösophagusballonsteuerprozeduren statt. Beispielsweise kann vorgesehen sein, dass das Ösophagusballonsteuersystem bereits eine vorbestimmte Zeitdauer vor dem Beginn einer geplanten Ösophagusballonsteuerprozedur ein Steuersignal an die Beatmungssteuerung sendet, das ankündigt, dass nach Ablauf der vorbestimmten Zeitdauer eine Ösophagusballonsteuerprozedur beginnen wird. In Reaktion auf den Empfang eines solchen Steuersignals kann die Beatmungssteuerung bereits das Einfrieren von Beatmungsparametern und/oder Beatmungsmodi aktivieren (sofort oder falls gewünscht nach Ablauf einer Aktivierungszeit), so dass sichergestellt ist, dass bei Beginn der Ösophagusballonsteuerprozedur stabile Beatmungsverhältnisse vorliegen. Für den Fall, dass Ösophagusballonsteuerprozeduren von der Beatmungssteuerung initiiert werden, ist auch denkbar, dass die Beatmungssteuerung das Einfrieren von Beatmungsparametern und/oder Beatmungsmodi ohne vorherigen Empfang eines entsprechenden Steuersignals von dem Ösophagusballonsteuersystem aktiviert.

Es sei bemerkt, dass das Starten einer Ösophagusballonsteuerprozedur nach Maßgabe von vorbestimmten Zeitintervallen nicht unbedingt regelmäßige oder gar gleichmäßige Zeitintervalle zwischen aufeinander folgenden Ösophagusballonsteuerprozeduren erforderlich macht. Beispielsweise sollen mit dieser Formulierung auch Ausgestaltungen gemeint sein, bei denen Ösophagusballonsteuerprozeduren in unregelmäßigen Zeitabständen aufeinander folgen, solange das Ösophagusballonsteuersystem mit rechtzeitigem Vorlauf vor Beginn einer jeweiligen Ösophagusballonsteuerprozedur ein entsprechendes Steuersignal an die Beatmungssteuerung sendet, das den Beginn der Ösophagusballonsteuerprozedur nach Ablauf einer vorbestimmten Zeitdauer angekündigt. Entsprechendes soll auch für den Fall gelten, dass Ösophagusballonsteuerprozeduren von der Beatmungssteuerung initiiert werden.

In weiteren Ausführungsformen kann das Druckermittlungssystem eine zur Bestimmung des Ösophagusballondrucks ausgelegte Sensorik aufweisen mit einem in den Ösophagus einführbaren Ösophagusballonkatheter mit Ballonsonde und einer Vorrichtung zum Erfassen eines Drucks in der Ballonsonde in vivo. Das Druckermittlungssystem kann insbesondere eine zur Bestimmung eines alveolären Drucks ausgelegte Sensorik aufweisen und den jeweiligen transpulmonalen Druck anhand einer Differenz zwischen dem jeweiligen alveolären Druck und dem jeweiligem Ösophagusballondruck ermitteln. Die Sensorik zur Bestimmung des alveolären Drucks kann zur Bestimmung des Atemwegswiderstands ausgelegt sein und darüber hinaus einen Sensor zum Erfassen des Gasflusses im Atemweg umfassen, insbesondere am Ende einer Exspirationsphase und/oder am Ende einer Inspirationsphase. Dann kann die Sensorik den jeweiligen alveolären Druck anhand des jeweiligen Gasflusses sowie des Atemwegswiderstands bestimmen. Die Sensorik zur Bestimmung des alveolären Drucks kann beispielsweise einen an einem Tubusanfang der Beatmungsvorrichtung angeordneten oder einem Atemwegeingangsventil der Beatmungsvorrichtung zugeordneten Atemwegsdrucksensor zur Erfassung des eingangsseitigen Atemwegsdrucks umfassen.

In weiterer Ausgestaltung kann das Ösophagusballonsteuersystem eine Anordnung zum Befüllen der Ballonsonde mit einem Messfluid und/oder zum Entnehmen von Messfluid aus der Ballonsonde nach Platzieren der Ballonsonde im Ösophagus umfassen. Eine solche Anordnung kann eine Pumpeneinheit aufweisen, mittels derer Messfluid in die Ballonsonde gepumpt werden kann. Falls gewünscht, kann mittels der Pumpeneinheit auch Messfluid aus der Ballonsonde abgepumpt werden. Auf diese Weise lässt sich eine schnelle und präzise Befüllung der Ballonsonde mit einer gewünschten Menge an Messfluid und/oder Entnahme einer gewünschten Menge an Messfluid aus der Ballonsonde realisieren. Es ist insbesondere möglich, die Ballonsonde auf einen Unterdruck gegenüber dem Umgebungsdruck abzupumpen. Es ist allerdings auch denkbar, die Entnahme von Messfluid aus der Ballonsonde ohne Unterstützung durch eine Pumpeneinheit vorzunehmen, sofern die Ballonsonde gegenüber ihrer Umgebung unter Überdruck steht. In solchen Fällen wird man eine Vorrichtung zur Bestimmung des aus der Ballonsonde strömenden Flusses an Messfluid vorsehen, wie auch in Fällen, in denen dieser Messfluidfluss nicht schon durch die Ansteuerung der Pumpeneinheit hinreichend gut bestimmbar ist.

In weiterer Ausgestaltung kann vorgesehen sein, dass das Einleiten und/oder Entnehmen von Messfluid aus der Ballonsonde schrittweise erfolgt. Das Ösophagusballonsteuersystem wird hierzu zum schrittweisen Verändern einer jeweils eingestellten Menge von Messfluid in der Ballonsonde ausgebildet sein, insbesondere derart, dass es die Menge an Messfluid in der Ballonsonde in einem Schritt oder in mehreren Schritten verändert, bei jeder der auf diese Weise schrittweise als Messpunkte eingestellten Mengen an Messfluid in der Ballonsonde einen Ösophagusballondruck erfasst und den erfassten Ösophagusballondruck der jeweils eingestellten Menge an Messfluid in der Ballonsonde zuordnet. Es lässt sich auf diese Weise bei jedem Einleitevorgang von Messfluid in die Ballonsonde und/oder Entnahmevorgang von Messfluid aus der Ballonsonde wenigstens ein Messwertpaar, insbesondere eine Mehrzahl von Messwertpaaren, aus eingeleiteter/entnommener Menge an Messfluid und jeweils zugeordnetem Ösophagusballondruck gewinnen. Anhand dieser Messwertpaare können Aussagen über den Befüllungszustand der Ballonsonde gewonnen werden und entsprechende Steuerprozeduren aktiviert und durchgeführt werden.

In weiterer Ausgestaltung kann das Ösophagusballonsteuersystem und/oder die Beatmungssteuerung wenigstens eines aus einem Fluidmengen-Überwachungssystem, einem Fluidbefüllungssystem und einem Kalibriersystem umfassen. Insbesondere kann im Fall, dass das Ösophagusballonsteuersystem wenigstens eines aus einem Fluidmengen-Überwachungssystem, einem Fluidbefüllungssystem und einem Kalibriersystem umfasst, die Beatmungssteuerung zu bidirektionaler Interaktion mit dem wenigstens einen aus Fluidmengen-Überwachungssystem, Fluidbefüllungssystem und Kalibriersystem ausgebildet sein. Auf diese Weise kann die Beatmungssteuerung das Ösophagusballonsteuersystem zum Start von vorbestimmten Überwachungsprozeduren, Fluid-Befüllungsprozeduren und/oder Kalibrierprozeduren ansteuern und/oder den Ablauf von Überwachungsprozeduren, Fluid-Befüllungsprozeduren und/oder Kalibrierprozeduren steuern. Hinsichtlich der durch bidirektionale Interaktion zwischen Ösophagusballonsteuersystem und dem wenigstens einen aus einem Fluidmengen-Überwachungssystem, einem Fluidbefüllungssystem und einem Kalibriersystem realisierbaren Möglichkeiten sei auf die vorangehend beschriebenen Beispiele verwiesen. Es ist insbesondere möglich, das Ösophagusballonsteuersystem vollständig mit der Beatmungssteuerung zu synchronisieren oder sogar in die Beatmungssteuerung zu integrieren. Auf diese Weise kann die Beatmungssteuerung die Beatmung eines Patienten auch über längere Zeiträume hinweg unter Berücksichtigung der vom Ösophagusballonkatheter gelieferten Informationen, insbesondere hinsichtlich des den Druck im Pleuraspalt näherungsweise wiedergebenden Ösophagusballondrucks, steuern/regeln. Dies schließt die Möglichkeit ein, bestimmte Steuerprozeduren am Ösophagusballonkatheter, wie Überwachung einer korrekten Befüllung der Ballonsonde mit Messfluid, Neubefüllung oder Nachfüllung der Ballonsonde mit Messfluid, Entnahme von Messfluid aus der Ballonsonde und/oder Kalibrierung der Ballonsonde zur Bestimmung einer Menge an Messfluid, mit der die Ballonsonde befüllt werden soll, in Koordination mit der weiterlaufenden Beatmung durchzuführen.

In Ausführungsformen kann vorgesehen sein, dass die Beatmungssteuerung und/ oder das Ösophagusballonsteuersystem in Reaktion auf eine Änderung eines der folgenden Beatmungsparameter bzw. einer der folgenden bei der Beatmung erfassten Größen um einen vorbestimmten Schwellenwert oder mehr den Start einer Kalibrierprozedur oder einer Fluidmengen-Überwachungsprozedur - sowie falls erwünscht einer Fluid-Befüllungsprozedur - im Ösophagusballonsteuersystem veranlasst:
(i) In Reaktion auf Änderungen des eingestellten Sollwerts für wenigstens einen der folgenden Beatmungsparameter um einen vorbestimmten Schwellenwert oder mehr: vorgegebener positiver endexpiratorischer Druck (PEEP); vorgegebener maximaler Atemwegsdruck (Paw_max); appliziertes Tidalvolumen (Vt); applizierter Tidalhub (IE); eingestellte Beatmungsrate (BR).
(ii) In Reaktion auf Erfassung von Änderungen um einen Schwellenwert oder mehr bei wenigstens einer der folgenden Größen (soweit nicht als Beatmungsparameter vorgegebenen): endexpiratorischer Druck (Pes_ee); Differenz zwischen endinspiratorischem Druck und endexpiratorischem Druck (Pes_ei - Pes_ee); Schwankung des Ösophagusdrucks (Pes); Nachgiebigkeit der Thoraxwand (chest wall compliance, Ccw = Vt/(Pes_ei-Pes_ee). Auch die unter (i) genannten Größen können als erfasste Größen angesehen werden sofern diese Größen in bestimmten Beatmungsmodi nicht als Beatmungsparameter vorgegeben sind, sondern frei oder in gewissen Grenzen einstellbar sind.

Häufig nimmt man den erfassten Druck in der Ösophagusballonsonde am Ende der Exspiration (Pes_ee) als Referenzdruck für Bestimmungen dieser Art. Dieser Druck wird dann auch als "Baseline-Druck" oder "Basis-Ösophagusballondruck" bezeichnet.

Das vorangehend angesprochene Kalibriersystem ist insbesondere zur automatisierten Einstellung einer betriebsgemäßen Befüllung der Ballonsonde des Ösophagusballonkatheters ausgebildet. Bei einer solchen Kalibrierung sucht man als betriebsgemäße Befüllung diejenige Befüllung an Messfluid in der Ballonsonde des Ösophagusballonkatheters, bei der das durch das Druckermittlungssystem bzw. einen Drucksensor zur Erfassung des Drucks in der Ballonsonde ermittelte Signal den im Pleuraspalt herrschenden Druck möglichst genau wiedergibt.

Zur Bestimmung der betriebsgemäßen Befüllung durch Kalibrierung kann das vorangehend angesprochene Kalibriersystem eine Kalibriersteuerung umfassen, die derart ausgebildet ist, dass sie die Menge an Messfluid in der Ballonsonde schrittweise verändert, wobei die Kalibriersteuerung bei jeder der auf diese Weise schrittweise als Messpunkte eingestellten Mengen an Messfluid in der Ballonsonde den zugehörigen Ösophagusballondruck aufnimmt und der jeweils eingestellten Menge an Messfluid in der Ballonsonde zuordnet. Der Ösophagusballondruck kann durch das Druckermittlungssystem erfasst werden oder durch einem zum Kalibriersystem gehörenden Drucksensor.

Bei sich zeitlich nicht ändernden Bedingungen der Beatmung und/oder des Ösophagusballonkatheters ist normalerweise die betriebsgemäße Befüllung dann erreicht, wenn für einen jeweiligen Atemzug die Differenz zwischen dem am Ende der Exspirationsphase erfassten Ösophagusballondruck und dem am Ende der Inspirationsphase erfassten Ösophagusballondruck maximal ist.

In Ausführungsformen umfasst das Kalibriersystem folgende Komponenten:
- eine Anordnung zum Befüllen der Ballonsonde mit einem Messfluid nach Platzieren der Ballonsonde im Ösophagus,
- einen Drucksensor zum Erfassen eines in der Ballonsonde herrschenden Ösophagusballondrucks,
- eine Kalibriersteuerung, die derart ausgebildet ist, dass sie die Menge an Messfluid in der Ballonsonde schrittweise verändert, wobei die Kalibriersteuerung bei jeder der auf diese Weise schrittweise als Messpunkte eingestellten Mengen an Messfluid in der Ballonsonde einen von dem Drucksensor erfassten Ösophagusballondruck aufnimmt und der jeweils eingestellten Menge an Messfluid in der Ballonsonde zuordnet. Der Begriff "einen Ösophagusballondruck aufnehmen" soll insbesondere die Bedeutung haben, einen Differenzdruck zwischen dem am Ende der Inspirationsphase und dem am Ende der Exspirationsphase erfassten Druck in der Ballonsonde zu erfassen.

Wenn die Beatmungssteuerung, neben ihrer eigentlichen Funktion zur Steuerung/ Regelung von bei der Beatmung von der Beatmungsvorrichtung durchgeführten Beatmungsmodi bzw. Beatmungsprozeduren, auch zur automatisierten Ansteuerung des Ösophagusballonsteuersystems ausgebildet ist, lässt sich mittels des Kalibriersystems eine Kalibrierung zur Bestimmung der betriebsgemäßen Befüllung der Ballonsonde des Ösophagusballonkatheters und ggf. eine Neubefüllung oder Nachfüllung der Ballonsonde mit Messfluid mit ausreichender Genauigkeit auch unter Bedingungen durchführen, bei denen zeitlich veränderliche Bedingungen während der Beatmung auftreten. Dies gilt insbesondere dann, wenn sich während des Ablaufs einer Kalibrierung bestimmte vorgegebene Beatmungsparameter oder bei der Beatmung erfasste Größen ändern. Die Beatmungssteuerung kann dann dazu ausgebildet sein, diese Änderungen an das Ösophagusballonsteuersystem zu übermitteln.

Beispielsweise gibt es Beatmungsmodi, in denen das in einem jeweiligen Beatmungszug applizierte Tidalvolumen Vt nicht konstant ist, sondern sich von Beatmungszug zu Beatmungszug verändert. Diese Veränderung des Tidalvolumens Vt hat normalerweise einen Einfluss auf die Differenz dPeso zwischen dem am Ende der Inspirationsphase gemessenen Ösophagusballondruck Peso_ei und dem am Ende der Exspirationsphase eines Beatmungszugs gemessenen Ösophagusballondruck Peso_ee. Dieser Einfluss lässt sich kompensieren, wenn die Änderung des Tidalvolumes Vt in die Bestimmung der bei der Kalibrierung zu maximierenden Differenz mit einbezogen wird. In einer Ausführungsform der Offenbarung kann demgemäß vorgesehen sein, dass die Kalibriersteuerung die Differenz dPeso zwischen dem Ösophagusballondruck am Ende der Inspirationsphase Peso_ei und dem Ösophagusballondruck am Ende der Exspirationsphase Peso_ee auf das in einem jeweiligen Beatmungszug applizierte Tidalvolumen Vt bezieht und als betriebsgemäßen Befüllung der Ballonsonde die Menge an Messfluid in der Ballonsonde bestimmt, bei der der auf diese Weise erhaltene Quotient dPeso/Vt maximal wird. Die Maximierung des Quotienten dPeso/Vt ist somit weniger von atemzugsweisen Schwankungen abhängig als dPeso. Sie entspricht bei konstantem Tidalvolumen Vt jedoch der Maximierung von dPeso.

Wenn sich das Tidalvolumen Vt für jeden Beatmungszug ändert, bedeutet dies, dass man die erfassten Werte der Druckdifferenz dPeso auf das jeweils applizierte Tidalvolumen Vt bezieht. Dadurch kann man zu einem von der Tiefe der Beatmungszüge unabhängigeren Wert für die Druckdifferenz im Ösophagusballondruck kommen. Diese Maßnahme verbessert die Genauigkeit der Kalibrierung.

Da das Tidalvolumen Vt eine Größe ist, die von der Beatmungssteuerung geliefert wird, erlaubt die hier vorgeschlagene bidirektionale Kommunikation zwischen der Beatmungssteuerung (gehört zur Beatmungsvorrichtung) und dem Kalibriersystem (gehört zur Ösophagusballonsteuerung) eine Kalibrierung des Ösophagusballonkatheters unter Bedingungen, wie dies zuvor nicht möglich war. Weil die Beatmungsvorrichtung für das Kalibriersystem zusätzliche Informationen bereitstellt (Tidalvolumen), kann die Genauigkeit der Kalibrierung verbessert werden und damit auch die Genauigkeit von Druckmesswerten, die von einem in den Ösophagus eingeführten Ballonkatheter geliefert werden, verbessert werden. Insbesondere erlaubt das Kalibriersystem eine genauere Wiedergabe des im Pleuraspalt herrschenden Drucks durch den Ballonkatheter und schnellere Reaktion der Messwerte auf sich verändernde Umgebungsbedingungen während andauernder Beatmung. Damit wird eine Kalibrierung des Ballonkatheters während der Beatmung möglich, ohne dass die Beatmung dazu unterbrochen werden muss. Dies gilt selbst dann, wenn die Beatmung mittels vollautomatischer Beatmungsmodi erfolgt, beispielsweise bei Beatmung mittels geschlossener Regelkreise, wie bei der von der Anmelderin entwickelten "Adaptive Support Ventilation" ("ASV-Beatmung") sowie der INTELLiVENT-ASV.

In Ausführungsformen ist die Kalibriersteuerung derart ausgebildet, dass sie zum Anfahren der jeweiligen Messpunkte die Menge an Messfluid ausgehend von einem Startwert bis zum Erreichen eines Endwertes in mindestens zwei Schritten in monotoner Weise verändert.

Das Kalibriersystem kann insbesondere auch eine Anordnung zum Entnehmen von Messfluid aus der Ballonsonde (Fluidablasseinrichtung) nach Platzieren der Ballonsonde im Ösophagus umfassen. Sowohl die Anordnung zum Befüllen der Ballonsonde mit einem Messfluid nach Platzieren der Ballonsonde im Ösophagus als auch die Anordnung zum Entnehmen von Messfluid aus der Ballonsonde nach Platzieren der Ballonsonde im Ösophagus können eine oder mehrere Ventile aufweisen, das/die in einer mit der Ballonsonde in Fluidverbindung stehenden Förderleitung für Messfluid angeordnet ist/sind. Soweit das Messfluid in der Ballonsonde unter einem Überdruck steht, kann die Fluidablasseinrichtung einfach durch Ansteuerung von Ventilen in einer mit der Ballonsonde in Fluidverbindung stehenden Fluidleitung realisiert sein.

Die Anordnung zum Befüllen der Ballonsonde mit einem Messfluid nach Platzieren der Ballonsonde im Ösophagus kann eine Pumpvorrichtung umfassen, mittels derer Messfluid in die Ballonsonde gepumpt werden kann. In diesem Fall kann die Pumpvorrichtung auch zum Entnehmen (Abpumpen) von Messfluid aus der Ballonsonde ausgebildet sein. Die Fluidablasseinrichtung kann die Pumpvorrichtung aufweisen.

In weiterer Ausgestaltung kann die Anordnung zum Befüllen der Ballonsonde mit einem Messfluid und/oder die Anordnung zum Entnehmen von Messfluid aus der Ballonsonde einen Flusssensor aufweisen, insbesondere einen Massenflusssensor, welcher zur Bestimmung einer Menge an in die Ballonsonde eingeleitetem und/oder einer Menge an aus der Ballonsonde entnommenen Messfluid ausgebildet ist. Beispielsweise kann durch Integrieren eines von dem Flusssensor gemessenen Flusses über einen Zeitraum zwischen einem Startzeitpunkt und einem Endzeitpunkt die jeweils in die Ballonsonde eingeleitete bzw. aus der Ballonsonde entnommene Menge an Messfluid bestimmt werden. Wenn ein Flusssensor eingesetzt wird, der den Fluss an Messfluid anhand eines Differenzdrucks bestimmt, kann der Flusssensor auch zur Erfassung des in der Ballonsonde herrschenden Ösophagusballondrucks eingesetzt werden. Ein separater Drucksensor ist ebenfalls möglich.

Die Kalibriersteuerung kann als eigenständiges Bauteil "in Hardware" realisiert werden. Alternativ kann die Kalibriersteuerung auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocomputer kann zu einer Steuerung des Kalibriersystems gehören.

Mit dem Begriff "in monotoner Weise" soll zum Ausdruck gebracht werden, dass die Menge an Messfluid in der Ballonsonde sich während eines Messzyklus immer in derselben Richtung verändert. Das heißt, die Menge an Messfluid nimmt während eines Messzyklus zwischen Startwert und Endwert zum Erreichen jedes weiteren Messpunktes entweder immer weiter ab oder immer weiter zu. Dabei definiert das Anfahren von Messpunkten zwischen dem Startwert und dem Endwert einen Messzyklus. Startwert und Endwert eines Messzyklus können durch vorbestimmte Mengen an in die Ballonsonde geleitetem bzw. aus der Ballonsonde geleitetem Messfluid definiert sein. Alternativ ist auch denkbar, Startwert und Endwert durch vorbestimmte Werte des in der Ballonsonde erfassten Drucks festzulegen. Gleiches gilt auch für die einzelnen Messpunkte, die zwischen Startwert und Endwert eines Messzyklus angefahren werden. Es hat sich herausgestellt, dass es einfacher und schneller ist, jedenfalls beim Anfahren aufeinander folgender Messpunkte zwischen dem Startwert und dem Endwert (wobei Start - und Endwert ggf. nicht einbegriffen sind) immer eine vorbestimmte Menge an Messfluid aus der Ballonsonde abzuleiten bzw. in die Ballonsonde zu einzuleiten.

Das offenbarungsgemäß vorgeschlagene monotone Durchschreiten des Messbereichs zwischen Startwert und Endwert innerhalb eines Messzyklus beschleunigt die Kalibrierung enorm. Dies liegt insbesondere daran, dass aufeinander folgende Messpunkte innerhalb eines Messzyklus ausgehend vom Startwert bis mindestens zum Endwert unmittelbar nacheinander angefahren werden können. Man benötigt insbesondere keine Zwischenschritte zum Evakuieren der Ballonsonde und/oder zum Einstellen von jeweils genau reproduzierbaren Anfangsbedingungen für die einzelnen Messpunkte während einer Kalibrierung mehr. Zudem müssen keine Druckausgleichsvorgänge oder Relaxationsvorgänge mehr abgewartet werden. Es hat sich überraschenderweise gezeigt, dass die Kalibrierung durch das Weglassen einer für jeden Messpunkt gleichen Prozedur mit denselben Anfangsbedingungen für jeden Messpunkt keinen - jedenfalls nicht inakzeptabel großen - negativen Einflüssen unterliegt. Insbesondere scheinen sich Hystereseeffekte - soweit vorhanden - auf alle Messpunkte innerhalb eines Messzyklus in etwa gleich auswirken und deshalb die Kalibrierung nicht zu stören.

Besondere Ausgestaltungen des hier vorgeschlagenen Kalibriersystems können eines oder mehrere der nachfolgend dargelegten optionalen Merkmale aufweisen. Soweit Merkmale ausschließlich alternative Ausgestaltungen betreffen, ist dies im Folgenden ausdrücklich vermerkt. Es versteht sich daher, dass die nachfolgenden Merkmale in beliebiger Weise mit den jeweils vorangehend oder nachfolgend dargelegten Merkmalen kombinierbar sind, soweit nicht ausdrücklich ausgeschlossen. Wie bereits angesprochen, kann das Kalibriersystem ferner eine Fluidablasseinrichtung umfassen, die zum Ablassen von Messfluid aus der Ballonsonde ausgebildet ist. Dabei steuert die Kalibriersteuerung die Fluidablasseinrichtung zum Anfahren der jeweiligen Messpunkte derart an, dass die Menge an Messfluid in der Ballonsonde ausgehend von dem Startwert bis zum Erreichen des Endwertes in mindestens zwei Schritten in monotoner Weise abnimmt. Das Ansteuern kann beispielsweise durch zeitweiliges Öffnen von Ventilen in einer mit der Ballonsonde in Fluidverbindung stehenden Förderleitung erfolgen. Auch aktives Pumpen mittels einer entsprechenden Pumpeinrichtung ist denkbar.

Die Kalibriersteuerung kann ferner derart ausgebildet sein, dass sie die Anordnung zum Befüllen der Ballonsonde mit einem Messfluid wenigstens in einem ersten Messzyklus zum Befüllen der Ballonsonde mit einer Menge an Messfluid derart ansteuert, dass die Menge an in die Ballonsonde eingeleitetem Messfluid größer ist als eine obere Grenze des dem Messzyklus zugeordneten Messbereichs zwischen Startwert und Endwert. In diesem Fall wird der Startwert, der den Beginn des Messzyklus definiert, erst nach Ablassen einer ersten Menge an Messfluid aus der Ballonsonde angefahren. Die Kalibriersteuerung kann die Anordnung zum Befüllen der Ballonsonde derart ansteuern, dass diese die Ballonsonde zunächst mit einer größeren Menge an Messfluid befüllt als die dem Startwert entsprechende Menge an Messfluid. Die Kalibriersteuerung kann dann die Fluidablasseinrichtung derart ansteuern, dass diese Messfluid aus der Ballonsonde ablässt, bis sich in der Ballonsonde die dem Startwert entsprechende Menge an Messfluid befindet.

Diese Maßnahmen führen zu einem gewissen Überdehnen der Ballonsonde vor Beginn des anstehenden Messzyklus. Damit ist sichergestellt, dass der Messzyklus tatsächlich den gesamten als Messbereich nutzbaren Bereich abdeckt, in dem sich der von der Ballonsonde gemessene Druck ungefähr linear mit Menge an Messfluid in der Ballonsonde ändert. Bei der vorgeschlagenen Vorgehensweise mit Überdehnen der Ballonsonde vor Anfahren des Startwerts nimmt also während des Messzyklus zumindest in einem mittleren Bereich der in der Ballonsonde gemessene Druck ungefähr linear mit Menge an Messfluid in der Ballonsonde ab. Diese lineare Abnahme legt den grundsätzlich als Messbereich für den Ösophagusballonkatheter nutzbaren Bereich zwischen einer oberen Befüllung an Messfluid in der Ballonsonde und einer unteren Befüllung an Messfluid in der Ballonsonde fest.

Da die Messung in vivo, also bei im Ösophagus eines Patienten platzierter Ballonsonde erfolgt, wird im als Messbereich herangezogenen linearen Teil die Steigung der Ösophagusballondruck/Messfluidmenge-Kurve mehr von der Dehnbarkeit des Ösophagus und weniger von der Dehnbarkeit der Ballonsonde bestimmt. Der Ösophagus dehnt sich im linearen Teil mit abnehmender Messfluidmenge immer weniger bzw. verringert seinen Querschnitt mit abnehmender Messfluidmenge bei in etwa gleichbleibender Dehnbarkeit.

Im Bereich oberhalb des linearen Teils des Messbereichs ist - jedenfalls bei ausreichend großem Durchmesser der Ballonsonde - die maximale Dehnbarkeit des Ösophagus erreicht. Die Ballonsonde kann sich dann bei weiterer Erhöhung der eingefüllten Fluidmenge kaum mehr weiter dehnen. Daher steigt der Druck im Ösophagusballon mit zunehmender Messfluidmenge steiler an als im linearen Teil des Messbereichs. In der Regel wird, jedenfalls in einem ersten Messzyklus, der Startwert für die Messfluidmenge so gewählt sein, dass der Startwert für den gemessenen Ösophagusballondruck in dem Bereich oberhalb des linearen Teils des Messbereichs liegt.

In weiterer Ausgestaltung kann die Kalibriersteuerung dazu ausgebildet sein, mindestens zwei Messzyklen nacheinander auszuführen. Dabei kann der Messbereich der mindestens zwei aufeinanderfolgenden Messzyklen verschieden sein. Insbesondere kann ein vorangehender Messzyklus den Messbereich für einen nachfolgenden Messzyklus festlegen.

Beispielsweise kann im vorangehenden Messzyklus ein erster Messbereich zwischen einem ersten Startwert und einem ersten Endwert durchfahren werden, um in etwa den linearen Bereich zu finden, in dem die Dehnbarkeit des Ösophagus in etwa gleich bleibt. Danach kann im nachfolgenden Messzyklus eine feinere Abstufung der Messpunkte zwischen einem zweiten Startwert und einem zweiten Endwert, die beide im linearen Bereich liegen, erfolgen.

Die Kalibriersteuerung kann ferner derart ausgebildet sein, dass sie den Abstand zwischen aufeinanderfolgenden Messpunkten für den vorangehenden Messzyklus und für den nachfolgenden Messzyklus unterschiedlich einstellt. Dabei soll sich der Begriff "Abstand zwischen Messpunkten" auf die Differenz zwischen den zugehörigen Mengen an Messfluid in der Ballonsonde beziehen.

Beispielsweise kann in einem vorangehenden Messzyklus ein relativ weiter Abstand zwischen aufeinanderfolgenden Messpunkten eingestellt sein, um den Bereich zu identifizieren, in dem der im Ösophagusballonkatheter ermittelte Ösophagusballondruck sich in etwa linear mit der Menge an Messfluid in der Ballonsonde verändert. In einem nachfolgenden Messzyklus kann dann dieser lineare Bereich zwischen der zugehörigen maximalen Menge an Messfluid und minimalen Menge an Messfluid mit geringerer Schrittweite durchfahren werden, um die optimale Befüllungsmenge an Messfluid für den Ösophagusballonkatheter zu finden.

Ferner kann die Kalibriersteuerung dazu ausgebildet sein, die Schrittweite zwischen aufeinander folgenden Messpunkten innerhalb des Messbereichs in einem Messzyklus in adaptiver Weise zu bestimmen. Eine solche adaptive Bestimmung der Schrittweite kann beispielsweise anhand eines Gradientenverfahrens wie dem Newton-Algorithmus erfolgen.

Bei mehreren aufeinanderfolgenden Messzyklen kann es vorteilhaft sein, die bestimmte "Überdehnung" der Ballonsonde am Anfang, also das anfängliche Einleiten einer größeren Menge an Messfluid als es dem Startwert des jeweiligen Messzyklus entspricht, vor jedem Messzyklus durchzuführen. Dann kann nämlich die Kalibriersteuerung derart ausgebildet sein, dass zwischen einem vorangehenden Messzyklus und einem nachfolgenden Messzyklus keine vollständige Entleerung des Messfluids aus der Ballonsonde stattfindet. Es findet in solchen Ausführungsformen insbesondere kein vollständiges Evakuieren des von der Ballonsonde umschlossenen Hohlraums bzw. Volumens statt, auch nicht zwischen aufeinander folgenden Messzyklen. Man macht sich vielmehr zu Nutze, dass es auf die genaue Menge an vor Beginn des jeweiligen Messzyklus in der Ballonsonde befindlichen Messfluid nicht ankommt, wenn man ausgehend von einem beliebigen Startwert am Anfang eine größere Menge an Messfluid in die Ballonsonde einleitet, als für den Startwert benötigt wird, weil die so bewirkte Überdehnung viel mehr von der Menge an in die Ballonsonde eingeleitetem Messfluid abhängt als vom Anfangszustand, von dem aus die Befüllung gestartet ist. Zudem kommt es häufig auch auf den genauen Grad der Überdehnung der Ballonsonde gar nicht an, weil man beim zum Erreichen des Startwerts für den nachfolgenden Messzyklus erforderlichen Entnehmen von Messfluid aus der Ballonsonde den für den jeweiligen Messzyklus gewünschten Startwert anhand des erfassten Ösophagusballondrucks erkennen kann.

Zur Verbesserung der Genauigkeit kann es allerdings auch vorgesehen sein, vor Beginn eines Messzyklus, oder wenigstens vor Beginn eines ersten Messzyklus bei mehreren aufeinander folgenden Messzyklen, die Ballonsonde auf einen vorbestimmten "Nullzustand" einzustellen, beispielsweise durch Abpumpen oder andersartiges Ableiten von Messfluid aus der Ballonsonde, bis ein vorbestimmter Unterdruck (z.B. - 20 hPa gegenüber Umgebungsdruck) erreicht ist. Auf diese Weise kann sichergestellt werden, dass die Kalibrierprozedur von einem vorbestimmten Anfangszustand der Ballonsonde aus startet, in dem beispielsweise sicher ist, dass die Ballonsonde eine vollständig zusammengefaltete Konfiguration aufweist. Ausgehend von diesem Nullzustand kann dann Messfluid in die Ballonsonde eingeleitet bzw. gepumpt werden, bis eine Überfüllung der Ballonsonde auf einen vorbestimmten Druck, der oberhalb des Startwerts für den ersten Messzyklus liegt, erfolgt ist. Von diesem Zustand aus kann dann die Kalibrierprozedur in der vorangehend beschriebenen Weise durch aufeinander folgendes Anfahren von mehreren Messpunkten in monotoner Weise zwischen einem Startwert und einem Endwert erfolgen.

In weiteren Ausführungsformen kann vorgesehen sein, dass die Kalibriersteuerung derart ausgebildet ist, dass sie für jeden Messpunkt, d.h. für jede eingestellte Menge an Messfluid in der Ballonsonde zwischen dem Startwert und dem Endwert eines Messzyklus (falls gewünscht, einschließlich des Startwerts und des Endwerts), einen Messwert für den Ösophagusballondruck am Ende einer Inspirationsphase und einen Messwert für den Ösophagusballondruck am Ende einer Exspirationsphase ermittelt und dann die Differenz zwischen dem Ösophagusballondruck am Ende der Inspirationsphase und dem Ösophagusballondruck am Ende der Exspirationsphase bestimmt.

Dabei kann die Kalibriersteuerung derart ausgebildet sein, dass sie für einen zwischen dem Startwert für die Menge an Messfluid in der Ballonsonde und dem Endwert für die Menge an Messfluid in der Ballonsonde liegenden Bereich (falls gewünscht, einschließlich des Startwerts und des Endwerts) einen Maximalwert für die Differenz zwischen dem Ösophagusballondruck am Ende der Inspirationsphase und dem Ösophagusballondruck am Ende der Exspirationsphase bestimmt (Maximaldruck). Die dem Maximaldruck entsprechende Menge an Messfluid in der Ballonsonde entspricht dann der optimalen Befüllung für die Ballonsonde, d.h. der Menge an Messfluid in der Ballonsonde, mit der die Beatmung durchgeführt werden sollte.

Ferner kann die Kalibriersteuerung derart ausgebildet sein, dass sie für einen jeweiligen Messpunkt zwischen dem Startwert und dem Endwert den Messwert für den Ösophagusballondruck am Ende einer Inspirationsphase und den Messwert für den Ösophagusballondruck am Ende einer Exspirationsphase bei weiterlaufender Beatmung ermittelt.

Ein laufender Beatmungszyklus bzw. Atemzyklus braucht somit zur Ermittlung eines Messwerts für den Ösophagusballondruck nicht unterbrochen zu werden. Es ist insbesondere zur Ermittlung dieser Messwerte keine Einstellung einer Totzeit erforderlich, in der die Atemwege verschlossen werden, so dass der Fluss an Beatmungsgas bzw. Atemgas zum Erliegen kommt. Die Beatmung eines Patienten kann somit unverändert weiterlaufen, während eine Kalibrierung bzw. Neukalibrierung des Ösophagusballonkatheters durchgeführt wird. Dies ist ein großer Vorteil, nicht nur, weil jede Unterbrechung oder Störung der Beatmung aus Patientensicht zu vermeiden ist, sondern auch weil die Kalibrierung des Ösophagusballonkatheters auf diese Weise unter möglichst lebensnahen Bedingungen erfolgen kann.

Ferner kann die Kalibriersteuerung derart ausgebildet sein, dass sie die für die jeweiligen Messpunkte ermittelten Differenzen zwischen dem Ösophagusballondruck am Ende der Inspirationsphase und dem Ösophagusballondruck am Ende der Exspirationsphase miteinander vergleicht, und dann, wenn über eine Mehrzahl von aufeinander folgenden Messpunkten die für einen jeweiligen Messpunkt ermittelte Druckdifferenz innerhalb einer vorgegebenen Schwankungsbreite bezüglich eines Maximalwerts der Druckdifferenz liegt, die einer optimalen Befüllung der Ballonsonde entsprechende Menge an Messfluid als eine Menge an Messfluid bestimmt, die einen vorgegebenen Abstand zur kleinsten Menge an Messfluid in der Ballonsonde (unterer Rand) und/oder zur größten Menge an Messfluid in der Ballonsonde (oberer Rand), bei der die erfasste Druckdifferenz noch in die Schwankungsbreite fällt, hat.

Beispielsweise kann vorgegeben sein, dass die der optimalen Befüllung der Ballonsonde entsprechende Menge an Messfluid näher am unteren Rand eines solchen Bereichs mit in etwa gleichen Druckdifferenzen in der Nähe des Maximalwertes liegt als am oberen Rand. Die der optimalen Befüllung der Ballonsonde entsprechende Menge an Messfluid in der Ballonsonde ("optimale Befüllung") kann beispielsweise um 1/3 des Abstands zwischen der dem oberen Rand des Bereichs mit in etwa gleicher Druckdifferenz entsprechenden Menge an Messfluid in der Ballonsonde und der dem unteren Rand des Bereichs in etwa gleicher Druckdifferenz entsprechenden Menge an Messfluid in der Ballonsonde größer sein als die dem unteren Rand des Bereichs mit in etwa gleicher Druckdifferenz entsprechende Menge an Messfluid.

Insbesondere kann die Kalibriersteuerung derart ausgebildet sein, dass sie für jeden Messpunkt zwischen dem Startwert und dem Endwert (ggf. einschließlich Startwert und Endwert) eine Mehrzahl von Messwerten, insbesondere eine Mehrzahl von Messwertpaaren für den Ösophagusballondruck am Ende einer Inspirationsphase und für den Ösophagusballondruck am Ende einer Exspirationsphase ermittelt. Dies wird in der Regel in aufeinander folgenden Beatmungszyklen geschehen. Dann kann die Kalibriersteuerung anhand der Mehrzahl von Messwerten für jeden Messpunkt einen Durchschnitt und eine statistische Streuung für den Messwert oder eine daraus abgeleitete Größe, insbesondere die Differenz zwischen dem Ösophagusballondruck am Ende der Inspirationsphase und dem Ösophagusballondruck am Ende der Exspirationsphase (Druckdifferenz), bestimmen und die Anzahl von Messungen je Messpunkt so festlegen, dass der erhaltene Durchschnitt als statistisch signifikant angesehen werden kann.

Generell gilt: Je stärker die statistische Schwankung der erhaltenen Messwerte ist, desto mehr Atemzyklen werden für die Ermittlung des einem jeweiligen Messpunkt zugeordneten Messwerts für die Druckdifferenz zugrunde gelegt. Hier können beispielsweise kardiogene Oszillationen oder Bewegungen eine Rolle spielen. Solche Effekte können durch Erhöhung der Anzahl von Atemzyklen pro Messung besser unterschieden werden. Als Durchschnitt kann insbesondere ein arithmetisches Mittel zugrunde gelegt werden. Aber auch andere Arten der Durchschnittsbildung sind denkbar, beispielsweise ein geometrischer Durchschnitt.

Die statistische Streuung kann insbesondere durch eine Gaussche Standardabweichung repräsentiert sein. Dann wäre es denkbar, als statistisch signifikant ein 2-Sigma Niveau festzulegen, also eine Gaussche Standardabweichung von 95% oder besser also signifikant zu werten. Eine weitere Variante könnte darin bestehen, zur Ermittlung der Streuung adaptiv vorzugehen, beispielsweise indem für einen jeweiligen Messpunkt die Änderung des Mittelwertes nach jedem neuen Messwert als Maß ist für die Signifikanz herangezogen wird. Je kleiner die Änderung des Mittelwerts nach einem jeweiligen Messwert ist, desto höher ist die Signifikanz. Es können für einen jeweiligen Messpunkt solange Messungen wiederholt werden, bis die Änderung des Mittelwerts nach der letzten Messung kleiner geworden ist als ein vorbestimmter Schwellenwert. Danach kann zum nächsten Messpunkt übergegangen werden, etwa durch Entnehmen einer vorbestimmten Menge an Messfluid aus der Ballonsonde.

In weiteren Ausführungsformen kann die Kalibriersteuerung derart ausgebildet sein, dass sie für jeden Messpunkt zwischen dem Startwert und dem Endwert überwacht, ob die jeweilige Messung (bei mehrmaliger Messung einer Druckdifferenz pro Messpunkt bei jeder dieser Messungen) durch äußere Umstände beeinträchtigt ist, und die jeweilige Messung verwirft, wenn solche äußeren Umstände festgestellt werden. Äußere Umstände können beispielsweise Schluckbemühungen eines Patienten sein. Dabei kann vorgesehen sein, dass nicht die gesamte Kalibrierung verworfen oder abgebrochen wird, sondern lediglich der durch äußere Umstände beeinträchtige Messwert verworfen wird. Insbesondere kann ein neuer Messwert für den jeweiligen Messpunkt unmittelbar nachfolgend ermittelt werden. Danach kann die Kalibrierungsprozedur normal weiterlaufen. Es werden also artifizielle Messwerte "in Echtzeit" eliminiert, ohne dass die Kalibrierungsprozedur abgebrochen oder für längere Zeit unterbrochen werden muss. Ein erneutes Starten der gesamten Kalibrierung nach Erkennen von äußeren Umständen würde deutlich länger dauern und könnte, beispielsweise im Fall von Schluckbemühungen des Patienten während einer oder mehrerer Messungen zudem dazu führen, dass erneut Schluckbemühungen beim Patienten ausgelöst werden. Dies ist gegenüber bekannten Ansätzen ein enormer Fortschritt, denn bei den bekannten Ansätzen ist entweder die Entscheidung einer Bedienperson erforderlich, was jede Automatisierung unmöglich macht, oder es müssen automatisiert ablaufende Kalibrierungen ohne Berücksichtigung äußerer Umstände durchgezogen werden. Erst hinterher wird eine Kalibrierung komplett verworfen, wenn durch Analyse der Daten im Raum steht, dass wenigstens einer oder einige der Messwerte, die in die fragliche Kalibrierung eingeflossen sind, möglicherweise von äußeren Einflüssen wie Schluckbemühungen o.Ä. beeinträchtigt sind. Es lässt sich also nicht mit Sicherheit und bereits vor Beginn einer Kalibrierung sagen, ob diese Kalibrierung gültig ist oder möglicherweise verworfen werden muss.

Ungewöhnliche äußere Umstände wie Schluckbemühungen des Patienten können vom Ösophagusballonsteuersystem erkannt werden, beispielsweise durch Überwachung des zeitlichen Verlaufs des Ösophagusballondrucks, insbesondere wenn am Ende der maschinell vorgegebenen Inspirationsphase und/oder am Ende der maschinell vorgegebenen Exspirationsphase Veränderungen des Drucksignals auftreten.

Es kann auch vorgesehen sein, dass die Beatmungssteuerung Unregelmäßigkeiten bei der Beatmung erfasst und nach Maßgabe einer solchen Erfassung entsprechende Steuersignale für das Ösophagusballonsteuersystem bereitstellt, wenn Unregelmäßigkeiten bei der Beatmung auftreten, etwa Schluckbemühungen des Patienten.

Die Kalibriersteuerung kann derart ausgebildet sein, dass sie bei Auftreten solcher Störungen die Kalibrierung unterbricht und erst wieder fortsetzt, wenn feststellbar ist, dass keine weiteren Störungen mehr auftreten. Derartige Feststellungen können beispielsweise anhand des erfassten Ösophagusballondrucks und/oder Drucks im Atemweg getroffen werden. Auch in diesem Zusammenhang kann vorgesehen sein, dass die Beatmungssteuerung entsprechende Steuersignale bereitstellt.

Ferner kann die Kalibriersteuerung dazu ausgebildet sein, anhand der während der Kalibrierprozedur ermittelten Daten einen Qualitätsindex zu berechnen. Der Qualitätsindex kann eine gewichtete Zusammenfassung von Einflüssen verschiedener Faktoren darstellen. In solchen Ausführungsformen kann anhand des Qualitätsindex entschieden werden, ob Änderungen von Beatmungsparametern auf Grundlage von Daten des Ösophagusballondrucks zugelassen werden oder nicht. Für den Qualitätsindex relevante Kriterien können die statistische Streuung der Messdaten oder ein ungewöhnlicher Verlauf der Ösophagusballondruck /Messfluidmenge-Kurve sein. Als ein Maß für den Qualitätsindex kann die Streuung der Messwerte, beispielsweise ausgedrückt als Gaussche Standardabweichung herangezogen werden. Darüber hinaus können bestimmte Regeln festgelegt werden, die in den Qualitätsindex einfließen. Beispiele solcher Regeln können sein: Wenn während einer Kalibierprozedur ein Schlucken des Patienten erkannt wird, wird der Qualitätsindex reduziert. Wenn das Maximum der Differenz zwischen dem erfassten Ösophagusballondruck am Ende der Inspiration und dem erfassten Ösophagusballondruck am Ende der Exspiration bei einem Messpunkt außerhalb, insbesondere oberhalb des linearen Bereichs der Kurve für die Beziehung zwischen Ösophagusballondruck und Menge an Messfluid in der Ballonsonde liegt, wird der Qualitätsindex reduziert. Wenn der Basisdruck, d.h. der Ösophagusballondruck am Ende der Exspirationsphase im Laufe aufeinander folgender Messungen nicht gleich bleibt oder sich stetig ändert, sondern Sprünge macht, wird der Qualitätsindex reduziert. Wenn ein bei einem höheren Wert an Fluidmenge in der Ballonsonde erfasster Ösophagusballondruck kleiner ist als der Wert bei der niedrigeren Fluidmenge in der Ballonsonde, wird der Qualitätsindex reduziert.

Ferner kann die Kalibriersteuerung derart ausgebildet sein, dass der Ösophagusballondruck einen vorgegebenen Maximaldruck nicht überschreiten darf. Beispielsweise kann vorgesehen sein, dass der Ösophagusballondruck maximal das Doppelte des maximalen Atemwegsdrucks bei der Beatmung betragen darf. Bei Erreichen dieses Drucks wird kein weiteres Fluid in die Ballonsonde geleitet bzw. Fluid aus der Ballonsonde abgelassen. Auf diese Weise soll übermäßige Überdehnung am Gewebe des Ösophagus vermieden werden. Auch eine übermäßige Überdehnung der Ballonsonde, die zu deren Beschädigung führen könnte, kann so vermieden werden.

Schließlich kann vorgesehen sein, dass die Kalibriersteuerung eine bereits oben angesprochene Ablasseinrichtung, durch welche Messfluid aus der Ballonsonde ableitbar ist, beim Anfahren der jeweiligen Messpunkte derart ansteuert, dass die Menge an Messfluid in der Ballonsonde ausgehend von dem Startwert schrittweise immer weiter verringert wird, bis bei Erreichen oder Unterschreiten eines vorgegebenen minimalen endexpiratorischen Ösophagusballondruck, beispielsweise - 5 hPa, der Endwert erreicht ist.

In weiteren Ausführungsformen kann die Kalibriersteuerung über ein mathematisches Modell verfügen, das das Atemsystem eines beatmeten Patienten beschreibt, und derart ausgebildet ist, dass sie anhand des Modells und für einen jeweiligen Messpunkt, d.h. für eine jeweils eingestellte Menge an Messfluid in der Ballonsonde zwischen dem Startwert und dem Endwert, vorliegender Messwerte für den Ösophagusballondruck relevante Parameter wie Atemwegswiderstand, Dehnbarkeit des Lungengewebes und/oder Dehnbarkeit des Brustkorbs ermittelt, insbesondere mittels jeweiliger Regressionsverfahren, und daraus eine vorgesehene Befüllung für die Ballonsonde als Optimierungsaufgabe ableitet. Beispielsweise kann die Kalibriersteuerung als optimale Befüllung eine Menge an Messfluid in der Ballonsonde festlegen, bei der die sich aus dem Regressionsverfahren ergebende Dehnbarkeit des Brustkorbs minimal ist.

In weiteren Ausführungsformen kann das vorangehend angesprochene Ösophagusballonsteuersystem ein Fluidbefüllungssystem mit einer Befüllungssteuerung umfassen, die derart ausgebildet ist, dass sie nach Ansteuerung durch das Ösophagusballonsteuersystem und/oder die Beatmungssteuerung eine Fluid-Befüllungsprozedur zum Befüllen der Ballonsonde mit einer vorbestimmten Menge an Messfluid durchführt. Eine vorbestimmte Menge an Messfluid soll hierbei eine jeweils gewünschte Befüllung an Messfluid sein, im Sinne einer Soll-Fluidmenge bei einer Regelung der in die Ballonsonde eingeleiteten Menge an Messfluid. Die vorbestimmte Menge an Messfluid wird häufig eine als optimal angesehene Befüllung sein und kann beispielsweise durch eine vorangehend durchgeführte Kalibrierungsprozedur, wie sie zuvor beschrieben worden ist, ermittelt werden.

Die Befüllungsteuerung kann als eigenständiges Bauteil "in Hardware" realisiert werden. Alternativ kann die Befüllungssteuerung auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocomputer kann zu einer Steuerung des Fluidbefüllungssystems gehören und je nach Ausgestaltung des Fluidbefüllungssystems der Ösophagusballonsteuerung und/oder der Beatmungssteuerung zugeordnet sein.

Um eine Vergleichbarkeit und Reproduzierbarkeit des nach Abschluss eines Befüllungsvorgangs jeweils erreichten Füllzustands der Ballonsonde sicherzustellen, kann die Befüllungssteuerung derart ausgebildet sein, dass sie vor jedem Start der Fluid-Befüllungsprozedur zur Befüllung der Ballonsonde eine Entleerungsprozedur zur Entnahme von in der Ballonsonde befindlichem Messfluid durchführt. Die Entleerungsprozedur kann am einfachsten dadurch erfolgen, dass man unter Überdruck stehendes Messfluid aus der Ballonsonde ausströmen lässt, bis ein Druckausgleich mit der Umgebung erfolgt ist. Einfacher und schneller lässt sich eine Entleerung der Ballonsonde aber mit Hilfe einer aktiven Pumpanordnung durchführen, mittels derer Messfluid aus der Ballonsonde abgepumpt wird, bis ein vorbestimmter Enddruck erreicht ist. In diesem Fall kann der Enddruck auch ein Unterdruck, d.h. ein niedrigerer Druck als der Umgebungsdruck, sein.

In weiteren Ausführungsformen kann die Entleerungsprozedur zur Entnahme von in der Ballonsonde befindlichem Messfluid eine Prozedur zur Einstellung eines Nullvolumens umfassen. Mit dem Begriff "Nullvolumen" soll eine vorbestimmte Befüllung an Messfluid im Ösophagusballonkatheter gemeint sein, bei der das Messfluid im Wesentlichen nur die Zuleitungen zu der Ballonsonde füllt, die Ballonsonde selbst aber noch vollständig kollabiert ist. Dieses Nullvolumen dient als reproduzierbarer Startpunkt für jeden Befüllungsvorgang. Es ist denkbar, dieses Nullvolumen auf einen bestimmten Druck zu beziehen, beispielsweise den Umgebungsdruck.

Die Prozedur zur Einstellung eines Nullvolumens kann beispielsweise folgende Schritte umfassen: (i) Entnehmen von Messfluid aus der Ballonsonde, bis ein erster vorbestimmter Unterdruck in der Ballonsonde erreicht ist, (ii) Einleiten von Messfluid in die Ballonsonde, bis ein zweiter vorbestimmter Unterdruck in der Ballonsonde erreicht ist, der größer ist als der erste vorbestimmte Unterdruck, und (iii) Einleiten eines vorbestimmten Volumens an Messfluid in die Ballonsonde. Dabei soll insbesondere der Ballon sowohl beim ersten Unterdruck als auch beim beim zweiten Unterdruck vollständig kollabiert sein.

Sofern für einen jeweiligen Ösophagusballonkatheter ein diesem zugeordnetes "Totvolumen" bekannt ist, kann man zur Bereitstellung eines Nullvolumens auch so vorgehen, dass in einem ersten Schritt der Ösophagusballonkatheter weitgehend evakuiert wird, also auf einen möglichst geringen Unterdruck abgepumpt wird, und ausgehend von dem dann erreichten Zustand eine vorbestimmte Menge an Messfluid in den Ösophagusballonkatheter eingeleitet wird, wobei die vorbestimmte Menge an Messfluid dem Totvolumen multipliziert mit einem Bezugsdruck für das Nullvolumen (beispielsweise dem Umgebungsdruck) entspricht. Das Totvolumen hängt von den Besonderheiten eines jeweiligen Ösophagusballonkatheters ab (beispielsweise von der Länge der Leitungen) und muss daher für einen jeweiligen Ösophagusballonkatheter in aller Regel experimentell bestimmt werden.

In weiteren Ausführungsformen kann vorgesehen sein, dass die Befüllungssteuerung derart ausgebildet ist, dass sie nach der vorangehend beschriebenen Entleerungsprozedur zur Entnahme von in der Ballonsonde befindlichem Messfluid eine Fluid-Befüllungsprozedur zum Einleiten von Messfluid in die Ballonsonde durchführt, bis ein vorbestimmter Überdruck in der Ballonsonde erreicht ist. Das Einleiten von Messfluid bis zu dem vorbestimmten Überdruck geschieht dann noch vor dem eigentlichen Einstellen einer vorbestimmten Menge an Messfluid in der Ballonsonde. Es handelt sich also um einen Zwischenschritt zwischen dem Entleeren der Ballonsonde und dem Einstellen der vorbestimmten Menge an Messfluid in der Ballonsonde.

Nachdem in dem Zwischenschritt der vorbestimmte Überdruck erreicht worden ist, kann wieder so viel Messfluid aus der Ballonsonde entnommen werden (passiv durch Ausströmenlassen oder aktiv durch Abpumpen), bis der der vorbestimmten Befüllung entsprechende vorbestimmte Ballondruck erreicht ist.

In weiterer Ausgestaltung kann vorgesehen sein, dass das Ösophagusballonsteuersystem dazu ausgebildet ist, einen Basis-Ösophagusballondruck zu erfassen und die Befüllungssteuerung zur Durchführung der Fluid-Befüllungsprozedur zum Befüllen der Ballonsonde mit Messfluid anzusteuern, wenn der Basis-Ösophagusballondruck um einen vorbestimmten Betrag oder mehr gegenüber einem Bezugsniveau absinkt.

Mit dem Begriff "Basis-Ösophagusballondruck" soll ein Druck oder eine Druckdifferenz gemeint sein, der/die sich über viele auf einander folgende Beatmungszyklen möglichst nicht verändert und der deswegen als Maßgröße für eine Veränderung des Füllungszustands der Ballonsonde dienen kann. Zu diesem Zweck ist es sinnvoll, wenn als Basis-Ösophagusballondruck eine Größe herangezogen wird, die die Veränderung des Ösophagusballonddrucks auf Grund der einzelnen Beatmungsprofile während aufeinander folgender Beatmungszyklen nicht widerspiegelt. Anders ausgedrückt, sollte der Basis-Ösophagusballondruck ein erfasster Ösophagusballondruck sein, dem kein Beatmungsprofil überlagert ist, das die Inspirationsvorgänge und Exspirationsvorgänge während der Beatmung wiedergibt. In der Praxis kann man beispielsweise zur Bestimmung des Basis-Ösophagusballondrucks den Ösophagusballondruck am Ende der Exspirationsphase von aufeinander folgenden Beatmungszyklen bestimmen. Alternativ kann auch der Ösophagusballondruck am Ende der Inspirationsphase aufeinander folgender Beatmungszyklen als Basis-Ösophagusballondruck definiert werden.

Die zuvor beschriebene Ösophagusballonsteuerung überwacht fortlaufend, ob der einmal eingestellte Befüllungszustand der Ballonsonde (dieser kann beispielsweise durch die oben beschriebene Kalibrierprozedur ermittelt werden) stabil bleibt und reagiert mit einer entsprechenden Korrektur der Befüllung an Messfluid in der Ballonsonde, wenn eine Veränderung des Basis-Osophagusballondrucks um einen vorbestimmten Wert oder mehr gegenüber dem Bezugsniveau festgestellt wird. Damit können insbesondere durch Leckageeffekte bedingte Verluste an Messfluid in der Ballonsonde ausgeglichen werden, jedenfalls solange sich die Leckagerate und/oder der Verlust an Messfluid in der Ballonsonde in gewissen Grenzen hält. Es ist denkbar, eine Leckagerate abzuschätzen, beispielsweise indem überwacht wird, in welchem Zeitraum der Basis-Ösophagusballondruck um einen vorbestimmten Betrag oder mehr absinkt, oder indem überwacht wird, wie stark der Basis-Ösophagusballondruck nach einer vorbestimmten Zeitdauer abgesunken ist. Bei bekannter Verlustmenge oder Leckagerate an Messfluid könnte die Kompensation des Leckageeffekts dadurch erfolgen, dass eine vorgewählte Menge an Messfluid in die Ballonsonde nachgefüllt wird, die sich aus der Verlustmenge oder der Leckagerate multipliziert mit der zugehörigen Zeitdauer ergibt. Ansonsten könnte das Nachfüllen von Messfluid derart erfolgen, dass der mit dem Einleiten von Messfluid in die Ballonsonde einhergehende Anstieg des Ösophagusballondrucks überwacht wird und das Nachfüllen beendet wird, wenn ein vorbestimmter Ösophagusballondruck erreicht wird.

Die vorangehend beschrieben Maßnahmen erlauben eine Überwachung, jedenfalls eine Abschätzung, einer Leckagerate des Ösophagusballonkatheters. Anhand dieser Überwachung kann entschieden werden ob und ggf. welche weiteren Maßnahmen zu ergreifen sind, z.B. Start einer Nachfüllprozedur, Start einer Entleerung der Ballonsonde, vollständige Neukalibrierung der Ballonsonde. Letzteres wird immer dann angemessen sein, wenn man annehmen muss, dass eine Veränderung des erfassten Ösophagusballondrucks nicht nur auf Leckageeffekte zurückgeführt werden kann, sondern noch andere Effekte eine Rolle spielen, beispielsweise eine Veränderung der Lage des Ballonkatheters im Ösophagus. Sollten Hinweise auf solche anderen Effekte vorhanden sein, wird man in der Regel eine vollständige Neukalibrierung des Ösophagusballonkatheters in Betracht ziehen oder ggf. sogar die Ballonsonde austauschen.

Wenn die Leckagerate zu groß ist, wird der zeitliche Abstand zwischen aufeinander folgenden Füllprozeduren sehr gering. Es kann vorgesehen sein, dass dann dem Bedienpersonal eine Warnung angezeigt wird, dass der Ösophagusballonkatheter mit Ballonsonde neu kalibriert werden muss oder ausgetauscht werden muss. Derartige Maßnahmen können beispielsweise vorgesehen sein, wenn innerhalb eines vorbestimmten Zeitraums der Ösophagusballondruck um einen vorbestimmten Betrag, z.B. um 2 cm H₂O in 5 Minuten, oder mehr absinkt.

Es sei angemerkt, dass die hier beschriebene Möglichkeit der Bereitstellung eines Fluidbefüllungssystems für einen Ösophaguskatheter mit Ballonsonde sowie die verschiedenen möglichen Ausführungsformen eines solchen Fluidbefüllungssystems nach Ansicht der Anmelderin einen eigenständig schützenswerten Beitrag zum Stand der Technik leisten. Dieser Beitrag ist insbesondere unabhängig von weiteren Merkmalen zu sehen, die im Anspruch 1 enthalten sind oder vorangehend näher erläutert worden sind. Die Anmelderin behält sich vor, im weiteren Verlauf des Prüfungsverfahrens zu der vorliegenden Erfindung eigenständige Ansprüche zu formulieren, die auf das Fluidbefüllungssystem gerichtet sind, oder gegebenenfalls solche Ansprüche in einer Teilanmeldung zu verfolgen.

In weiteren Ausführungsformen kann das vorangehend angesprochene Fluidmengen-Überwachungssystem eine Fluidmengenüberwachung umfassen, die derart ausgebildet ist, dass sie nach Ansteuerung eine Prozedur zur Überprüfung einer gewünschten oder betriebsgemäßen Menge an Messfluid in der Ballonsonde durchführt. Die gewünschte oder betriebsgemäße Menge an Messfluid in der Ballonsonde kann insbesondere die Menge an Messfluid in der Ballonsonde sein, bei der die Ballonsonde das optimale Signal zur Erfassung des Ösophagusballondrucks liefert und die aus diesem Grund als betriebsgemäße Befüllung ausgehend von einem evakuierten Ösophagusballonkatheter in die Ballonsonde einzufüllen ist. Diese gewünschte oder betriebsgemäße Menge an Messfluid kann beispielsweise als das Ergebnis einer Kalibrierung erhalten werden, wie vorangehend ausführlich mit Bezug auf das Kalibriersystem zur automatisierten Einstellung einer betriebsgemäßen Befüllung der Ballonsonde des Ösophagusballonkatheters beschrieben. Die Überwachung der Fluidmenge in der Ballonsonde soll insbesondere die Möglichkeit geben, zu erfassen, ob der einmal eingestellte gewünschte oder betriebsgemäße Füllzustand der Ballonsonde stabil bleibt oder zeitlichen Veränderungen unterliegt. Sofern durch das angesprochene Fluidmengen-Überwachungssystem solche Veränderungen festgestellt werden, kann darauf reagiert werden, entweder durch Nachfüllen von Messfluid in die Ballonsonde oder sogar durch Aktivieren einer vollständigen Neukalibrierung des Ösophagusballonkatheters, insbesondere zur Einstellung einer neuen betriebsgemäßen Befüllung der Ballonsonde.

Die Fluidmengenüberwachung kann als eigenständiges Bauteil "in Hardware" realisiert werden. Alternativ kann die eine Fluidmengenüberwachung auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocomputer kann zu einer Steuerung des Fluidmengen-Überwachungssystems gehören und je nach Ausgestaltung des FluidmengenÜberwachungssystems der Ösophagusballonsteuerung und/oder der Beatmungssteuerung zugeordnet sein.

In einer weiteren Ausgestaltung kann vorgesehen sein, dass das Fluidmengen-Überwachungssystem nach Start der Prozedur zur Überprüfung einer gewünschten Befüllung an Messfluid in der Ballonsonde die vorhandene Menge an Messfluid in der Ballonsonde schrittweise verändert, bei einer jeweiligen auf diese Weise als Messpunkt eingestellten Menge an Messfluid in der Ballonsonde einen Ösophagusballondruck erfasst und der jeweiligen als Messpunkt eingestellten Menge an Messfluid in der Ballonsonde zuordnet. Der Begriff "einen Ösophagusballondruck erfassen" soll insbesondere die Bedeutung haben, einen Differenzdruck zwischen dem am Ende der Inspirationsphase und dem am Ende der Exspirationsphase erfassten Druck in der Ballonsonde zu erfassen.

Das Fluidmengen-Überwachungssystem kann insofern ähnlich arbeiten wie das Kalibriersystem, als in beiden Systemen ein Differenzdruck zwischen dem am Ende der Inspirationsphase und dem am Ende der Exspirationsphase eines Beatmungszyklus erfassten Druck in der Ballonsonde ermittelt und der jeweiligen Kalibrierung (Einstellung einer betriebsgemäßen Befüllung der Ballonsonde des Ösophagusballonkatheters) bzw. Überwachung des Befüllungszustands der Ballonsonde zu Grunde gelegt werden kann. Allerdings unterscheidet sich die Überwachung des Befüllungszustands gemäß dem hier beschriebenen Fluidmengen-Überwachungssystem vor allem dadurch von dem vorangehend beschriebenen Kalibriersystem, dass die Anzahl von Messpunkten, die zur Überwachung des Befüllungszustands der Ballonsonde vorgesehen ist, sehr viel kleiner gewählt ist als die Anzahl der während einer Kalibrierung vorgesehenen Messpunkte.

Insbesondere kann vorgesehen sein, dass das Fluidmengen-Überwachungssystem nach Start der Prozedur zur Überprüfung einer gewünschten oder betriebsgemäßen Befüllung an Messfluid in der Ballonsonde die vorhandene Menge an Messfluid in der Ballonsonde auf eine erste als Messpunkt eingestellte Vergleichsmenge an Messfluid verändert, die größer ist als die vorhandene Menge an Messfluid. Bei der eingestellten ersten Vergleichsmenge an Messfluid wird ein erster Vergleichs-Ösophagusballondruck erfasst, so dass sich ein erstes (oberes) Vergleichs-Messwertpaar aus erster Vergleichsmenge an Messfluid in der Ballonsonde und zugeordnetem erstem Vergleichs-Ösophagusballondruck ergibt.

Ferner kann vorgesehen sein, dass das Fluidmengen-Überwachungssystem nach Start der Prozedur zur Überprüfung einer gewünschten Befüllung an Messfluid in der Ballonsonde die vorhandene Menge an Messfluid in der Ballonsonde auf eine zweite als Messpunkt eingestellte Vergleichsmenge an Messfluid verändert, die kleiner ist als die vorhandene Menge an Messfluid. Bei der zweiten Vergleichsmenge an Messfluid wird ein zweiter Ösophagusballondruck erfasst, so dass sich ein zweites Vergleichs-Messwertpaar aus zweiter Vergleichsmenge an Messfluid in der Ballonsonde und zugeordnetem zweitem Vergleichs-Ösophagusballondruck ergibt.

Insgesamt liegen dann für eine aktuelle Befüllung der Ballonsonde mit Messfluid drei Messwertpaare vor, ein der aktuellen Befüllung zugeordnetes Ist-Messwertpaar aus vorliegender Menge an Messfluid in der Ballonsonde und vorliegendem Ösophagusballondruck sowie je ein erstes Vergleichs-Messwertpaar, das einer größeren Menge an Messfluid in der Ballonsonde zugeordnet ist, und je ein zweites Vergleichs-Messwertpaar, das einer geringeren Menge an Messfluid in der Ballonsonde zugeordnet ist.

Falls gewünscht, können auch mehrere erste Vergleichs-Messwertpaare und mehrere zweite Vergleichs-Messwertpaare erzeugt werden und der Überprüfung einer gewünschten Befüllung an Messfluid in der Ballonsonde zugrunde gelegt werden.

Es sei angemerkt, dass bei der Prozedur zur Überprüfung einer gewünschten oder betriebsgemäßen Befüllung an Messfluid in der Ballonsonde die Reihenfolge, in der die einzelnen Vergleichs-Messwertpaare bestimmt werden, auch anders gewählt werden kann. Es kann insbesondere in einem ersten Schritt die vorhandene Menge an Messfluid in der Ballonsonde auf die zweite Vergleichsmenge an Messfluid verringert werden, um das zweite Vergleichs-Messwertpaar, das einer geringeren Menge an Messfluid in der Ballonsonde zugeordnet ist, zu erhalten. Erst danach kann in einem zweiten Schritt die vorhandene Menge an Messfluid in der Ballonsonde auf die erste Vergleichsmenge an Messfluid erhöht werden, um das erste Vergleichs-Messwertpaar, das einer größeren Menge an Messfluid in der Ballonsonde zugeordnet ist, zu erhalten.

In Ausführungsformen kann vorgesehen sein, dass das Fluidmengen-Überwachungssystem feststellt, dass die vorhandene Menge an Messfluid in der Ballonsonde der gewünschten oder betriebsgemäßen Menge an Messfluid in der Ballonsonde entspricht, wenn der bei dem ersten Vergleichs-Messwertpaar erfasste Ösophagusballondruck und der bei dem zweiten Vergleichs-Messwertpaar erfasste Ösophagusballondruck innerhalb eines vorbestimmten Bereichs um den dem Ist-Messwertpaar zugeordneten Ösophagusballondruck (d. h. dem der vorhandenen Menge an Messfluid in der Ballonsonde zugeordneten Ösophagusballondruck) liegt.

Es sei noch einmal erwähnt, dass mit dem Begriff "erfasster Ösophagusballondruck" insbesondere ein Differenzdruck zwischen dem erfassten Ösophagusballondruck am Ende der Inspirationsphase und dem erfassten Ösophagusballondruck am Ende der Exspirationsphase eines Beatmungszyklus gemeint sein soll. Dieser Differenzdruck soll bei der gewünschten Befüllung an Messfluid in der Ballonsonde (also bei dem Ist-Messwertpaar) maximal sein. Stellt das Fluidmengen-Überwachungssystem im Zuge der Überprüfungsprozedur fest, dass dieser Differenzdruck um einen vorbestimmten Wert oder mehr größer wird, wenn man die vorhandene Menge an Messfluid in der Ballonsonde verändert (d.h. vergrößert oder verkleinert), so ergibt sich daraus, dass die gewünschte oder betriebsgemäße Befüllung an Messfluid in der Ballonsonde nicht beim Ist-Messwertpaar liegt, sondern sich verschoben hat, und zwar in der Richtung, in der der Differenzdruck zunimmt. In diesem Fall können Gegenmaßnahmen vorgesehen sein. Diese Gegenmaßnahmen können davon abhängen, wie stark sich der bei dem ersten oder zweiten Vergleichs-Messwertpaar erfasste Differenzdruck von dem Ist-Messwertpaar zugeordneten Differenzdruck unterscheidet.

Beispielsweise kann vorgesehen sein, dass das Fluidmengen-Überwachungssystem feststellt, dass die vorhandene Menge an Messfluid in der Ballonsonde der gewünschten oder betriebsgemäßen Menge an Messfluid in der Ballonsonde entspricht, wenn ein erster Gradient, der durch den bei dem ersten Vergleichs-Messwertpaar (d.h. bei der ersten als Vergleichs-Messpunkt eingestellten Menge an Messfluid) erfassten Ösophagusballondruck und den bei dem Ist-Messwertpaar (d.h bei der vorhandenen Menge an Messfluid in der Ballonsonde) erfassten Ösophagusballondruck definiert ist, und ein zweiter Gradient, der durch den bei dem zweiten Vergleichs-Messwertpaar (d.h. bei der zweiten als Vergleichs-Messpunkt eingestellten Menge an Messfluid) erfassten Ösophagusballondruck und den bei dem Ist-Messwertpaar erfassten Ösophagusballondruck definiert ist, jedenfalls hinsichtlich seines Betrags einen vorbestimmten ersten Schwellenwert nicht überschreitet.

Der Gradient kann beispielsweise definiert sein als Differenz zwischen dem bei dem ersten Vergleichs-Messwertpaar oder bei dem zweiten Vergleichs-Messwertpaar erfassten Ösophagusballondruck und dem bei dem Ist-Messwertpaar erfassten Ösophagusballondruck, bezogen auf die jeweilige Differenz zwischen der bei dem ersten Vergleichs-Messwertpaar oder bei dem zweiten Vergleichs-Messwertpaar eingestellten Menge an Messfluid in der Ballonsonde und der bei dem Ist-Messwertpaar eingestellten Menge an Messfluid in der Ballonsonde. Ggf. kann der Gradient noch bezogen auf einen Referenzwert (z.B. den bei der vorhandenen Menge an Messfluid in der Ballonsonde erfassten Ösophagusballondruck) ausgedrückt sein. Alternativ könnte auch ein Verhältnis dieser Werte als Gradient herangezogen werden.

Der wie vorangehend erläutert definierte Gradient sollte dann sowohl für das erste Vergleichs-Messwertpaar als auch das zweite Vergleichs-Messwertpaar kleiner als Null sein, wenn die Menge an Messfluid in der Ballonsonde beim Ist-Messwertpaar, also bei der vorhandenen Menge an Messfluid in der Ballonsonde, immer noch der gewünschten oder betriebsgemäßen Menge an Messfluid entspricht, bei der die Druckdifferenz am Ende der Inspiration die Druckdifferenz am Ende der Exspiration maximal ist. Für den Fall, dass der Gradient für mindestens eines aus erstem Vergleichs-Messwertpaar und zweitem Vergleichs-Messwertpaar größer ist als Null, sollte dieser Wert für alle Messwertpaare jedenfalls kleiner sein als ein vorbestimmter erster Schwellenwert. Der vorbestimmte erste Schwellenwert kann dabei einen Druckdifferenz-Bereich definieren, innerhalb dem eine Änderung der Druckdifferenz als nicht signifikant angesehen wird.

Liegen beide der auf diese Weise erfassten Gradienten unterhalb des vorbestimmten ersten Schwellenwerts, kann man davon ausgehen, dass die vorhandene Menge an Messfluid in der Ballonsonde die gewünschte Befüllung darstellt, bzw. jedenfalls innerhalb eines Bereichs um die vorhandene Menge an Messfluid in der Ballonsonde liegt, der durch die für das erste Vergleichs-Messwertpaar bzw. das zweite Vergleichs-Messwertpaar eingestellte Menge an Messfluid definiert ist. In diesem Fall sind keine Maßnahmen veranlasst. Insbesondere kann die vorhandene Menge an Messfluid in der Ballonsonde weiterhin als die gewünschte oder betriebsgemäße Menge an Messfluid in der Ballonsonde herangezogen werden. Der vorbestimmte erste Schwellenwert kann beispielsweise Null sein oder ein Wert geringfügig größer als Null.

In weiteren Ausführungsformen kann vorgesehen sein, dass das Fluidbefüllungssystem derart ausgebildet ist, dass es die vorangehend beschriebene Befüllungssteuerung zur Durchführung der Prozedur zur Befüllung der Ballonsonde mit einer vorbestimmten Menge an Messfluid aktiviert oder ansteuert, wenn das Fluidmengen-Überwachungssystem feststellt, dass wenigstens einer aus dem ersten Gradient und dem zweiten Gradient größer ist als der erste Schwellenwert.

In diesem Fall wird insbesondere das Fluidmengen-Überwachungssystem veranlassen, dass die Befüllungssteuerung zur Durchführung der Fluid-Befüllungsprozedur zum Befüllen der Ballonsonde eine neue vorbestimmte Befüllung für die Ballonsonde einstellt, die gegenüber der vorhandenen Menge an Messfluid in der Ballonsonde in Richtung zu der derjenigen Menge an Messfluid in der Ballonsonde aus erstem und zweitem Vergleichs-Messwertpaar verschoben ist, für die der größere Gradient bestimmt wurde. Es wird also für die zukünftige Beatmung eine neue gewünschte oder betriebsgemäße Befüllung der Ballonsonde festgelegt und eingestellt.

Beispielsweise kann die neue gewünschte oder betriebsgemäße Befüllung der Ballonsonde der Menge an Messfluid in der Ballonsonde entsprechen, die demjenigen aus erstem Vergleich-Messwertpaar und zweitem Vergleichs-Messwertpaar zugeordnet ist, für das der größere Gradient bestimmt wurde.

Ferner kann vorgesehen sein, dass das Kalibriersystem derart ausgebildet ist, dass es die vorangehend beschriebene Kalibriersteuerung zur Durchführung der Kalibrierprozedur zur Neu-Einstellung einer gewünschten oder betriebsgemäßen Befüllung der Ballonsonde mit Messfluid aktiviert oder ansteuert, wenn das Fluidmengen-Überwachungssystem feststellt, dass wenigstens einer aus erstem Gradient und zweitem Gradient größer ist als ein zweiter Schwellenwert, der größer ist als der erste Schwellenwert.

Wenn die durch das Fluidmengen-Überwachungssystem festgestellten erfassten Ösophagusballondrücke zu weit auseinander liegen, ist davon auszugehen, dass sich die Verhältnisse, denen die Ballonsonde ausgesetzt ist, so stark geändert haben, dass eine neue Kalibrierung wünschenswert ist.

In weiteren Ausführungsformen kann vorgesehen sein, dass das Ösophagusballonsteuersystem dazu ausgebildet ist, in Reaktion auf eine Veränderung eines Beatmungsparameters die Fluidmengenüberwachung zum Durchführen einer Prozedur zur Überprüfung einer vorbestimmten Befüllung an Messfluid in der Ballonsonde anzusteuern. Auf diese Weise kann sichergestellt werden, dass nach Veränderung eines Beatmungsparameters, wie z.B. PEEP, maximaler Atemwegsdruck, Tidalvolumen, oder Beatmungsfrequenz überprüft wird, ob diese Veränderung der Beatmungsbedingungen oder gar des Beatmungsmodus eine Auswirkung auf die jeweils gewünschte oder betriebsgemäße Befüllung der Ballonsonde des Ösophagusballonkatheters genommen hat. Sollte ein solcher Einfluss festgestellt werden, so kann umgehend eine Korrektur der Befüllung vorgenommen werden, oder sogar eine vollständige Neu-Kalibrierung des Ösophagusballonkatheters durchgeführt werden. Die Beatmung kann dabei in automatisierter Form weiterlaufen, ohne dass manuelle Eingriffe von Seiten des Bedienpersonals erforderlich werden.

Trotzdem bleiben die von dem Ösophagusballonkatheter gelieferten Informationen, insbesondere hinsichtlich des herrschenden Drucks im Pleuraspalt, immer so genau wie möglich.

Auch die hier beschriebene Möglichkeit der Bereitstellung eines Fluidmengen-Überwachungssystem für einen Ösophaguskatheter mit Ballonsonde sowie die verschiedenen möglichen Ausführungsformen eines solchen Fluidmengen-Überwachungssystems stellen nach Ansicht der Anmelderin einen eigenständig schützenswerten Beitrag zum Stand der Technik dar. Dieser Beitrag ist insbesondere unabhängig von weiteren Merkmalen zu sehen, die im Anspruch 1 enthalten sind oder vorangehend näher erläutert worden sind. Die Anmelderin behält sich vor, im weiteren Verlauf des Prüfungsverfahrens zu der vorliegenden Erfindung eigenständige Ansprüche zu formulieren, die auf das Fluidbefüllungssystem gerichtet sind, oder gegebenenfalls solche Ansprüche in einer Teilanmeldung zu verfolgen.

Die vorliegende Offenbarung betrifft darüber hinaus auch eine Vorrichtung zur maschinellen Beatmung, welche eine Vorrichtung zur automatisierten Einstellung eines durch die Beatmungsvorrichtung vorgegebenen Beatmungsparameters, insbesondere eines Drucks, insbesondere eines positiven endexpiratorischen Drucks (PEEP) und/oder eines maximalen Atemwegsdrucks (Paw_max) und/oder zur automatisierten Darstellung von für maschinelle Beatmung relevanter Information nach einer der vorangehend dargelegten Ausführungsformen aufweist.

In einem weiteren Aspekt betrifft die vorliegende Offenbarung ein Ösophagusballonsteuersystem für einen in den Ösophagus eines Patienten einführbaren Ösophagusballonkatheter mit Ballonsonde zur Bestimmung eines Ösophagusballondrucks. Ein solches Ösophagusballonsteuersystem ist insbesondere zum Einsatz in einer Vorrichtung zur automatisierten Einstellung eines durch eine Beatmungsvorrichtung vorgegebenen Beatmungsparameters, insbesondere eines Drucks, insbesondere eines positiven endexpiratorischen Drucks (PEEP) und/oder eines maximalen Atemwegsdrucks (Paw_max), und/oder zur automatisierten Darstellung von für maschinelle Beatmung relevanter Information, nach einem der vorangehend dargelegten Ausführungsbeispiele vorgesehen und ausgebildet.

Ein solches Ösophagusballonsteuersystem umfasst demgemäß eine Anordnung, insbesondere einen Sensor, zum Erfassen des Ösophagusballondrucks und Weitergeben des erfassten Ösophagusballondrucks an ein Druckermittlungssystem zum Ermitteln eines transpulmonalen Drucks, insbesondere eines transpulmonalen Drucks am Ende einer Exspirationsphase und/oder eines transpulmonalen Drucks am Ende einer Inspirationsphase, auf Grundlage des erfassten Ösophagusballondrucks. Das Ösophagusballonsteuersystem ist zur automatisierten Überwachung und/oder Einstellung einer betriebsgemäßen Befüllung der Ballonsonde des Ösophagusballonkatheters in vivo ausgebildet und ist ferner zu bidirektionaler Interaktion mit einer Beatmungssteuerung der Beatmungsvorrichtung ausgebildet.

Ein solches Ösophagusballonsteuersystem kann als eigenständiges Bauteil "in Hardware" realisiert werden. Alternativ kann ein solches Ösophagusballonsteuersystem auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocomputer kann zu einer Steuerung eines Ösophagusballonkatheters mit Ballonsonde gehören und/oder je nach Ausgestaltung des Ösophagusballonkatheters der Beatmungssteuerung einer Vorrichtung zur maschinellen Beatmung zugeordnet sein.

Insbesondere kann das Ösophagusballonsteuersystem so ausgebildet sein, dass es nach Maßgabe von durch die Beatmungssteuerung der Beatmungsvorrichtung gelieferten Steuerbefehlen arbeitet.

Das Ösophagusballonsteuersystem kann ferner so ausgebildet sein, dass es vorbestimmte Ösophagusballonsteuersignale an die Beatmungssteuerung sendet. Das bedeutet, dass die Beatmungssteuerung nicht nur zum Empfang von den Ösophagusballondruck und/oder den transpulmonalen Druck repräsentierenden Signalen von dem Ösophagusballonsteuersystem ausgebildet ist, sondern auch zum Empfang von Steuersignalen von dem Ösophagusballonsteuersystem. Ferner kann die Beatmungssteuerung zusätzlich noch zur automatisierten Ansteuerung des Ösophagusballonsteuersystems ausgebildet sein, jedenfalls im Hinblick auf Anstoßen und/oder Durchführung bestimmter Funktionen und/oder Prozeduren des Ösophagusballonsteuersystems. Dabei kann das Ösophagusballonsteuersystem insbesondere wenigstens eines oder eine Kombination mehrerer der mit Bezug auf die vorangehend beschriebenen Ausführungsformen genannten Merkmale aufweisen, soweit sich diese Merkmale zur Charakterisierung eines Ösophagusballonsteuersystems geeignet sind.

Gemäß einem weiteren Aspekt betrifft die vorliegende Offenbarung auch einen Ösophagusballonkatheter zur Bestimmung eines Ösophagusballondrucks in vivo nach Einführen in den Ösophagus eines Patienten. Ein solcher Ösophagusballonkatheter ist insbesondere zum Einsatz in einer Vorrichtung zur automatisierten Einstellung eines durch eine Beatmungsvorrichtung vorgegebenen Beatmungsparameters, insbesondere eines Drucks, insbesondere eines positiven endexpiratorischen Drucks (PEEP) und/oder eines maximalen Atemwegsdrucks (Paw_max), und/oder zur automatisierten Darstellung von für maschinelle Beatmung relevanter Information nach einem der vorangehend beschriebenen Ausführungsbeispiele bestimmt und ausgebildet. Der Ösophaguskatheter umfasst einen in den Ösophagus des Patienten einführbaren Ösophagusballonkatheter mit Ballonsonde zur Bestimmung des Ösophagusballondrucks und ein Ösophagusballonsteuersystem der vorangehend beschriebenen Art.

Das Ösophagusballonsteuersystem ist bestimmungsgemäß dazu konfiguriert, Signale, die den erfassten Ösophagusballondruck repräsentieren, für die Beatmungssteuerung zur Verfügung zu stellen. Dementsprechend ist die Beatmungssteuerung bestimmungsgemäß dazu konfiguriert, solche Signale von dem Ösophagusballonsteuersystem zu empfangen.

Offenbarungsgemäß ist die Beatmungssteuerung aber auch zur automatisierten Ansteuerung des Ösophagusballonsteuersystems ausgebildet. In diesem Sinn ist die Beatmungssteuerung zu bidirektionaler Interaktion mit dem Ösophagusballonsteuersystem ausgebildet. Dabei kann die Beatmungssteuerung das Ösophagusballonssteuersystem zum Start von vorbestimmten Prozeduren ansteuern. Zusätzlich oder alternativ kann die Beatmungssteuerung den Ablauf von vorbestimmten Prozeduren im Ösophagusballonssteuersystem steuern. Auf diese Weise lässt sich ein optimales Zusammenspiel der beiden Steuersysteme Beatmungssteuerung und Ösophagusballonssteuersystem verwirklichen, so dass die von den beiden Systemen ausgeführten Prozeduren jeweils aufeinander abgestimmt und synchronisiert ablaufen.

Aus diesem Grund betrifft gemäß einem weiteren Aspekt die vorliegende Offenbarung auch eine Beatmungssteuerung für eine Vorrichtung zur automatisierten Einstellung eines durch eine Beatmungsvorrichtung vorgegebenen Beatmungsparameters, insbesondere eines Drucks, insbesondere eines positiven endexpiratorischen Drucks (PEEP) und/oder eines maximalen Atemwegsdrucks (Paw_max), und/oder zur automatisierten Darstellung von für maschinelle Beatmung relevanter Information, insbesondere gemäß einem der vorangehend dargelegten Ausführungsbeispiele. Die Beatmungssteuerung ist insbesondere zur Einstellung des durch die Beatmungsvorrichtung vorgegebenen Beatmungsparameters auf Grundlage eines mittels eines Ösophagusballonkatheters ermittelten transpulmonalen Drucks ausgebildet.

Die Beatmungssteuerung kann ein Druckermittlungssystem umfassen zum Ermitteln eines transpulmonalen Drucks, insbesondere eines transpulmonalen Drucks am Ende einer Exspirationsphase und/oder eines transpulmonalen Drucks am Ende einer Inspirationsphase, auf Grundlage des in den Ösophagus eines Patienten einführbaren Ösophagusballonkatheters mit Ballonsonde zur Bestimmung eines Ösophagusballondrucks.

Die Beatmungssteuerung kann zu bidirektionaler Interaktion mit dem Ösophagusballonsteuersystem ausgebildet sein, derart dass die Beatmungssteuerung das Ösophagusballonsteuersystem ansteuert, insbesondere nach Maßgabe von durch das Druckermittlungssystem gelieferten Druckwerten.

Die Beatmungssteuerung kann derart konfiguriert sein, dass sie vorbestimmte Beatmungsparameter und/oder Beatmungsmodi nach Maßgabe von dem Ösophagusballonsteuersystem gelieferten Ösophagusballonsteuersignalen einstellt/steuert/regelt.

Es versteht sich, dass die Beatmungssteuerung insbesondere eines oder eine Kombination mehrerer der mit Bezug auf die vorangehend erläuterten Ausführungsbeispiele genannten Merkmale einer Beatmungssteuerung aufweisen kann.

Dies betrifft insbesondere die mit Bezug zu den anderen Ausführungsbeispielen näher dargelegte Druckermittlungsanordnung.

Gemäß einem weiteren Aspekt betrifft die Erfindung ferner eine Beatmungsvorrichtung, aufweisend eine Beatmungssteuerung wie vorangehend dargelegt.

Gemäß einem weiteren Aspekt betrifft die vorliegende Offenbarung darüber hinaus ein Verfahren zur automatisierten Einstellung eines durch eine Beatmungsvorrichtung vorgegebenen Beatmungsparameters, insbesondere eines Drucks, insbesondere eines positiven endexpiratorischen Drucks (PEEP) und/oder eines maximalen Atemwegsdrucks (Paw_max), und/oder zur automatisierten Darstellung von für maschinelle Beatmung relevanter Information, insbesondere eines transpulmonalen Drucks (Ptp).

Das Verfahren umfasst folgende Schritte:
- Ermitteln eines transpulmonalen Drucks, insbesondere eines transpulmonalen Drucks am Ende einer Exspirationsphase und/oder eines transpulmonalen Drucks am Ende einer Inspirationsphase, auf Grundlage eines in den Ösophagus einführbaren Ösophagusballonkatheters mit Ballonsonde zur Bestimmung eines Ösophagusballondrucks durch ein Druckermittlungssystem;
- automatisierte Überwachung und/oder Einstellung einer betriebsgemäßen Befüllung der Ballonsonde des Ösophagusballonkatheters in vivo durch ein Ösophagusballonsteuersystem; und
- Einstellen des durch die Beatmungsvorrichtung vorgegebenen Beatmungsparameters und/oder Darstellen der für maschinelle Beatmung relevanten Information auf Grundlage des durch das Druckermittlungssystem ermittelten transpulmonalen Drucks durch eine Beatmungssteuerung.

Bei dem offenbarungsgemäßen Verfahren ist vorgesehen, dass die Beatmungssteuerung und das Ösophagusballonsteuersystem bidirektional miteinander interaqieren. Bidirektional miteinander interagieren soll insbesondere bedeuten, dass die Beatmungssteuerung auch zur automatisierten Ansteuerung des Ösophagusballonsteuersystems ausgebildet ist und/oder das Ösophagusballonsteuersystem auch zur automatisierten Ansteuerung der Beatmungssteuerung ausgebildet ist.

Zum Ermitteln des transpulmonalen Drucks kann ein Druckermittlungssystem vorgesehen sein, das der Beatmungssteuerung des Beatmungsgeräts und/oder dem Ösophagusballonsteuersystem des in den Ösophagus einführbaren Ösophagusballonkatheters mit Ballonsonde zur Bestimmung eines Ösophagusballondrucks zugeordnet ist.

Zur Vermeidung von Wiederholungen wird bezüglich weiterer Ausgestaltungen des offenbarungsgemäßen Verfahrens auf die näheren Erläuterungen zu den vorangehenden Ausführungsbeispielen verwiesen, die sinngemäß auch in Bezug auf das hier beanspruchte Verfahren gelten.

Weitere Ausführungsformen der vorliegenden Offenbarung werden nachfolgend unter Bezugnahme auf die Figuren näher erläutert.
Fig. 1 zeigt in stark schematisierter Darstellung in Form eines Blockdiagramms die wesentlichen Elemente einer Beatmungsvorrichtung mit einer Ausführungsform einer offenbarungsgemäßen Vorrichtung, samt intubierter Trachea und Thorax eines beatmeten Patienten;
Fig. 2 zeigt den zeitlichen Verlauf des Atemwegeingangsdrucks Paw (oben), Ösophagusballondrucks Peso (Mitte) und der Differenz Paw - Peso aus beiden Drücken während mehrerer aufeinander folgender Atemzyklen bei maschineller Beatmung einschließlich Okklusionsmanövern;
Fig. 3 zeigt anhand eines Flussdiagramms den Ablauf einer Kalibrierung eines Ösophagusballonkatheters in vivo gemäß einer Ausführungsform der vorliegenden Offenbarung.
Fig. 4 zeigt schematisch die während einer Kalibrierung gemäß einer Ausführungsform der vorliegenden Offenbarung eingestellte Menge an Messfluid in der Ballonsonde des Ösophagusballonkatheters sowie den erfassten Druck in der Ballonsonde des Ösophagusballonkatheters; und
Fig. 5 zeigt in den Teilbildern a) und b) schematisch für jede eingestellte Menge an Messfluid in der Ballonsonde während eines Messzyklus den jeweils erfassten Druck in der Ballonsonde am Ende der Inspirationsphase, den jeweils erfassten Druck in der Ballonsonde am Ende der Exspirationsphase (Teilbild a)), sowie den sich daraus jeweils ergebenden Differenzdruck zwischen Druck in der Ballonsonde am Ende der Inspirationsphase und Druck in der Ballonsonde am Ende der Exspirationsphase (Teilbild b)).
Fig. 6 zeigt anhand eines Flussdiagramms den Ablauf einer Überwachung der Fluidmenge in einem Ösophagusballonkatheter in vivo gemäß einer Ausführungsform der vorliegenden Offenbarung.
Fig. 7 zeigt anhand eines Flussdiagramms den Ablauf einer Befüllung eines Ösophagusballonkatheters gemäß einer Ausführungsform der vorliegenden Offenbarung.

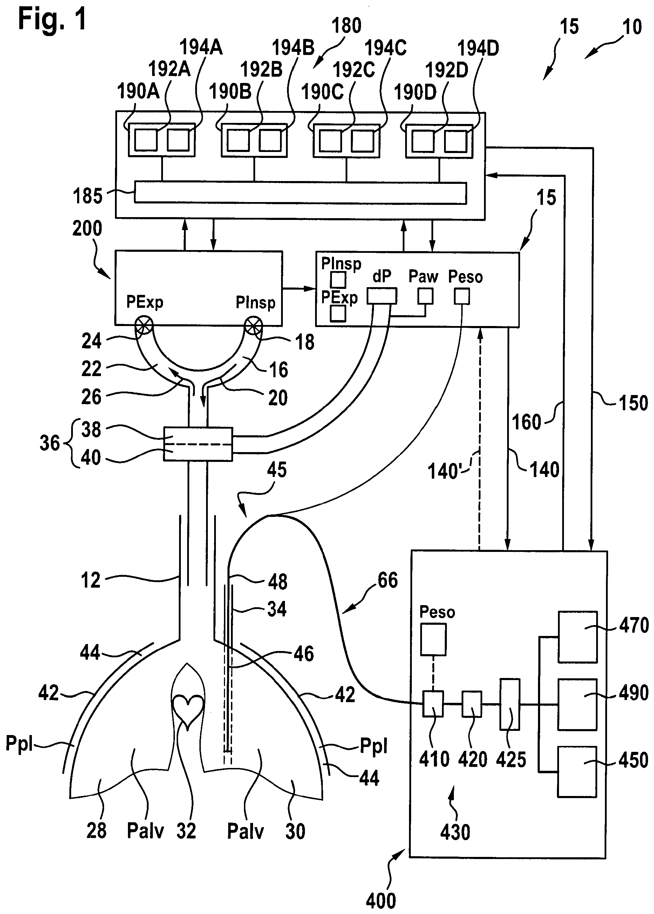

Fig. 1 zeigt in stark schematisierter Darstellung und in Form eines Blockdiagramms die wesentlichen Elemente einer Beatmungsvorrichtung 10 samt intubierter Trachea 12 und Thorax eines beatmeten Patienten. Die Beatmungsvorrichtung 10 weist eine Vorrichtung 15 zur Einstellung eines durch die Beatmungsvorrichtung 10 vorgegebenen Beatmungsparameters und/oder zur automatisierten Darstellung von für maschinelle Beatmung relevanter Information auf Grundlage eines Ösophagusballondrucks, gemäß einer Ausführungsform der vorliegenden Offenbarung auf.

Die Beatmungsvorrichtung 10 ist in Fig. 1 in einem Zustand mit intubierter Luftröhre (Trachea) 12 eines beatmeten Patienten gezeigt. Neben der Trachea 12 sind in Fig. 1 noch die Lungenlappen 28, 30, das Herz 32, die Speiseröhre (Ösophagus) 34 und die Wand des Thorax 42 des Patienten sehr schematisch angedeutet. Der Tubus 14 der Beatmungsvorrichtung 10 ist, in der Regel über die nicht gezeichnete Mundöffnung des Patienten, ein Stück weit in die Trachea 12 eingeschoben. um den Atemweg mit Atemgas zu beaufschlagen. Ausgeatmete Luft wird ebenfalls über den Tubus 14 abgeleitet, der sich an seinem stromaufwärtigen bzw. distalen Ende in ein erstes Ende 16 und ein zweites Ende 22 verzweigt. Das erste Ende 16 ist über ein Atemwegeingangsventil 18 mit einem Atemwegeingangsanschluss eines Atemgassystems 200 der Beatmungsvorrichtung 10 verbunden. Über den Atemwegeingangsanschluss wird während einer Inspirationsphase Atemgas zur Beatmung unter einem als Inspirationsdruck Plnsp bezeichneten Überdruck in die Lunge geleitet. Deshalb ist während der Inspirationsphase in Öffnungsstellung des Atemwegeingangsventils 18 der Atemwegeingangsanschluss mit dem Inspirationsdruck Plnsp beaufschlagt, der von dem Atemgassystem 200 nach Maßgabe von Steuerbefehlen von einer Beatmungssteuerung 180 erzeugt wird. Das zweite Ende 22 ist über ein Atemwegausgangsventil 24 mit einem Atemwegausgangsanschluss des Atemgassystems 200 der Beatmungsvorrichtung 10 verbunden. In Öffnungsstellung des Atemwegausgangsventils 24 ist der Atemweg mit dem Exspirationsdruck Pexp beaufschlagt. Sowohl der Inspirationsdruck Plnsp als auch der Exspirationsdruck Pexp werden von einem Druckermittlungssystem 300 der Beatmungsvorrichtung 10 erfasst.

Das Druckermittlungssystem 300 weist eine zur Bestimmung des Ösophagusballondrucks ausgelegte Sensorik auf, die einen in den Ösophagus 34 einführbaren Ösophagusballonkatheter 45 mit Ballonsonde 46 und wenigstens eine Vorrichtung zum Erfassen eines Drucks in der Ballonsonde 46 in vivo umfasst. Das Druckermittlungssystem 300 kann insbesondere eine zur Bestimmung eines alveolären Drucks Palv ausgelegte Sensorik aufweisen und den jeweiligen transpulmonalen Druck Ptp anhand einer Differenz zwischen dem jeweiligen alveolären Druck Palv und dem jeweiligem Ösophagusballondruck Peso ermitteln. Die Sensorik zur Bestimmung des alveolären Drucks Palv kann zur Bestimmung des Atemwegswiderstands R ausgelegt sein und darüber hinaus einen Sensor zum Erfassen des Gasflusses im Atemweg umfassen, insbesondere am Ende einer Exspirationsphase und/oder am Ende einer Inspirationsphase. Dann kann die Sensorik den jeweiligen alveolären Druck Palv anhand des jeweiligen Atemasflusses sowie des Atemwegswiderstands R bestimmen. Die Sensorik zur Bestimmung des alveolären Drucks Palv kann beispielsweise einen an einem Tubusanfang der Beatmungsvorrichtung 10 angeordneten oder dem Atemwegeingangsventil 18 der Beatmungsvorrichtung 10 zugeordneten Atemwegsdrucksensor zur Erfassung des eingangsseitigen Atemwegsdrucks umfassen.

Der Inspirationsdruck Plnsp wird von der Beatmungsvorrichtung 10, insbesondere von dem Atemgassystem 200 nach Maßgabe von Steuerbefehlen von der Beatmungssteuerung 180, erzeugt, derart dass einzuatmendes Atemgas während der Inspirationsphase nach vorgegebenen zeitlichen Mustern in Richtung der Lunge 28, 30 des Patienten strömt, wie durch den Pfeil 20 in Fig. 1 angedeutet. Die Exspiration kann durch passives Ausströmenlassen von Atemgas aus der Lunge 28, 30 zum Atemwegausgangsanschluss während einer Exspirationsphase erfolgen, wie durch den Pfeil 26 angedeutet. Der Exspirationsdruck Pexp ergibt sich dann während der Exspirationsphase anhand physiologischer Eigenschaften des Patienten und dem am Anfang der Exspirationsphase herrschenden maximalen Inspirationsdruck. Gegebenenfalls kann die Exspiration auch durch die Beatmungsvorrichtung 10, insbesondere durch das Atemgassystem 200 nach Maßgabe von Steuerbefehlen von der Beatmungssteuerung 180, unterstützt oder sogar vollständig erzeugt werden, so dass ein einem vorgegebenen zeitlichen Muster entsprechender Verlauf der Atemgasströmung und/oder des Exspirationsdruck Pexp während der Exspirationsphase ergibt.

Normalerweise bleibt während der Inspirationsphase das Atemwegeingangsventil 18 geöffnet und das Atemwegausgangsventil 24 geschlossen. Der Atemwegeingang wird mit dem Inspirationsdruck Plnsp beaufschlagt. Während der Exspirationsphase ist das Atemwegeingangsventil 18 geschlossen und das Atemwegausgangsventil 24 ist geöffnet. Dann ist der Atemweg mit dem Exspirationsdruck Pexp beaufschlagt.

Das Atemgas kann Umgebungsluft enthalten, wird aber in der Regel einen vorbestimmten Anteil reinen Sauerstoff enthalten, der über dem Sauerstoffanteil der Umgebungsluft liegt. Das Atemgas kann außerdem befeuchtet sein.

Der Fluss von Atemgas am Atemwegeingang während einer jeweiligen Inspirationsphase wird mit Hilfe eines Atemwegeingangsflusssensors 36 bestimmt. Der Atemwegeingangsflusssensor 36 beruht auf der Erfassung einer Druckdifferenz dP zwischen einem Eingangsvolumen 38 und einem mit dem Eingangsvolumen 38 in Verbindung stehenden Ausgangsvolumen 40, und liefert eine Bestimmung des Atemgasmassenflusses am Atemwegeingang. Aus dem Drucksignal im Ausgangsvolumen 40 lässt sich zugleich recht einfach der Wert des Atemwegeingangsdrucks Paw ableiten. Die Druckdifferenz dP zwischen dem Eingangsvolumen 38 und dem Ausgangsvolumen 40 sowie der Atemwegeingangsdrucks Paw werden ebenfalls von dem Druckermittlungssystem 300 der Beatmungsvorrichtung 10 erfasst.

Der Atemwegeingangsflusssensor 36 dient auch zur Erfassung des Atemgasflusses F und Atemwegsausgangsdrucks Paw am Atemwegausgang während einer jeweiligen Exspirationsphase. Es wird insofern auf die vorangehende Beschreibung verwiesen mit der Maßgabe, dass infolge der nunmehr umgekehrten Richtung der Atemgasströmung Eingangsvolumen und Ausgangsvolumen ihre Rollen vertauschen, also das mit 40 bezeichnete Volumen das Eingangvolumen ist und das mit 38 bezeichnete Volumen das Ausgangsvolumen ist.

Die Steuerung von einzuatmendem Atemgas während der Inspirationsphase und von auszuatmendem Atemgas während der Exspirationsphase erfolgt durch eine Beatmungssteuerung 180 der Beatmungsvorrichtung 10. Die Beatmungssteuerung 180 steht in Kommunikationsverbindung mit dem Atemgassystem 200, um die Beaufschlagung des Atemwegs mit dem Inspirationsdruck Plnsp bzw. Exspirationsdruck Pexp zu steuern/regeln. Falls gewünscht, kann die Beatmungssteuerung 180 auch vom Atemgassystem 200 Signale empfangen, so dass die Kommunikationsverbindung zwischen der Beatmungssteuerung 180 und dem Atemgassystem 200 bidirektional ist. Die Beatmungssteuerung 180 steht in Kommunikationsverbindung mit dem Druckermittlungssystem 300, um Signale zu empfangen, die für die Beatmung relevante Größen, insbesondere Signale von Drucksensoren, zu empfangen. Beispielsweise kann die Beatmungssteuerung 180 vom Druckermittlungssystem 300 Signale bezüglich wenigstens einer der folgenden Größen empfangen: Inspirationsdruck Pinsp, Exspirationsdruck Pexp, Atemgasfluss F am Atemwegseingang, Atemwegsdruck Paw, sowie einen nachfolgend noch näher erläuterten Ösophagusballondruck Peso. Falls gewünscht, kann die Beatmungssteuerung 180 auch Signale, insbesondere Steuersignale, zum Druckermittlungssystem 300 senden, so dass die Kommunikationsverbindung zwischen Beatmungssteuerung 180 und Druckermittlungssystem bidirektional ist.

Die Beatmungssteuerung 180 ist in Fig. 1 lediglich schematisch als Block 180 angedeutet und wird in der vorliegenden Beschreibung lediglich insoweit näher erläutert, als ihre Funktion zum Verständnis der vorliegenden Offenbarung erforderlich ist.

Hinsichtlich weiterer Aspekte der Beatmungssteuerung 180 wird auf bekannte Beatmungsvorrichtungen verwiesen, insbesondere auf solche Beatmungsvorrichtungen, die über eine Steuerung der Beatmung in verschiedenen Beatmungsformen oder Beatmungsmodi verfügen. Beispielsweise sei auf bekannte Beatmungsvorrichtungen verwiesen, die über Beatmungsmodi verfügen, bei denen die Beatmung mittels geschlossener Regelkreise gesteuert wird, wie im ASV-Beatmungsmodus ("Automatic Support Ventilation") sowie im INTELLiVENT-ASV-Beatmungsmodus bei Beatmungsvorrichtungen der Anmelderin.

Entsprechendes gilt auch in Bezug auf die anderen in Fig. 1 angedeuteten Teilsysteme Atemgassystem 200, Druckermittlungssystem 300, Ösophagusballonsteuersystem 400.

Die Darstellung von Beatmungssteuerung 180, Atemgassystem 200, Druckermittlungssystem 300 und Ösophagusballonsteuersystem 400 in Fig. 1 als jeweils separate Systeme ist lediglich beispielhaft zu verstehen und wurde zum Zwecke der besseren Darstellung der dem jeweiligen System zugeordneten Funktionen gewählt. Obwohl im Folgenden nicht mehr explizit ausgeführt, versteht es sich, dass in praktisch realisierten Ausführungsformen mehrere dieser Systeme, oder sogar alle dieser Systeme, zu einem die jeweiligen Funktionen umfassenden Gesamtsystem zusammengefasst sein können.

Im Zusammenhang mit der vorliegenden Offenbarung sind jegliche Formen bekannter Beatmungsformen bzw. Beatmungsmodi einsetzbar, beispielsweise druckkontrollierte Beatmungsformen, volumenkontrollierte Beatmungsformen oder auch Beatmungsformen, bei denen druckkontrollierte und volumenkontrollierte Aspekte kombiniert sind. Neben rein maschinenkontrollierten Beatmungsformen, bei denen der zeitliche Verlauf des Inspirationsdrucks Plnsp und ggf. auch des Exspirationsdrucks Pexp von der Beatmungsvorrichtung 10, insbesondere von der Beatmungssteuerung 180, bestimmt werden, sind auch Beatmungsformen denkbar, in denen Spontanatmungsbemühungen des Patienten entweder die maschinelle Beatmung unterstützten können oder die maschinelle Beatmung zur Unterstützung von Spontanatmungsbemühungen des Patienten dient. In solchen Beatmungsformen werden der zeitliche Verlauf von Inspirationsdruck Plnsp bzw. Exspirationsdruck Pexp sowie häufig ebenfalls die Stellung von Atemwegseingangsventil 18 bzw. Ausgangsventil 24 nicht allein von der Beatmungsvorrichtung 10 vorgegeben, sondern die Beatmungssteuerung 180 ist derart konfiguriert, dass Spontanatmungsbemühungen des Patienten auf Größen wie den zeitlichen Verlauf von Inspirationsdruck Plnsp und/oder Exspirationsdruck Pexp, Stellung von Atemwegseingangsventil 18 bzw. Ausgangsventil 24, u.Ä. Einfluss nehmen können.

In Fig. 1 ist schematisch dargestellt, dass die Beatmungssteuerung 180 eine Koordinationseinheit 185 aufweist, welche anhand von Benutzereinstellungen und/oder erfassten Signalen einen aus einer Mehrzahl von Beatmungsmodi 190A, 190B, 190C, 190D als aktiven Beatmungsmodus auswählt und anhand von Steuersignalen, die von dem jeweils aktiven der Beatmungsmodus 190A, 190B, 190C, 190D geliefert werden, das Atemgassystem 200 zur Beaufschlagung des Atemwegs mit dem jeweiligen Inspirationsdruck Plnsp bzw. Exspirationsdruck Pexp steuert/regelt.

Ein jeder der Beatmungsmodi 190A, 190B, 190C und 1900 umfasst eine Einheit zur Einstellung von Beatmungsparametern (wie den positiven endexpiratorischen Druck PEEP oder den maximalen Atemwegsdruck Paw_max), die in Fig. 1 jeweils mit den Bezugszeichen 192A, 192B, 192C und 192D bezeichnet sind. Ein jeder der Beatmungsmodi 190A, 190B, 190C und 190D umfasst zudem eine Einheit zur automatisierten Darstellung von für die Beatmung relevanten Informationen und/ oder Einstellungen, wie z.B. dem transpulmonalen Druck Ptp, die in Fig. 1 jeweils mit den Bezugszeichen 194A, 194B, 194C und 194D bezeichnet sind.

Die Koordinationseinheit 185 sorgt ebenfalls für die Einstellung der von dem jeweils aktiven Beatmungsmodus 190A, 190B, 190C, 190D festgelegten Werte für die Beatmungsparameter 192A, 192B, 192C, 192D bzw. für die Darstellung der von dem jeweils aktiven Beatmungsmodus festgelegten Informationen und/oder Einstellungen 194A, 194B, 194C und 194D auf einem Display, Anzeigegerät oder einer anderen Ausgabevorrichtung der Beatmungsvorrichtung 10. Die Ausgabevorrichtung ist in Fig. 1 nicht explizit gezeigt. Die Koordinationseinheit 185 ist auch für das Umschalten zwischen verschiedenen der Beatmungsmodi 190A, 190B, 190C und 190D zuständig. Die jeweils einzustellenden Beatmungsparameter 192A, 192B, 192C, 192D bzw. deren Parameterwerte sowie die jeweils darzustellende Informationen 194A, 194B, 194C und 194D ändern sich bei einem Wechsel zwischen den Beatmungsmodi 190A, 190B, 190C, 190D entsprechend.

In Fig. 1 ist in schematischer Weise ein zusätzlicher Ösophagusballonkatheter 45 mit Ballonsonde 46 zur Messung des als Ösophagusballondrucks oder ösophagealer Druck bezeichneten Drucks im Ösophagus (Speiseröhre) 34 angedeutet. Die Ballonsonde 46 hat die Form eines Ballons und ist an einem in den Ösophagus 34 einführbaren Katheterschlauch 48 angebracht. Fig. 1 zeigt schematisch den in den Ösophagus 34 eines zu beatmenden Patienten eingeführten Ösophagusballonkatheter 45 mit Ballonsonde 46 in seiner Messstellung. In dieser Messstellung ist der Ösophagusballonkatheter 45 zur Erfassung eines Ösophagusballondrucks Peso in der Ballonsonde 46 ausgebildet, aus dem auf den transpulmonalen Druck Ptp rückgeschlossen werden kann. Die Ballonsonde 46 liegt innen an der Wand des Ösophagus 34 an. Der Ösophagusballondruck Peso in der Ballonsonde 46 liefert nach einer geeigneten Kalibrierung den in auf den Ösophagus 34 am Ort der Ballonsonde 46 einwirkenden ösophagealer Druck. Der ösophageale Druck entspricht bei geeigneter Lagerung des Patienten in guter Näherung dem Druck Ppl im Pleuraspalt. Die Ballonsonde 46 zur Bestimmung des ösophagealen Drucks und der Umgang mit dieser Sonde ist beispielsweise in Benditt J., Resp. Care, 2005, 50: S. 68-77 beschrieben.

Dem Ösophagusballonkatheter 45 ist ein Ösophagusballonsteuersystem 400 zugeordnet, welches zur Überwachung, Kalibrierung und/oder Befüllung der Ballonsonde 46 des Ösophagusballonkatheters 45 mit einem Messfluid ausgebildet ist. Die Beatmungsvorrichtung 10 und das Ösophagusballonsteuersystem 400 sind derart ausgebildet, dass eine interaktive Kommunikation zwischen der Beatmungssteuerung 180 und dem Ösophagusballonsteuersystem 400 besteht. Das bedeutet, dass die Beatmungssteuerung 180 auch zur automatisierten Ansteuerung des Ösophagusballonsteuersystems 400 ausgebildet ist und/oder dass das Ösophagusballonsteuersystem 400 auch zur automatisierten Ansteuerung die Beatmungssteuerung 180 ausgebildet ist.

In Fig. 1 ist diese interaktive Kommunikation zwischen der Beatmungssteuerung 180 und Ösophagusballonsteuersystem 400 durch die Pfeile 150 und 160 dargestellt. Eine derartige Konfiguration ist insbesondere auf Beatmungsformen zugeschnitten, bei denen die Beatmung mittels vollautomatischer Beatmungsmodi erfolgt, beispielsweise bei Beatmung mittels geschlossener Regelkreise, wie sie etwa bei der von der Anmelderin entwickelten Adaptive Support Ventilation (ASV-Beatmung) und der ebenfalls von der Anmelderin entwickelten INTELLiVENT-ASV-Beatmung Anwendung finden. Solche Beatmungsformen zeichnen sich dadurch aus, dass nur minimale manuelle Eingriffe seitens des Bedienpersonals erfolgen müssen und die Beatmungsvorrichtung 10 wichtige Beatmungsparameter wie den positiven endexpiratotischen Druck PEEP oder den maximalen Atemwegsdruck Paw_max im Rahmen vorgegebener Wertebereiche mit Hilfe von geeigneten geschlossenen Regelkreise selbsttätig einstellt bzw. nachstellt.

Das Ösophagusballonsteuersystem 400 ist auch zur Kommunikation, insbesondere zu bidirektionaler Kommunikation, 140, 140' mit dem Druckermittlungssystem 300 ausgebildet, so dass das Ösophagusballonsteuersystem 400 auf die von dem Druckermittlungssystem 300 bestimmten Drücke Pinsp, Pexp, dP, Paw und Peso zugreifen kann.

Der in den Alveolen der Lunge 28, 30 herrschende Druck, der auch als "aveolärer Druck" bezeichnet wird, wird in Fig. 1 durch Palv symbolisiert. Der aveoläre Druck Palv hängt ab vom Atemwegeingangsdruck Paw, dem Atemwegswiderstand R und der Strömung des Atemgases V in die Lunge bzw. aus der Lunge heraus. Im Falle eines vollständigen Druckausgleichs zwischen dem Atemwegeingang und den Alveolen ist der alveoläre Druck Palv gleich dem Atemwegeingangsdruck Paw (Palv = Paw).

Ein derartiger vollständiger Druckausgleich hat zur Konsequenz, dass der Atemgasfluss F zum Erliegen kommt. Beispielsweise kann ein kurzzeitiges Okklusionsmanöver des Atemwegs, bei dem das Atemwegeingangsventil 18 und das Atemwegausgangsventil 24 gleichzeitig geschlossen werden, zu einem Druckausgleich führen. Das Okklusionsmanöver muss gerade solange dauern, dass der Gasfluss F im Atemweg zum Erliegen kommt. Das sind in der Regel zwischen 1 s und 5 s. In diesem Zustand kann dann der alveoläre Druck Palv durch Bestimmen des Atemwegeingangsdrucks Paw bestimmt werden.

Sowohl bei der physiologischen Atmung als auch bei maschineller Beatmung wird der Fluss F des Atemgases durch eine Druckdifferenz Δp = Palv - Paw zwischen dem alveolären Druck Palv und dem Atemwegeingangsdruck Paw bestimmt.

Im Falle der rein physiologischen Atmung wird zum Einatmen durch Ausdehnung des Thorax 42 eine negative Druckdifferenz, d.h. ein Unterdruck, zwischen dem alveolären Druck Palv und dem Atemwegeingangsdruck Paw erzeugt. Damit verbunden ist eine Absenkung des Drucks Ppl in dem Pleuraspalt 44, der zwischen Thorax 42 und Lunge 28, 30 ausgebildet ist. Das Ausatmen erfolgt passiv durch Entspannen des Thorax 42 und elastischer Rückstellung des Lungengewebes. Aus diesem Grund ist bei physiologischer Atmung der Druck im Pleuraspalt Ppl immer kleiner als der alveoläre Druck Palv (Ppl > Palv). Der als Differenz zwischen dem alveolären Druck Palv und dem Druck im Pleuraspalt Ppl definierte transpulmonale Druck Ptp = Palv - Ppl ist damit generell positiv und wird im Falle eines vollständigen Druckausgleichs Null.

Bei einer maschinellen Beatmung wird das Atemgas mit Überdruck in die Lunge gepumpt. Aus diesem Grund ist der Atemwegeingangsdruck Paw = Plnsp bei maschineller Beatmung während der Inspirationsphase größer als der alveoläre Druck Palv und dieser ist wiederum größer als der Druck im Pleuraspalt Ppl: $Paw > Palv > Ppl .$

Aus diesen Druckverhältnissen folgt, dass der transpulmonale Druck Ppl bei maschineller Beatmung während der Inspiration positiv ist (Ppl > 0). Während der Exspiration wird der Atemwegeingang mit einem Atemwegsdruck Pexp beaufschlagt, der geringer ist als der alveoläre Druck Palv (Pexp < Palv), so dass Atemgas aus den Alveolen herausströmt. Im Falle eines sehr kleinen Atemwegsdrucks Pexp kann es vorkommen, dass am Ende der Exspiration, wenn nur noch sehr wenig Gas in der Lunge vorhanden ist, der Druck im Pleuraspalt Ppl den alveolären Druck Palv in so hohem Maße übersteigt, dass ein Teil der Alveolen der Lunge kollabiert. Der transpulmonale Druck Ptp ist dann negativ (Ptp < 0).

Das unerwünschte und für die Lunge schädliche Kollabieren der Alveolen lässt sich verhindern, wenn man auch in der Exspirationsphase den Atemwegeingang mit einem zusätzlichen positiven Druck beaufschlagt. Es liegt dann am Atemwegeingang dauerhaft, d.h. während der Inspirationsphase und auch während der Exspirationsphase ein positiver Atemwegsdruck an. Dieser positive Atemwegsdruck wird als positiver endexpiratorischer Druck PEEP bezeichnet.

Der transpulmonale Druck Ptp ist demzufolge eine geeignete Größe zur Einstellung des PEEP. Allerdings ist der transpulmonale Druck Ptp einer unmittelbaren Erfassung nicht zugänglich und lässt sich auch nicht aus den bei maschineller Beatmung regelmäßig erfassten Drücken, wie sie oben beschrieben sind, ermitteln.

Um den transpulmonalen Druck Ptp zu bestimmen, benötigt man neben dem Druck im Pleuraspalt Ppl noch Information über den alveolären Druck Palv. Eine elegante Möglichkeit zur Bestimmung des alveolären Drucks Palv(t) zu einem bestimmten Zeitpunkt t bietet die Erfassung des Atemgasflusses F(t), die mit Hilfe des Atemwegseingangsflusssensors 36 vorgenommen werden kann.

Nach dem Erfassen des Atemgasflusses F(t) kann man über die Beziehung: Palv(t) = Paw (t) - R * V(t) auf den alveolären Druck zum Zeitpunkt t schließen, wobei R den Atemwegswiderstand bezeichnet. Der Atemwegswiderstand R ist für ein und denselben Patienten eine sich im Wesentlichen nicht oder nur vergleichsweise langsam ändernde Größe und kann durch im Stand der Technik bekannte Methoden bestimmt werden. Beispielsweise sei verwiesen auf lotti I.A. et al., Intensive Care Med., 1995, 21: 406-413. Da es zur Bestimmung eines geeigneten PEEP vor allem auf den transpulmonalen Druck am Ende der Exspirationsphase Ptp_ee ankommt, wird man im Zusammenhang mit einer automatischen Einstellung des PEEP eine Bestimmung des alveolären Drucks Palv vorzugsweise am Ende der Exspirationsphase vornehmen, gemäß der Formel: $Ptp_ee = Palv_ee - Peso_ee = Paw_ee - R * V_ee - Peso_ee .$

Der PEEP sollte dann so eingestellt werden, dass Ptp_ee immer positiv bleibt, jedenfalls nie deutlich unter Null sinkt.

Leider lässt die beschriebene Methode der Bestimmung des alveolären Drucks Palv, die in einer automatisiert arbeitenden Beatmungsvorrichtung 10 recht einfach realisierbar ist, nur eine vergleichsweise grobe Abschätzung des geeigneten PEEP zu. Das liegt vor allem an dem nur recht ungenau abschätzbaren Atemwegswiderstand R, der zudem in der Regel im zeitlichen Verlauf einer Therapie durchaus einem gewissen Trend unterworfen sein wird.

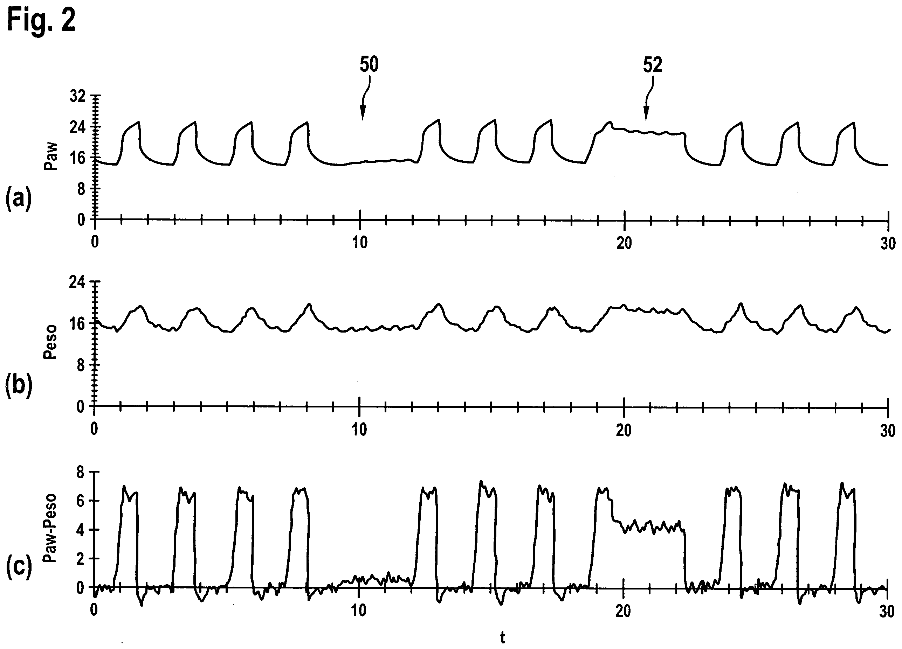

Eine alternative Methode zur Bestimmung des alveolären Drucks Palv beruht auf einem kurzzeitigen Okklusionsmanöver, bei dem sowohl das Atemwegeingangsventil 18 als auch das Atemwegausgangsventil 24 gleichzeitig geschlossen bleiben. In diesem Okklusionszustand tritt ein Ausgleich der im Atemweg herrschenden Drücke ein. Führt man ein solches Okklusionsmanöver am Ende einer Exspirationsphase durch, so ist der sich nach einer genügend langen Okklusion im Atemweg einstellende Druck in guter Näherung gleich dem alveolären Druck Palv am Ende der Exspirationsphase. Dieser Druck kann dann recht einfach mit Hilfe der am Atemwegeingang angeordneten Drucksonde zur Messung des Atemwegsdrucks Paw erfasst werden. In Fig. 2 ist diese Situation an der mit den Bezugszeichen 50 bezeichneten Stelle dargestellt.

Fig. 2 zeigt übereinander jeweils den zeitlichen Verlauf des Atemwegsdrucks Paw (Fig. 2(a)), des in der Ballonsonde 46 gemessenen Ösophagusballondrucks Peso (Fig. 2(b)) und der Differenz Paw - Peso aus beiden Drücken während mehrerer aufeinander folgender Atemzyklen, zwischen denen auch Okklusionsmanöver durchgeführt wurden. In der Fig. 2 kann man deutlich die einzelnen Atemzyklen erkennen, mit je einer Inspirationsphase (hoher ansteigender Atemwegsdruck Paw in der Fig. 2(a)) und einer Exspirationsphase (abfallender Atemwegsdruck Paw).

Der Ösophagusballondruck Peso folgt dem Atemwegsdruck Paw, allerdings in abgeschwächter Form. Der in der unteren Kurve (Fig. 2(c)) dargestellte Differenzdruck Paw - Peso würde - bei einer ausreichend langsamen Strömung des Atemgases, so dass immer ein Druckausgleich stattfindet - recht gut dem transpulmonalen Druck Ptp entsprechen. Diese Voraussetzung ist in der Praxis jedoch wegen der sich stark ändernden Atemgasströmung in der Regel nicht erfüllt, abgesehen an den mit den Bezugszeichen 50 und 52 bezeichneten Stellen, bei denen eine kurzzeitige Okklusion am Ende einer Exspirationsphase durchgeführt wurde (Bezugszeichen 50, zwischen etwa 8 s und 12 s) bzw. eine kurzzeitige Okklusion am Ende einer Inspirationsphase durchgeführt wurde (Bezugszeichen 52, zwischen etwa 18,5 s und 24,5 s). Die Okklusion dauerte in beiden Fällen etwa 4 s. Das entspricht in dem gewählten Beispiel in etwa der Dauer eines Atemzyklus. Generell sollte die Okklusion so lange dauern, dass ein vollständiger Druckausgleich im Atemweg stattfindet und der Gasfluss im Atemweg damit zum Erliegen kommt.

Am Ende der mit dem Bezugszeichen 50 bezeichneten Stelle des zeitlichen Profils (etwa zwischen 11 s und 12 s, beispielsweise in den letzten ca. 200 ms der Okklusion) entspricht der in der dritten Zeile in der Fig. 2(c) dargestellte Druck Paw - Peso in recht guter Näherung dem transpulmonalen Druck am Ende der Exspirationsphase Ptp_ee. Zur Bestimmung von Ptp_ee könnte man daher beispielsweise den Mittelwert von Paw - Peso über den genannten Zeitraum hinweg bilden. Am Ende der mit dem Bezugszeichen 52 bezeichneten Stelle des zeitlichen Profils (etwa zwischen 21,5 s und 22,5 s, beispielsweise in den letzten ca. 200 ms der Okklusion) entspricht der in der dritten Zeile dargestellte Druck Paw - Peso in recht guter Näherung dem transpulmonalen Druck am Ende der Inspirationsphase Ptp_ei. Zur Bestimmung von Ptp_ei könnte man daher beispielsweise den Mittelwert von Paw - Peso über den genannten Zeitraum hinweg bilden.

Die Bestimmung des transpulmonalen Drucks Ptp mit Hilfe des beschriebenen Okklusionsmanövers ist genauer als die zuvor beschriebene Methode mit Hilfe des Atemwegswiderstands R. Allerdings erfordert diese Vorgehensweise das Durchführen eines Okklusionsmanövers am Ende einer Exspirationsphase bzw. am Ende einer Inspirationsphase. Deshalb stört diese Methode naturgemäß den Atemzyklus, und zwar umso erheblicher, je länger die Dauer der Okklusion im Vergleich zur Dauer des Atemzyklus ist. Aus diesem Grund ist es ratsam, recht häufig, beispielsweise nach jedem Atemzug oder alle n Atemzüge (n>1), mit Hilfe der Atemwegswiderstandsmethode zu überprüfen, ob ein eingestellter Wert des PEEP und/oder ein eingestellter Wert des maximalen Atemwegsdrucks noch im Rahmen der Vorgaben liegen und/oder ob ein sich ergebender Wert des transpulmonalen Drucks Ptp_ee noch innerhalb bestimmter Vorgaben für einen normierten transpulmonalen Druck Ptp_ee_ideal liegt.

Sollte sich bei dieser Prüfung herausstellen, dass dies nicht der Fall ist und deswegen ein neuer (höherer oder niedrigerer) Wert für den PEEP und/oder den maximalen Atemwegsdruck eingestellt werden sollte, so wird im folgenden Atemzyklus am Ende der Exspirationsphase ein Okklusionsmanöver durchgeführt und der neue Wert für den PEEP wie zuvor beschrieben ermittelt. Alternativ kann das zuvor beschriebene Okklusionsmanöver auch alle n Atemzyklen wiederholt werden, wobei n eine ganze Zahl größer als 1, beispielsweise n = 10, 50, oder 100 ist.

Eine Voraussetzung für die vorangehend beschriebene Bestimmung des transpulmonalen Drucks Ptp mit Hilfe eines Ösophagusballonkatheters 45 mit Ballonsonde 46 ist, dass zwischen dem transpulmonalen Druck Ptp und dem in der Ballonsonde 46 erfassten Ösophagusballondruck Peso eine feste Beziehung besteht. Im Idealfall sollte der in der Ballonsonde 46 erfasste Ösophagusballondruck Peso näherungsweise dem Druck im Pleuralspalt Ppl entsprechen, so dass sich der transpulmonale Druck Ptp dann aus der Differenz zwischen Atemwegsdruck Paw und Ösophagusballondruck Peso ergibt. In der Praxis kommt es aber regelmäßig vor, dass die Beziehung zwischen dem Ösophagusballondruck Peso, der in einem in den Ösophagus 34 eingeführten Ösophagusballonkatheter 45 mit Ballonsonde 46 gemessen wird, und dem Druck Ppl im Pleuraspalt sich während der Beatmung verändert. Solche Veränderungen können in aller Regel nicht eindeutig auf bestimmte Ursachen oder Ereignisse zurückgeführt werden und treten häufig schleichend auf.

Aus diesem Grund ist häufig eine fortlaufende Überwachung des Ösophagusballonkatheters 45, ggf. in Verbindung mit einer Nachfüllung oder Neubefüllung der Ballonsonde 46 mit Messfluid, eventuell sogar in Verbindung mit einer (Neu)-Kalibrierung einer bestimmungsgemäß zu wählenden Menge an Messfluid in der Ballonsonde 46, des Ösophagusballonkatheters 45 vorteilhaft.

Allerdings sind die derzeit bei der maschinellen Beatmung in vivo eingesetzten Ösophagusballonkatheter 45 äußerst empfindlich und ihr Einsatz ist für das Pflegepersonal aufwändig und zeitraubend. Ösophagusballonkatheter 45 sind daher derzeit zum Einsatz während der Beatmung von Patienten, insbesondere während länger andauernder Beatmung, zur laufenden Überwachung des transpulmonalen Drucks Ptp während der Beatmung und ggf. zur Korrektur einer fehlerhafter Befüllung nicht besonders gut geeignet. Insbesondere können derzeit Ärzte und Pflegepersonal zur Überwachung und/oder Einstellung von Beatmungsparametern während laufender Beatmung nur sehr eingeschränkt auf Messdaten zurückgreifen, die während der Beatmung von einem Ösophagusballonkatheter geliefert werden und damit eigentlich zur Verfügung stünden.

Die vorliegende Offenbarung stellt eine Vorrichtung und ein Verfahren bereit, die es ermöglichen, die Genauigkeit von Druckmesswerten, die von einem in den Ösophagus 34 eingeführten Ösophagusballonkatheter 45 mit Ballonsonde 46 geliefert werden, zu verbessern. Insbesondere erlauben eine offenbarungsgemäße Vorrichtung und ein offenbarungsgemäßes (nicht beanspruchtes) Verfahren eine genauere Wiedergabe des im Pleuraspalt herrschenden Drucks Ppl durch den Ösophagusballonkatheter 45 und eine schnellere Reaktion der Messwerte auf sich verändernde Umgebungsbedingungen während andauernder Beatmung.

Die vorliegende Offenbarung ermöglicht damit einen dauernden und weitgehend automatisierten Betrieb des Ösophagusballonkatheters 45, einschließlich einer dauernden Überwachung seiner Einstellung und ggf. Nachfüllung oder Neubefüllung der Ballonsonde 46 mit Messfluid, ohne dass die Beatmung hierzu unterbrochen werden muss. Es ist sogar möglich, während einer laufenden Beatmung eine Kalibrierung oder Neukalibrierung der Ballonsonde 46 vorzunehmen und/oder eine für die Messung des transpulmonalen Drucks Ptp optimale Füllmenge an Messfluid in der Ballonsonde 46 zu bestimmen. Dies gilt auch dann, wenn die Beatmung mittels vollautomatischer Beatmungsmodi erfolgt, beispielsweise bei einer Beatmung mittels geschlossener Regelkreise, insbesondere bei der von der Anmelderin entwickelten "Adaptive Support Ventilation" (ASV-Beatmung) und der ebenfalls von der Anmelderin entwickelten INTELLiVENT-ASV-Beatmung.

Dem Ösophagusballonkatheter 45 ist ein Ösophagusballonsteuersystem 400 zugeordnet. Das Ösophagusballonsteuersystem 400 hat insbesondere die Funktion, die Ballonsonde 46 des Ösophagusballonkatheters 45 mit einem Messfluid zu beaufschlagen. Als Messfluid kommt in der Regel Luft zum Einsatz, es können aber auch beliebige andere Fluide, insbesondere Gase, verwendet werden.

In der Regel wird die Ballonsonde 46 nach Einführen in den Ösophagus 34 und Platzieren der Ballonsonde 46 im Ösophagus 34 mit einer vorbestimmten Menge Vₒₚₜ an Messfluid befüllt, die derart bemessen ist, dass der in der Ballonsonde 46 gemessene Druck möglichst empfindlich auf Druckänderungen im Pleuraspalt reagiert, die vom Pleuraspalt 44 über die Außenwand des Ösophagus 34 auf die Ballonsonde 46 ausgeübt werden. Dazu sollte die Ballonsonde 46 an einer Stelle des Ösophagus 34 platziert werden, die möglichst vom Druck im Pleuraspalt 44 beaufschlagt wird. Gleichzeitig sollte die Ballonsonde 46 mit so viel Messfluid befüllt werden, dass sie möglichst gleichmäßig an der Innenwand des Ösophagus 34 anliegt. Dabei sollte der Druck in der Ballonsonde 46 nicht so groß werden, dass sich die Wand des Ösophagus 34 unter dem in der Ballonsonde 46 herrschenden Druck nicht mehr weiter nach außen ausdehnen kann. Um eine geeignete bzw. optimale Befüllung der Ballonsonde 46 mit Messfluid einzustellen, ist eine Kalibrierung der Ballonsonde vorgesehen. Das Ösophagusballonsteuersystem 400 ist dazu ausgebildet, eine solche Kalibrierung in zeitlich wiederkehrender Weise durchzuführen. Zu diesem Zweck umfasst das Ösophagusballonsteuersystem 400 eine Kalibriersteuerung 450.

Die Kalibriersteuerung 450 ist derart ausgebildet, dass sie mehrere Messpunkte einstellt, indem sie die Menge Vᵢ (i = 1, 2, 3, ...) an Messfluid in der Ballonsonde 46 schrittweise verändert. Für jede der auf diese Weise schrittweise als Messpunkte eingestellten Mengen Vᵢ an Messfluid in der Ballonsonde 46 nimmt die Kalibriersteuerung 450 einen Ösophagusballondruck Pesoᵢ auf und ordnet diesen jeweils eingestellten Menge Vᵢ an Messfluid in der Ballonsonde 46 zu. Hierzu wird das von der Ballonsonde 46 erfasste Drucksignal Pesoᵢ auch der Kalibriersteuerung 450 übermittelt.

Das Ösophagusballonsteuersystem 400 umfasst auch eine Pumpanordnung 430, die zum Einleiten von Messfluid in die Ballonsonde 46 und Entnehmen von Messfluid aus der Ballonsonde 46 nach Platzieren der Ballonsonde 46 im Ösophagus 34 ausgebildet ist. Die Pumpanordnung 430 weist eine Pumpe 425 und ein Ventil 420 auf, die in einer mit der Ballonsonde 46 in Fluidverbindung stehenden Förderleitung 66 für Messfluid angeordnet sind. Soweit das Messfluid in der Ballonsonde 46 unter einem Überdruck steht, kann das Messfluid ohne den Einsatz der eigentlichen Pumpe 425 einfach durch Öffnen des Ventils 420 aus der Ballonsonde 46 entnommen werden.

Das Ösophagusballonsteuersystem 400 weist weiterhin einen Flusssensor 410, insbesondere einen Massenflusssensor 410, auf, der zur Bestimmung einer Menge an in die Ballonsonde 46 eingeleitetem und/oder einer Menge an aus der Ballonsonde 46 entnommenen Messfluid ausgebildet ist. Die jeweils in die Ballonsonde 46 eingeleitete bzw. aus der Ballonsonde 46 entnommene Menge an Messfluid kann beispielsweise durch Integrieren eines von dem Flusssensor 410 gemessenen Flusses über einen Zeitraum zwischen einem Startzeitpunkt und einem Endzeitpunkt bestimmt werden. Der Flusssensor 410 kann beispielsweise so ausgebildet sein, dass er den Massenfluss an Messfluid anhand eines Differenzdrucks bestimmt. Ein solcher Flusssensor 410 kann auch zum Erfassen des in der Ballonsonde 46 herrschenden Ösophagusballondrucks Peso eingesetzt werden.

Die Kalibriersteuerung 450 kann als eigenständiges Bauteil "in Hardware" realisiert werden. Alternativ kann die Kalibriersteuerung 450 auch als Computerprogrammprodukt, d.h. durch ein entsprechendes Softwareprogramm realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocontroller kann der Steuerung des Ösophagusballonsteuersystems 400 zugeordnet sein, insbesondere Teil der Steuerung des Ösophagusballonsteuersystems 400 sein.

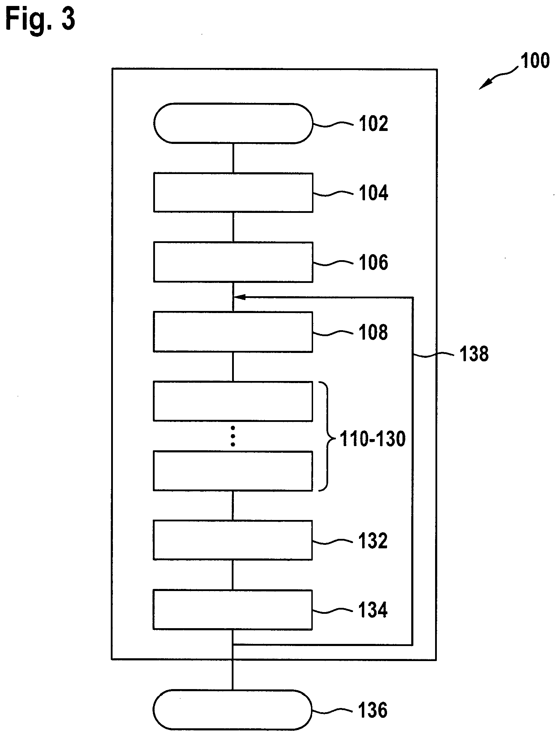

Fig. 3 veranschaulicht den Ablauf einer Kalibrierung eines Ösophagusballonkatheters 45 in vivo gemäß einer Ausführungsform der vorliegenden Offenbarung in einem Flussdiagramm.

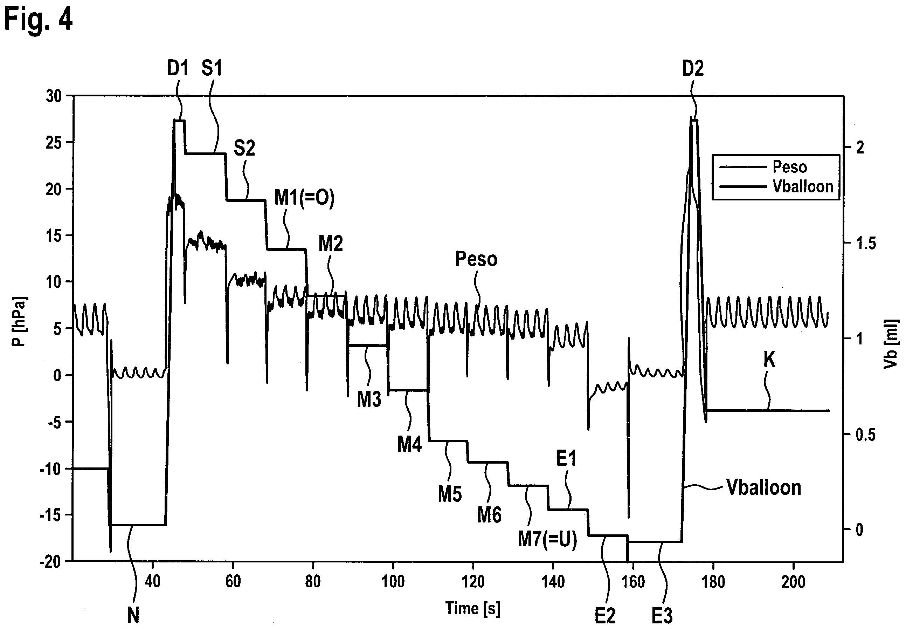

Fig. 4 zeigt schematisch den zeitlichen Verlauf der während einer Kalibrierung gemäß einer Ausführungsform der vorliegenden Offenbarung eingestellten Menge an Messfluid V_{b} in der Ballonsonde 46 des Ösophagusballonkatheters 45 sowie des in der Ballonsonde 46 des Ösophagusballonkatheters 45 erfassten Ösophagusballondruck Peso.

Die Kalibriersteuerung 450 ist derart ausgebildet, dass sie zum Anfahren der jeweiligen Messpunkte S1, S2, M1, M2,..., M7, E1, E2, E3 die Menge an Messfluid V_{b} in der Ballonsonde 46 ausgehend von einem Startwert S2 bis zum Erreichen eines Endwertes E3 in mindestens zwei Schritten in monotoner Weise verändert.

Das Messfluid ist insbesondere Luft, es kann aber auch ein anderes Fluid, insbesondere ein anderes Gas, sein.

Die in Fig. 3 gezeigte Kalibrierungsprozedur 100 beginnt in Schritt 102. Zunächst wird in Schritt 104 so viel Messfluid aus der Ballonsonde 46 abgepumpt, das in der Ballonsonde 46 ein vorbestimmter Anfangsdruck herrscht. Der in Schritt 104 erreichte Zustand ist in Fig. 4 mit "N" bezeichnet.

Danach wird in Schritt 106 wieder Messfluid in die Ballonsonde 46 gepumpt, bis in der Ballonsonde 46 ein vorbestimmter Überdruck herrscht, der oberhalb des erwarteten Messbereichs für den Messzyklus liegt. Der in Schritt 104 erreichte Zustand ist in Fig. 4 mit "D1" bezeichnet.

Alle weiteren in den nachfolgenden Schritten 108 bis 130 angefahrenen Messpunkte sind in Fig. 4 mit S1, S2, M1 - M7, E1, E2, E3 bezeichnet.

In dem in Schritt 106 eingestellten Zustand ist die Ballonsonde 46 deutlich überdehnt. Man erkennt das daran, dass in Fig. 4 der Wert des in der Ballonsonde 46 erfassten ösophagealen Drucks Peso deutlich höher liegt als in allen weiteren angefahrenen Messpunkten S1, S2, M1 - M7, E1, E2, E3.

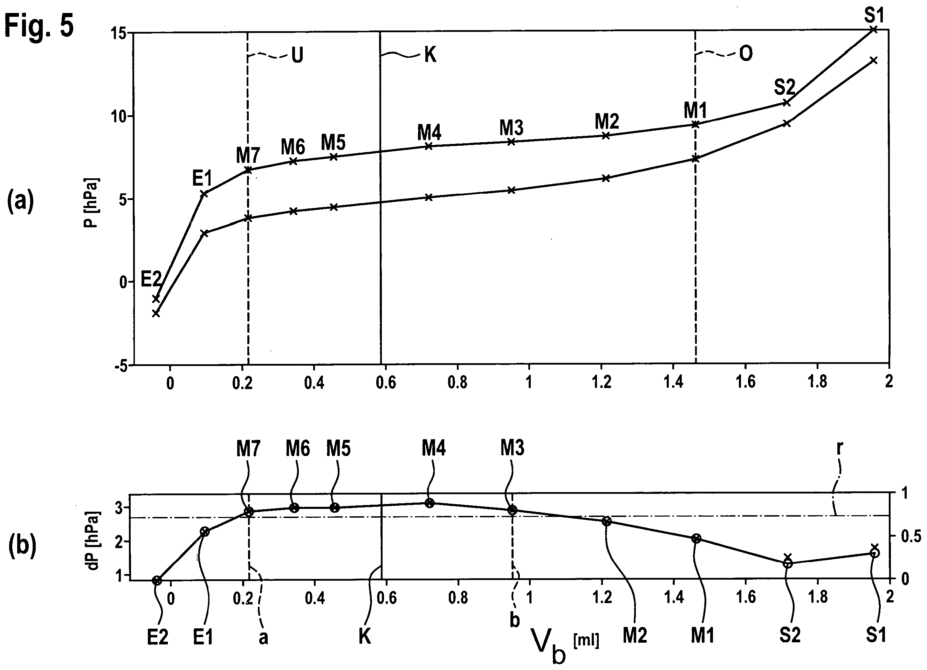

Die Obergrenze des Messbereichs ist in Fig. 4 und 5 mit O bezeichnet und die Untergrenze des Messbereichs ist in Fig. 4 und 5 mit U bezeichnet. Man erkennt, dass bei der in Fig. 4 und 5 gezeigten Kalibrierprozedur die Untergrenze U des Messbereichs bei dem Messpunkt M7 liegt und die Obergrenze O des Messbereichs bei dem Messpunkt M1 liegt. Es wurde für die Obergrenze O und Untergrenze U jeweils die zugehörige Menge Vb an Messfluid in der Ballonsonde 46 als Bezugsgröße gewählt. O bezeichnet somit die Menge an Messfluid in der Ballonsonde 46 bei dem Messpunkt M1, der der Obergrenze des Messbereichs entspricht. U bezeichnet die Menge an Messfluid in der Ballonsonde 46 bei dem Messpunkt M7, der der Untergrenze des Messbereichs entspricht.

Ausgehend von Schritt 106 wird in Schritt 108 eine vorbestimmte Menge an Messfluid aus der Ballonsonde 46 abgepumpt. Damit wird eine Menge an Messfluid in der Ballonsonde 46 eingestellt, bei der ein Ösophagusballondruck Peso erfasst wird, der ein wenig oberhalb des im Messbereich (siehe Messpunkte M1 bis M7) erwarteten ösophagealen Drucks liegt. Dieser Zustand ist in Fig. 4 und 5 mit "S1" bezeichnet. Alternative könnte auch solange Messfluid aus der Ballonsonde 46 abgepumpt werden, bis ein vorbestimmter Wert des Ösophagusballondrucks Peso erfasst wird, der ein wenig oberhalb des im Messbereich (siehe Messpunkte M1 bis M7) erwarteten Ösophagusballondrucks Peso liegt.

Während des Zustands "S1" wird über eine Mehrzahl von Atemzyklen hinweg der Ösophagusballondruck Peso in der Ballonsonde 46 erfasst. Man erkennt in Fig. 4, dass der Ösophagusballondruck Peso die einzelnen Atemzyklen wiedergibt, allerdings nur in einem sehr geringen Ausmaß, was auf geringe Sensitivität der Ballonsonde 46 hindeutet.

Ausgehend von Schritt 108 wird in Schritt 110 eine vorbestimmte Menge an Messfluid aus der Ballonsonde 46 abgepumpt bis der in Fig. 4 und 5 mit "S2" bezeichnete Zustand erreicht wird. Dabei wird in Schritt 110 für den Zustand "S2" dieselbe Prozedur wie in Bezug zu Schritt 108 beschrieben wiederholt. Auch hier erkennt man nur gering ausgeprägte Atemzyklen und damit eine nur geringe Sensitivität der Ballonsonde 46.

Nach Beendigung von Schritt 110 wird in Schritt 112 abermals eine vorbestimmte Menge an Messfluid aus der Ballonsonde 46 abgepumpt, bis der in Fig. 4 und 5 mit "M1" bezeichnete Zustand erreicht wird. Dabei wird in Schritt 112 für den Zustand "M1" dieselbe Prozedur wie in Bezug zu Schritt 108 beschrieben wiederholt.

Man erkennt in Fig. 4 und 5, dass in dem Zustand am Messpunkt M1 (und auch in den nachfolgenden Zuständen an den Messpunkten M2 bis M7) der Ösophagusballondruck Peso deutlich die einzelnen Atemzyklen wiedergibt. Dabei sind die Maxima der Peso-Kurve den am Ende einer jeweiligen Inspirationsphase erfassten Ösophagusballondrücken Peso_ei in der Ballonsonde 46 zuzuordnen und die Minima der Peso-Kurve den am Ende einer jeweiligen Exspirationsphase erfassten Ösophagusballondrücken Peso_ee in der Ballonsonde 46 zuzuordnen.

Diese Prozedur wird in der Folge (siehe Schritte 114 - 130) mehrmals wiederholt, um weitere Messpunkte M2 - M7, E1, E2 anzufahren. An jedem der weiteren Messpunkte M2 - M7, E1, E2 wird in derselben Weise wie mit Bezug zu Schritt 108, 110 und 112 beschrieben über eine Mehrzahl von Atemzyklen hinweg der Ösophagusballondruck Peso in der Ballonsonde 46 erfasst und die Maxima der Peso-Kurve den am Ende einer jeweiligen Inspirationsphase erfassten Ösophagusballondrücken Peso_ei in der Ballonsonde 46 zugeordnet sowie die Minima der Peso-Kurve den am Ende einer jeweiligen Exspirationsphase erfassten Ösophagusballondrücken Peso_ee in der Ballonsonde 46 zugeordnet.

Die einzelnen Schritte 110 - 130 sind in dem Flussdiagramm von Fig. 3 nicht im Einzelnen gezeigt.

In der Fig. 4 ist zu erkennen, dass diese Schritte jeweils zu einer bestimmten Menge an Messfluid in der Ballonsonde 46 gehören, die als Messpunkte S1, S2, M2 - M7, E1, E2 bezeichnet sind. Die Menge an Messfluid in der Ballonsonde 46 wird beim Übergang von einem der Messpunkte S1, S2, M1 - M7, E1, E2 zu dem nächsten Messpunkt immer in monotoner Weise verändert, insbesondere immer verringert.

Dabei kann die Schrittweite, d.h. die Menge, um welche die Menge an Messfluid in der Ballonsonde 46 beim Übergang von einem der Messpunkte zum nächsten Messpunkt verändert wird, immer gleich sein. Es ist aber auch möglich, dass die Menge, um die Menge an Messfluid in der Ballonsonde 46 verändert, in jedem Schritt anders gewählt wird, solange die Veränderung immer in derselben Richtung erfolgt, d.h., dass die Menge immer verringert wird oder immer vergrößert wird. Da die Zuordnung zwischen einem jeweiligen Messpunkt und der zugehörigen Menge an Messfluid in der Ballonsonde 46 eineindeutig ist, wird der Einfachheit halber im Folgenden nicht nur ein jeweiliger Messpunkt, sondern auch die jeweils zugehörige Menge an Messfluid in der Ballonsonde 46 mit D1, S1, S2, M1 - M7, E1, E2 usw. bezeichnet.

M1 bezeichnet beispielsweise sowohl den Messpunkt M1 als auch die zugehörige Menge an Messfluid in der Ballonsonde 46. In gleicher Weise bezeichnet M7 beispielsweise sowohl den Messpunkt M7 als auch die zugehörige Menge an Messfluid in der Ballonsonde 46. Entsprechendes gilt auch für die anderen Messpunkte M2 - M6 der Messreihe. Die Menge M1 an Messfluid in der Ballonsonde 46 stellt auch die Obergrenze O für den Messbereich dar. Die Menge M7 an Messfluid in der Ballonsonde 46 stellt auch die Untergrenze U für den Messbereich dar.

In Fig. 4 erkennt man, dass für die Messpunkte M1 - M7 das erfasste Signal des Ösophagusballondrucks Peso deutlich die einzelnen Atemzyklen abbildet, dass aber ab dem Messpunkt E1 das erfasste Signal des Ösophagusballondrucks Peso instabil zu werden beginnt. Das deutet an, dass man jetzt in einen Bereich gelangt, in dem die Ballonsonde 46 nur noch unzureichend mit Messfluid befüllt ist und somit auf die von den einzelnen Atemzyklen verursachten Druckänderungen nicht mehr ausreichend reagieren kann. Bei den Messpunkten E2 und E3 kann man davon ausgehen, dass die Ballonsonde 46 vollends kollabiert ist und der Ösophagusballondruck Peso kaum noch Atemzyklen anzeigt. Der Messzyklus wird dann mit Erreichen von Messpunkt E3 beendet. Im Messpunkt E3 wird kein Ösophagusballondruck Peso mehr erfasst und zugeordnet.

Der Zustand M1 bezeichnet die Obergrenze O des für den Messzyklus nutzbaren Messbereichs. Die eigentliche Kalibration beschränkt sich daher auf diesen durch die Messpunkte M1 - M7 festgelegten Messbereich. Man erkennt in Fig. 4, dass für alle Messpunkte M1 - M7 im Messbereich der in der Ballonsonde 46 erfasste Ösophagusballondruck Peso im Wesentlichen im gleichen Bereich liegt. Das zeigt an, dass die Ballonsonde 46 zusammen mit der Ösophaguswand in diesem Bereich ein im Wesentlichen elastisches System bildet, das sich entsprechend der Menge an Messfluid in der Ballonsonde 46 dehnt bzw. zusammenzieht. Diese Verhältnisse bleiben gleich, bis mit Erreichen von Messpunkt M7 die Untergrenze U des Messbereichs erreicht ist.

Im Messbereich zwischen den Messpunkten M1 und M7, und damit zwischen Werten von der Obergrenze O bis zur Untergrenze U für die Menge an Messfluid in der Ballonsonde 46, besteht eine annähernd lineare Beziehung zwischen der jeweiligen vorbestimmten Menge an Messfluid aus der Ballonsonde 46 und dem jeweils erfassten Ösophagusballondruck Peso. Die Steigung einer diese lineare Beziehung wiedergebenden Gerade ist annähernd gleich für die Beziehung zwischen dem am Ende der Exspirationsphase erfassten Ösophagusballondruck Peso_ei und der vorbestimmten Menge an Messfluid in der Ballonsonde 46 und für die Beziehung zwischen dem am Ende der Exspirationsphase erfassten Ösophagusballondruck Peso_ee und der vorbestimmten Menge an Messfluid in der Ballonsonde 46.

In den außerhalb des Messbereichs liegenden Bereichen (Messpunkte S1, S2 bzw. Messpunkte E1, E2) verändert sich die Beziehung zwischen der jeweiligen vorbestimmten Menge an Messfluid in der Ballonsonde 46 und dem jeweils erfassten Ösophagusballondruck Peso deutlich. Diese Beziehung ist in den außerhalb des Messbereichs liegenden Bereichen nicht mehr näherungsweise linear und zeigt einen sehr viel stärkeren Anstieg bzw. Abfall des erfassten Ösophagusballondruck Peso bei größeren und kleineren Mengen an Messfluid in der Ballonsonde 46. Auch die Differenz zwischen dem für eine vorbestimmte Menge an Messfluid in der Ballonsonde 46 jeweils am Ende der Exspirationsphase erfassten Ösophagusballondruck Peso_ei und dem am Ende der Exspirationsphase erfassten Ösophagusballondruck Peso_ee wird in den außerhalb des eigentlichen Messbereichs (Messpunkte M1 bis M7) liegenden Bereichen an den Messpunkte S1, S2 bzw. E1, E2 sehr schnell kleiner.

An den in den Figuren 5(a) und 5(b) gezeigten Graphen lässt sich die Bedeutung der Kalibrierprozedur für die Messergebnisse erkennen. Fig. 5(a) zeigt schematisch für jede als Messpunkte S1, S2, M1 - M7, E1, E2 eingestellte Menge an Messfluid V_{b} in der Ballonsonde 46 während eines Messzyklus den jeweils erfassten Ösophagusballondruck Peso_ei in der Ballonsonde 46 am Ende der Inspirationsphase und den jeweils erfassten Ösophagusballondruck Peso_ee in der Ballonsonde 46 am Ende der Exspirationsphase. Fig. 5b zeigt den sich daraus für jeden Messpunkt S1, S2, M1 - M7, E1, E2 jeweils ergebenden Differenzdruck dP zwischen Ösophagusballondruck Peso_ei am Ende der Inspirationsphase und Ösophagusballondruck Peso_ee am Ende der Exspirationsphase.

Man erkennt, dass innerhalb eines Bereichs zwischen den Messpunkten M1 bis M7 die Veränderung des erfassten Ösophagusballondrucks Peso_ei in der Ballonsonde 46 am Ende der Inspirationsphase sowie die Veränderung des erfassten Ösophagusballondrucks Peso_ee in der Ballonsonde 46 am Ende der Exspirationsphase eine in etwa lineare Beziehung mit der Veränderung der in der Ballonsonde 46 befindlichen Menge V_{b} an Messfluid aufweist. Die Steigung der beiden sich so ergebenden Kurven Peso/V_{b} ist sehr flach und für die ösophagealen Druckwerte am Ende der Inspirationsphase Peso_ei und die ösophagealen Druckwerte am Ende der Exspirationsphase Peso_ee im Wesentlichen gleich. Dementsprechend ergibt sich auch, dass die in der Fig. 5(b) aufgetragene Differenz dP zwischen dem jeweils erfassten Ösophagusballondruck Peso_ei in der Ballonsonde 46 am Ende der Inspirationsphase und dem jeweils erfassten Ösophagusballondruck Peso_ee in der Ballonsonde 46 am Ende der Exspirationsphase in dem durch die Messpunkte M1 bis M7 definierten Messbereich im Wesentlichen konstant bleibt, während diese Differenz dP in den außerhalb dieses Messbereichs liegenden Bereichen, in denen die Messpunkte S1, S2 und E1, E2 liegen, schnell sehr klein wird.

Hieraus ergibt sich, dass eine optimale Kalibration des Ösophagusballonkatheters 45 bei einer Menge V_{b} an Messfluid in der Ballonsonde 46 liegt, die innerhalb des durch die Messpunkte M1 bis M7 definierten Messbereichs liegt, also innerhalb des Bereichs an Menge V_{b} von Messfluid in der Ballonsonde 46, zwischen den beiden in Fig. 5(a) eingezeichneten vertikalen gepunkteten Linien O und U liegt. Dementsprechend wählt die Kalibriersteuerung 450 als Ergebnis der Kalibrierung eine Menge Vₒₚₜ an Messfluid in der Ballonsonde 46 aus, die innerhalb des durch die Messpunkte M1 bis M7 definierten Messbereichs liegt. Diese Menge Vₒₚₜ wird in den Figuren 5(a) und 5(b) durch die vertikale Linie K repräsentiert. Sie wird nach dem Abschluss der Kalibrierprozedur als Menge V_{b} an Fluid in der Ballonsonde 46 eingestellt, wie in dem mit K bezeichneten horizontal verlaufenden Teil der V_{b}-Kurve im rechten Teil der Fig. 4 zu sehen ist.

Fig. 5(b) veranschaulicht, wie die Auswahl der optimalen Menge Vₒₚₜ an Messfluid in der Ballonsonde 46 innerhalb des durch die Messpunkte M1 bis M7 definierten Messbereichs abläuft. Innerhalb des Messbereichs wird zunächst derjenige Messpunkt gesucht, bei dem die Differenz dP zwischen dem jeweils erfassten Ösophagusballondruck Peso_ei in der Ballonsonde 46 am Ende der Inspirationsphase und dem jeweils erfassten Ösophagusballondruck Peso_ee in der Ballonsonde 46 am Ende der Exspirationsphase maximal ist. In dem in Fig. 5(b) gezeigten Beispiel ist das bei dem Messpunkt M4 der Fall. Danach wird eine erlaubte Schwankungsbreite um diese maximale Differenz dPmax festgelegt. Innerhalb dieser Schwankungsbreite liegende Werte der Differenz dP werden als nicht signifikant verschieden von dem Maximalwert dPmax angesehen. In dem in Fig. 5(b) gezeigten Beispiel beträgt die erlaubte Schwankungsbreite 10 % der beim Messpunkt M4 ermittelten maximalen Differenz dPmax. Diese Schwankungsbreite wird in der Fig. 5(b) durch die strichpunktierte Linie r repräsentiert. Innerhalb der Schwankungsbreite liegen die für die Messpunkte M3, M4, M5, M6 und M7 ermittelten Differenzen dP.

Alle diese Messpunkte liegen in der Nachbarschaft des Messpunktes M4 und sind einander benachbart. Daher wird ein Bereich an Menge von Messfluid in der Ballonsonde 46, der zwischen den Messpunkten M3 und M7 liegt, mit Blick auf die Befüllung der Ballonsonde 46 mit Messfluid als gleichwertig angesehen. Dieser Bereich wird in Fig. 5(b) durch vertikale gestrichelte Linien a und b begrenzt. Als optimale Menge Vₒₚₜ an Messfluid in der Ballonsonde 46 wird die Mitte des zwischen den Linien a und b liegenden Bereichs gewählt. Auf diese Weise erhält man die optimale Menge Vₒₚₜ an Messfluid in der Ballonsonde 46 als Ergebnis der Kalibrierung, siehe die vertikale Linie K in Fig. 5(b). Diese Menge Vₒₚₜ = K wird nach dem Abschluss der Kalibrierprozedur als Menge an Fluid in der Ballonsonde 46 eingestellt, siehe den Teil der V_{b}-Kurve ganz rechts in Fig. 4.

Nach dem Abschluss der Kalibrierprozedur leitet die Kalibriersteuerung 450 in Schritt 132 (siehe Fig. 3) wieder eine Menge an Messfluid in die Ballonsonde 46, die deutlich oberhalb der den Messpunkten M1 bis M7 des Messbereichs entsprechenden Menge liegt. Auch in diesem Schritt 132 soll wieder eine Überdehnung der Ballonsonde 46 herbeigeführt werden, in Fig. 4 ist dieser Zustand mit "D2" bezeichnet, bevor dann in Schritt 134 durch Entnehmen einer entsprechenden Menge an Messfluid aus der Ballonsonde 46 der in der vorangehenden Kalibrieprozedur ermittelte, kalibrierte Zustand eingestellt wird, der in Fig. 4 mit "K" bezeichnet ist, und in dem die Ballonsonde 46 mit der optimalen Menge Vₒₚₜ = K an Messfluid gefüllt ist.

Optional kann auch vorgesehen sein, dass sich an den in den Fig. 3 bis 5 dargestellten Messzyklus noch wenigstens ein weiterer Messzyklus anschließt, bevor der kalibrierte Zustand Vₒₚₜ = K eingestellt wird. In einem solchen Fall geht die Prozedur von Schritt 134 zurück zu Schritt 108 und wiederholt die Schritte für den weiteren Messzyklus analog zu den Schritten 110 - 130 für den ersten Messzyklus. In Fig. 3 ist dies durch die Linie 138 dargestellt.

Da die Kalibriersteuerung 450 zum Anfahren der jeweiligen Messpunkte S1, S2, M1 - M7, E1, E2 die Menge an Messfluid in der Ballonsonde 46 ausgehend von einem Startwert S1 bis zum Erreichen eines Endwertes E3 in mindestens zwei Schritten in monotoner Weise verändert, lässt sich die Kalibrierungsprozedur 100 in kurzer Zeit, beispielsweise innerhalb von nur wenigen Minuten, durchführen. Dies erlaubt es, die Kalibrierungsprozedur 100 während einer laufenden Beatmung von Zeit zu Zeit zu wiederholen, um sicherzustellen, dass der Ösophagusballonkatheter 45 immer korrekt kalibriert ist, auch wenn sich im Laufe der Beatmung die optimale Befüllung Vₒₚₜ der Ballonsonde 46 verändert. Dies ermöglich es, Patienten über längere Zeiträume hinweg in automatisierten Beatmungsmodi zu beatmen.

Ein offenbarungsgemäßes Ösophagusballonsteuersystem 400 umfasst auch eine Füllmengenüberwachung 470. Die Füllmengenüberwachung 470 ist dazu ausgebildet, die Füllmenge V an Messfluid in der Ballonsonde 46 wenigstens einmal, insbesondere mehrfach und schrittweise, zu verändern und bei jeder der auf diese Weise als Messpunkt eingestellten Füllmenge V an Messfluid den in der Ballonsonde 4 erfassten Ösophagusballondruck Peso aufzunehmen und der jeweils eingestellten Füllmenge V an Messfluid in der Ballonsonde 46 zuzuordnen. Hierzu wird das von der Ballonsonde 46 erfasste Drucksignal Peso nicht nur an die Beatmungssteuerung 180, sondern auch an die Füllmengenüberwachung 470 übermittelt.

Die Füllmengenüberwachung 470 hat Zugriff auf die Pumpanordnung 430 (Pumpe 425 und Ventil 420) und den Flusssensor 410 des Ösophagusballonsteuersystems 400, wie sie zuvor im Zusammenhang mit der Kalibriersteuerung 450 beschrieben worden sind. Das heißt, die Füllmengenüberwachung 470 ist in der Lage, die Pumpe 425 und das Ventil 420 der Pumpanordnung 430 anzusteuern und Signale von dem Flusssensor 410 zu empfangen.

Der Flusssensor 410 kann ähnlich dem in der Trachea 12 angeordneten Atemwegeingangsflusssensor 36 ausgebildet sein, so dass er neben der Bestimmung des Flusses auch eine Bestimmung des Ösophagusballondrucks Peso in der Ballonsonde 46 ermöglicht.

Die Füllmengenüberwachung 470 kann als eigenständiges Bauteil "in Hardware" realisiert sein. Alternativ kann die Füllmengenüberwachung 470 auch als Computerprogrammprodukt, d.h. durch ein entsprechendes Softwareprogramm realisiert sein, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocontroller kann der Steuerung des Ösophagusbal-Ionsteuersystems 400 zugeordnet sein, insbesondere Teil der Steuerung des Ösophagusballonsteuersystems 400 sein.

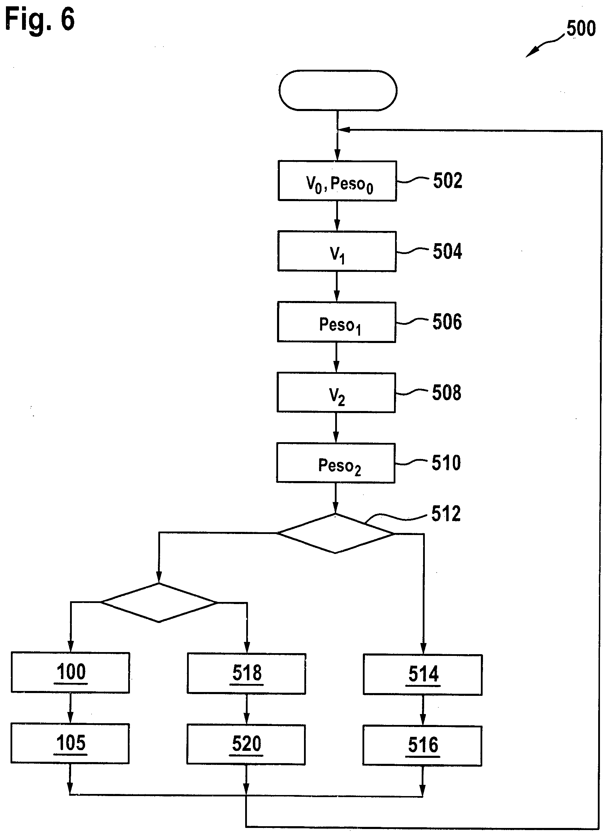

Fig. 6 zeigt ein Flussdiagramm, das den Ablauf einer Überwachung der Fluidmenge in einem Ösophagusballonkatheter 45 in vivo durch die Füllmengenüberwachung 470 gemäß einer Ausführungsform der vorliegenden Offenbarung veranschaulicht.

Die durch das in Fig. 6 gezeigte Flussdiagramm veranschaulichte Fluidmengen-Überwachungsprozedur 500 zum Überwachen der Fluidmenge V in einem Ösophagusballonkatheter 45 beginnt im Schritt 502 damit, dass die Füllmengenüberwachung 470 den Ösophagusballondruck Peso bei der aktuell in der Ballonsonde 46 des Ösophagusballonkatheters 45 vorhandenen Fluidmenge ("Ausgangsfluidmenge") V₀ misst und als Ausgangs-Osophagusballondruck Peso₀ speichert.

In einem zweiten Schritt 504 ändert die Füllmengenüberwachung 470 die in der Ballonsonde 46 des Ösophagusballonkatheters 45 vorhandenen Menge V an Messfluid auf eine erste Vergleichsmenge V₁ an Messfluid, die als erster Vergleichs-Messpunkt vorgegeben ist. Die als erster Vergleichs-Messpunkt vorgegebene Vergleichsmenge V₁ an Messfluid kann größer oder kleiner als die aktuell in der Ballonsonde 46 des Ösophagusballonkatheters 45 vorhandenen Menge V₀ an Messfluid sein.

Der zweite Schritt kann umfassen, zusätzliches Messfluid in die Ballonsonde 46 zu pumpen, um die Menge an Messfluid in der Ballonsonde 46 zu erhöhen (V, > V₀). Alternativ kann der zweite Schritt auch umfassen, Messfluid aus der Ballonsonde 46 abzulassen oder abzupumpen, um die Menge an Messfluid in der Ballonsonde 46 zu reduzieren (V₁ < V₀).

Nachdem die Menge an Messfluid in der Ballonsonde 46 auf die erste als Vergleichs-Messpunkt vorgegebene erste Vergleichsmenge V, an Messfluid eingestellt worden ist, wird der Ösophagusballondruck Peso in der Ballonsonde 46 im Schritt 506 gemessen und als erster Vergleichs-Ösophagusballondruck Peso₁ gespeichert.

In einem folgenden Schritt 508 ändert die Füllmengenüberwachung 470 die in der Ballonsonde 46 des Ösophagusballonkatheters 45 vorhandenen Menge an Messfluid auf eine als zweiter Vergleichs-Messpunkt vorgegebene zweite Vergleichsmenge V₂ an Messfluid, so dass die Messreihe, welche die Ausgangsfluidmenge V₀ sowie die erste und die zweite Vergleichsmenge V₃, V₂ an Messfluid umfasst, sowohl eine Vergleichsmenge V_{1,2} an Messfluid, die größer als die zu Beginn in der Ballonsonde 46 des Ösophagusballonkatheters 45 vorhandene Menge an Messfluid V₀ ist, als auch eine Vergleichsmenge V_{2,1} an Messfluid umfasst, die kleiner als die zu Beginn in der Ballonsonde 46 des Ösophagusballonkatheters 45 vorhandene Menge an Messfluid V₀ ist.

Mit anderen Worten, war die als erster Vergleichs-Messpunkt vorgegebene erste Vergleichsmenge V₁ an Messfluid größer als die zu Beginn (im Schritt 502) in der Ballonsonde 46 des Ösophagusballonkatheters 45 vorhandenen Menge an Messfluid V₀ (V₁ > V₀), so ist die als zweiter Vergleichs-Messpunkt vorgegebene zweite Vergleichsmenge V₂ an Messfluid vorzugsweise kleiner als die zu Beginn in der Ballonsonde 46 des Ösophagusballonkatheters 45 vorhandenen Menge an Messfluid V₀ (V₂ < V₀), so dass V₂ < V₀ < V₁.

War die als erster Vergleichs-Messpunkt vorgegebene erste Vergleichsmenge V₁ an Messfluid kleiner als die zu Beginn (im Schritt 502) in der Ballonsonde 46 des Ösophagusballonkatheters 45 vorhandenen Menge an Messfluid V₀ (V₁ < V₀), so ist die als zweiter Vergleichs-Messpunkt vorgegebene zweite Vergleichsmenge an Messfluid V₂ vorzugsweise größer als die zu Beginn in der Ballonsonde 46 des Ösophagusballonkatheters 45 vorhandene Menge an Messfluid V₀ (V₂ > V₀), so dass V₁ < V₀ < V₂.

Nachdem die Menge an Messfluid in der Ballonsonde 46 auf die als zweiter Vergleichs-Messpunkt vorgegebene zweite Vergleichsmenge an Messfluid V₂ eingestellt worden ist, wird der Ösophagusballondruck Peso in der Ballonsonde 46 im Schritt 510 gemessen und als zweiter Vergleichs-Ösophagusballondruck Peso₂ gespeichert.

In einem folgenden Schritt 512 werden der erste und der zweite Vergleichs-Osophagusballondruck Peso₁, Peso₂ mit dem Ausgangs-Ösophagusballondruck Peso₀ verglichen. Dabei können insbesondere die Differenzen zwischen dem ersten bzw. zweiten Vergleichs-Ösophagusballondruck Peso_{1,} Peso₂ und dem Ausgangs-Osophagusballondruck Peso₀ bestimmt werden: ${Δ}_{1} = \left|{Peso}_{1}-{Peso}_{0}\right|$ ${Δ}_{2} = \left|{Peso}_{2}-{Peso}_{0}\right| .$

Wenn der erste und der zweite Vergleichs-Ösophagusballondruck Peso_{1,} Peso₂ in einem vorgegebenen Bereich um den Ausgangs-Ösophagusballondruck Peso₀ liegen, d.h. wenn die Differenz Δ₁ zwischen dem ersten Vergleichs-Ösophagusballondruck p₁ und dem Ausgangs-Ösophagusballondruck Peso₀ und die Differenz Δ₂ zwischen dem zweiten Vergleichs-Ösophagusballondruck Peso₂ und dem Ausgangs-Ösophagusballondruck Peso₀ kleiner als ein vorgegebenen Grenzwert sind: ${Δ}_{1} < {Δ}_{Limit}$ und ${Δ}_{2} < {Δ}_{Limit ,}$ befanden sich der Ausgangs-Ösophagusballondruck Peso₀ und damit auch die Fluidfüllmenge V₀ im Ausgangszustand in dem in der Fig. 5b) durch die Linien a und b begrenzten "optimalen Bereich" zwischen den Messpunkten M3 und M7, in dem die Druckdifferenz dP bei (kleinen) Änderungen der Fluidfüllmenge in der Ballonsonde 46 im Wesentlichen konstant ist.

Die Fluidfüllmenge V₀ in der Ballonsonde 46 entsprach daher im Ausgangszustand im Wesentlichen der gewünschten optimalen Fluidfüllmenge Vₒₚₜ. Es ist daher nicht notwendig, die Fluidfüllmenge V in der Ballonsonde 46 anzupassen.

In einem folgenden Schritt 514 wird die Fluidfüllmenge V in der Ballonsonde 46 daher wieder auf die im Ausgangszustand in der Ballonsonde 46 vorhandene Fluidfüllmenge V₀ gebracht.

Nachdem die Fluidfüllmenge V in der Ballonsonde 46 auf die im Ausgangszustand in der Ballonsonde 46 vorhandene Fluidfüllmenge V₀ gebracht worden ist, meldet die Füllmengenüberwachung 470 im folgenden Schritt 516 der Beatmungssteuerung 180, dass eine Anpassung der Fluidfüllmenge V in der Ballonsonde 46 nicht erforderlich ist, und die Beatmung fortgesetzt werden kann, ohne die Fluidfüllmenge V in der Ballonsonde 46 zu verändern.

Alternativ oder zusätzlich zur Bestimmung der Differenz Δ_{1,2} zwischen dem ersten bzw. zweiten Vergleichs-Ösophagusballondruck Peso_{1,2} und dem Ausgangs-Osophagusballondruck Peso₀ kann die Füllmengenüberwachung 470 im Schritt 510 einen ersten Gradienten grad₁ = Δp/ΔV der Veränderung zwischen dem ersten Vergleichs-Ösophagusballondruck Peso₁ und dem Ausgangs-Ösophagusballondruck Peso₀ sowie einen zweiten Gradienten grad₂ = Δp/ΔV der Veränderung zwischen dem zweiten Vergleichs-Ösophagusballondruck Peso₂ und dem Ausgangs-Ösophagusballondruck Peso₀ bestimmen und die auf diese Weise bestimmten Gradienten grad_{1,2} im Schritt 512 mit einem vorgegebenen Gradienten-Schwellenwert gradₗᵢₘᵢₜ vergleichen.

Wenn die Absolutbeträge des ersten und des zweiten Gradienten kleiner als der vorgegebene Gradienten-Schwellenwert sind $\left|{grad}_{1 , 2}\right| < {grad}_{limit ,}$ wird angenommen, dass sich der Ausgangs-Ösophagusballondruck Peso₀ und damit auch die Fluidfüllmenge V₀ im Ausgangszustand in dem in der Fig. 5b) durch die Linien a und b begrenzten "optimalen Bereich" zwischen den Messpunkten M3 und M7 befindet, in dem die Druckdifferenz dP bei (kleinen) Änderungen der Fluidfüllmenge V in der Ballonsonde 46 im Wesentlichen konstant ist.

Die Menge V₀ an Messfluid in der Ballonsonde 46 im Ausgangszustand entsprach daher der gewünschten Fluidfüllmenge Vₒₚₜ, so dass es nicht notwendig ist, die Fluidfüllmenge V in der Ballonsonde 46 anzupassen. Auch in diesem Fall wird die Fluidfüllmenge V in der Ballonsonde 46 im Schritt 514 wieder auf die im Ausgangszustand in der Ballonsonde 46 vorhandene Fluidfüllmenge V₀ gebracht. Danach meldet die Füllmengenüberwachung 470 der Beatmungssteuerung 180 im Schritt 514, dass eine Anpassung der Fluidfüllmenge V in der Ballonsonde 46 nicht erforderlich ist und dass die Beatmung fortgesetzt werden kann, ohne Fluidfüllmenge V in der Ballonsonde 46 zu verändern.

Wenn dagegen wenigstens eine der Differenzen Δ_{1,2} zwischen dem ersten oder zweiten Vergleichs-Ösophagusballondruck Peso_{1,2} und dem Ausgangs-Ösophagusballondruck Peso₀ größer als der vorgegebenen Grenzwert Δₗᵢₘᵢₜ ist, und/oder wenn wenigstens einer der Gradienten grad_{1,2} größer als der vorgegebene Gradienten-Schwellenwert ist (grad_{1,2} > gradₗᵢₘᵢₜ), deutet dies darauf hin, dass sich die Fluidfüllmenge V₀ in der Ballonsonde 46 im Ausgangszustand außerhalb des in der Fig. 5b) durch die Linien a und b begrenzten vorteilhaften Bereichs zwischen dem Messpunkten M3 und M7 befand.

Um die Fluidfüllmenge V in der Ballonsonde 46 so anzupassen, dass sich die Fluidfüllmenge V in der Ballonsonde 46 wieder in dem in der Fig. 5b) durch die Linien a und b begrenzten, als vorteilhaft erkannten Bereich zwischen den Messpunkten M3 und M7 befindet, steuert die Füllmengenüberwachung 470 in diesem Fall im Schritt 518 die Befüllungssteuerung 490 eines Fluidbefüllungssystems, das ebenfalls Teil des Ösophagusballonsteuersystems 400 ist, so an, dass die Fluidmenge V in der Ballonsonde 46 durch die Befüllungssteuerung 490 von Neuen auf den vorgegebenen Wert Vₒₚₜ eingestellt wird. Die vorgegebene Fluidfüllmenge Vₒₚₜ kann größer oder kleiner als die Fluidfüllmenge V₀ im Ausgangszustand sein.

Um die Fluidmenge V in der Ballonsonde 46 anzupassen, wird die Befüllungssteuerung 490 vorzugsweise so angesteuert, dass die Fluidmenge V in der Ballonsonde 46 gegenüber der im Ausgangszustand in der Ballonsonde 46 vorhandenen Fluidmenge V₀ in Richtung zu derjenigen von der ersten und der zweiten Vergleichsmenge V₁, V₂ verschoben wird, für die der Gradient grad_{1,2} mit dem größeren Absolutbetrag |grad_{1,2}| bestimmt worden ist.

Darüber hinaus meldet die Füllmengenüberwachung 470 der Beatmungssteuerung 180 im Schritt 520, dass eine Anpassung der Fluidfüllmenge V in der Ballonsonde 46 erforderlich ist. Die Beatmungssteuerung 180 kann die Beatmung daraufhin vorübergehend aussetzen oder ohne Änderungen der Beatmungsmodi oder Beatmungsparameter weiterführen, bis die Fluidfüllmenge V in der Ballonsonde 46 durch die Befüllungssteuerung 490 auf einen neuen gewünschten Wert Vₒₚₜ verändert worden ist.

Nach dem Anpassen der Fluidfüllmenge V in der Ballonsonde 46 kann die Fluidmengen-Überwachungsprozedur 500 zum Überwachen der Fluidmenge V wiederholt werden. Führt dies erneut zu dem Ergebnis, dass die Fluidfüllmenge V in der Ballonsonde 46 angepasst werden muss, kann die Fluidfüllmenge V in der Ballonsonde 46 erneut durch die Befüllungssteuerung 490 angepasst werden.

Alternativ kann eine erneute Kalibrierung 100 des Ösophagusballonkatheters 45 durchgeführt werden, wie sie unter Bezugnahme auf die Figur 3 beschrieben worden ist.

Eine erneute Kalibrierung 100 des Ösophagusballonkatheters 45 kann insbesondere dann durchgeführt werden, wenn wenigstens eine der Differenzen Δ_{1,2} zwischen dem ersten oder zweiten Vergleichs-Ösophagusballondruck Peso_{1,2} und dem Ausgangs-Ösophagusballondruck Peso₀ größer als ein zweiter vorgegebenen Grenzwert Δₗᵢₘᵢₜ₂ ist (Δ_{1,2} > Δₗᵢₘᵢₜ₂), der größer als der zuvor erwähnte (erste) Grenzwert Δₗᵢₘᵢₜ ist (Δₗᵢₘᵢₜ₂ > Δₗᵢₘᵢₜ), oder wenn wenigstens einer der Gradienten grad_{1,2} größer als ein zweiter vorgegebener Gradienten-Schwellenwert gradₗᵢₘᵢₜ₂ ist (grad_{1,2} > gradₗᵢₘᵢₜ₂), der größer als der zuvor erwähnte (erste) Schwellenwert ${grad}_{limit} ist \left({grad}_{limit 2}>{grad}_{limit}\right) .$

Eine erneute Kalibrierung 100 des Ösophagusballonkatheters 45 kann auch dann durchgeführt werden, wenn ein wiederholtes Anpassen der Fluidfüllmenge V in der Ballonsonde 46 durch die Befüllungssteuerung 490 nicht zu dem gewünschten Ergebnis führt.

Führt auch eine erneute, ggf. wiederholte, Kalibrierung 100 des Ösophagusballonkatheters 45 nicht zu dem gewünschten Ergebnis, zeigt das Ösophagusballonsteuersystem 400 an, dass der Ösophagusballonkatheter 45 ersetzt werden muss.

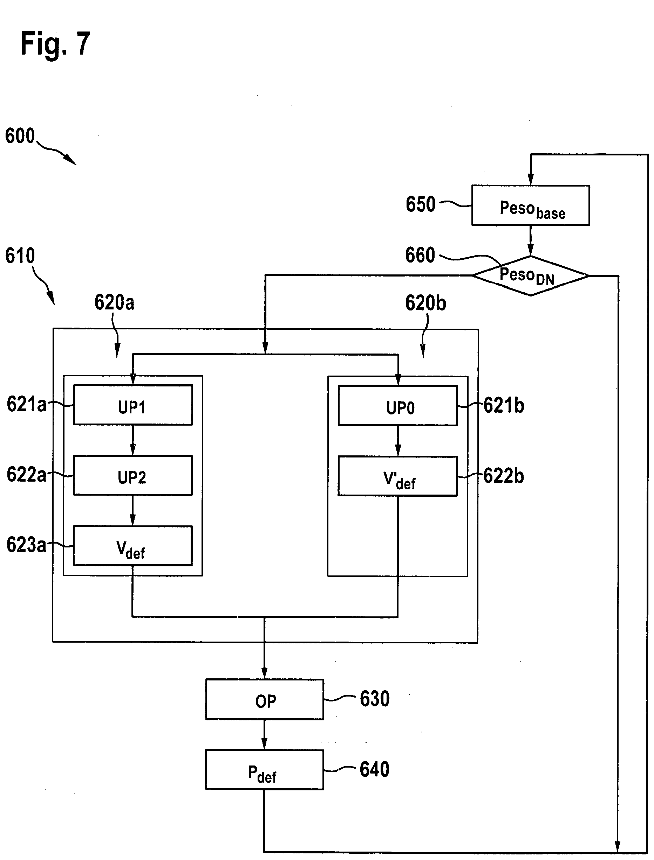

Fig. 7 zeigt ein Flussdiagramm, das den Ablauf einer Fluid-Befüllungsprozedur 600 veranschaulicht, wie sie von der Befüllungssteuerung 490 des Fluidbefüllungssystems gemäß einer Ausführungsform der vorliegenden Offenbarung nach einer Ansteuerung durch das Ösophagusballonsteuersystem 400 und/oder die Beatmungssteuerung 180 durchführt wird, um die Ballonsonde 46 mit einer vorbestimmten Menge V_{def} an Messfluid zu befüllen.

Die vorbestimmte Menge V_{def} an Messfluid ist hierbei eine jeweils gewünschte Befüllung V an Messfluid, im Sinne einer Soll-Fluidmenge bei einer Regelung der in die Ballonsonde 46 eingeleiteten Menge an Messfluid. Die vorbestimmte Menge V_{def} an Messfluid wird häufig eine als optimal angesehene Befüllung Vₒₚₜ sein. Die vorbestimmte Menge V_{def} an Messfluid kann beispielsweise durch eine zuvor durchgeführte Kalibrierungsprozedur 100, wie sie zuvor unter Bezugnahme auf die Figur 3 beschrieben worden ist, ermittelt worden sein.

Die Befüllungssteuerung 490 kann als eigenständiges Bauteil "in Hardware" realisiert werden. Alternativ kann die Befüllungssteuerung 490 auch als Computerprogrammprodukt, d.h. durch ein entsprechendes Softwareprogramm, realisiert werden, das auf einem Prozessor, insbesondere einem Mikroprozessor oder Mikrocontroller, ausgeführt wird. In diesem Fall kann die Software auf einem geeigneten lokalen oder über ein Netzwerk abrufbaren Speichermedium vorgehalten werden. Die Software enthält als Computerprogramm codierte Anweisungen, die dann, wenn die Software in einen Arbeitsspeicher des Prozessors geladen und in Maschinensprache übersetzt wird, den Prozessor dazu veranlasst, die hierin näher beschriebenen Prozeduren auszuführen. Mischformen zwischen Realisierung in Hardware und Realisierung in Software sind selbstverständlich ebenso denkbar. Der Mikroprozessor oder Mikrocomputer kann zu einer Steuerung des Fluidbefüllungssystems gehören und je nach Ausgestaltung des Fluidbefüllungssystems der Ösophagusballonsteuersystem 400 und/oder der Beatmungssteuerung 180 zugeordnet sein.

Um eine Vergleichbarkeit und Reproduzierbarkeit des nach Abschluss eines Befüllungsvorgangs jeweils erreichten Füllzustands der Ballonsonde 46 sicherzustellen, kann die Befüllungssteuerung 490 derart ausgebildet sein, dass sie vor dem Befüllen der Ballonsonde mit Messfluid eine Entleerungsprozedur 610 durchführt, um das in der Ballonsonde 46 befindliche Messfluid aus der Ballonsonde 46 zu entnehmen.

Die Entleerungsprozedur 610 kann beispielsweise umfassen, dass die Befüllungssteuerung 490 durch Öffnen des Ventils 420 des Ösophagusballonsteuersystem 400 (siehe Fig. 1) unter Überdruck stehendes Messfluid aus der Ballonsonde 46 ausströmen lässt, bis ein Druckausgleich mit der Umgebung erfolgt ist.

Einfacher und schneller lässt sich eine Entleerung der Ballonsonde 46 mit Hilfe der Pumpanordnung 430 des Ösophagusballonsteuersystems 400 (siehe Fig. 1) durchführen, die von der Befüllungssteuerung 490 des Fluidbefüllungssystems angesteuert wird, um das Messfluid aus der Ballonsonde 46 abzupumpen, bis ein vorbestimmter Enddruck erreicht ist. Der Enddruck kann auch ein Unterdruck, d.h. ein Druck, der niedriger als der Umgebungsdruck ist, sein.

Die Entleerungsprozedur 610 zur Entnahme von in der Ballonsonde 46 befindlichem Messfluid kann auch eine Prozedur 620a zur Einstellung eines sogenannten Nullvolumens V_{Null} umfassen. Der Begriff "Nullvolumen V_{Null}" bezeichnet eine vorbestimmte Befüllung des Ösophagusballonkatheters 45 mit Messfluid, bei der im Wesentlichen nur die Zuleitungen zu der Ballonsonde 46 mit Messfluid gefüllt sind, die Ballonsonde 46 selbst aber noch vollständig kollabiert ist und kein Messfluid enthält.

Ist das Nullvolumen V_{Null} bestimmt worden, kann es in jedem Befüllungsvorgang als reproduzierbarer Startpunkt dienen. Es ist denkbar, dass sich Nullvolumen V_{Null} auf einen bestimmten vorgegebenen Druck, beispielsweise den Umgebungsdruck, bezieht.

Eine Prozedur 620a zum Einstellen eines Nullvolumens V_{Null} kann insbesondere die folgenden Schritte umfassen:
Schritt 621a: Entnehmen von Messfluid aus der Ballonsonde 46, bis ein erster vorbestimmter Unterdruck up, in der Ballonsonde 46 erreicht ist;
Schritt 622: Einleiten von Messfluid in die Ballonsonde 46, bis ein zweiter vorbestimmter Unterdruck up₂ in der Ballonsonde 46 erreicht ist, der größer, d.h. weniger negativ, als der erste vorbestimmte Unterdruck (up₂ > up,) ist; und
Schritt 623: Einleiten einer vorbestimmten Menge V_{def} an Messfluid in die Ballonsonde 46. Der Ballon der Ballonsonde 46 soll dabei sowohl beim Einstellen des ersten Unterdrucks up, im Schritt 621a, als auch beim Einstellen des zweiten Unterdrucks up₂ im Schritt 622a vollständig kollabiert sein.

Wenn das "Totvolumen Vₜₒₜ" des verwendeten Ösophagusballonkatheters 45 bekannt ist, kann die Befüllungssteuerung 490 des Fluidbefüllungssystems in einer alternativen Prozedur 620b zur Einstellung eines Nullvolumens V_{Null} auch so vorgehen, dass der Ösophagusballonkatheter 45 in einem ersten Schritt 622a weitgehend evakuiert wird, indem der Ösophagusballonkatheter 45, z.B. durch die Pumpanordnung 430, auf einen möglichst geringen Unterdruck up₀ abgepumpt wird und ausgehend von dem so erreichten Zustand eine vorbestimmte Menge an Messfluid V'_{def} in den Ösophagusballonkatheter 45 eingeleitet wird (Schritt 622b). Dabei entspricht die vorbestimmte Menge an Messfluid V'_{def} dem Totvolumen Vₜₒₜ multipliziert mit einem Bezugsdruck für das Nullvolumen, beispielsweise dem Umgebungsdruck p_{ambient}: ${{V}^{′}}_{def} = {V}_{tot} \times {p}_{ambient .}$

Das Totvolumen Vₜₒₜ hängt von spezifischen Parametern des jeweiligen Ösophagusballonkatheters 45, beispielsweise von der Länge und dem Durchmesser der Förderleitung 66 des jeweiligen Ösophagusballonkatheters 45 ab. Das Totvolumen Vₜₒₜ muss daher für den jeweiligen Ösophagusballonkatheter 45, bzw. für die jeweilige Baureihe des Ösophagusballonkatheters 45, in aller Regel vorab experimentell bestimmt werden.

Die Befüllungssteuerung 490 kann darüber hinaus auch derart ausgebildet sein, dass die Fluid-Befüllungsprozedur 600, nachdem eine der zuvor beschriebenen Prozeduren 620a, 620b zur Entnahme des in der Ballonsonde 46 befindlichen Messfluids durchgeführt worden ist, in einem optionalen folgenden Schritt 630 Messfluid in die Ballonsonde 46 einleitet, bis ein vorbestimmter Überdruck in der Ballonsonde 46 erreicht ist.

Das Einleiten von Messfluid in die Ballonsonde 46, bis ein vorbestimmter Überdruck op erreicht ist, erfolgt vor dem eigentlichen Einstellen einer vorbestimmten Menge V_{def} an Messfluid in der Ballonsonde 46. Es handelt sich hierbei also um einen Zwischenschritt 630, der zwischen dem Entleeren der Ballonsonde 46, wie es zuvor beschrieben worden ist, und dem im Schritt 640 folgenden Einstellen der vorbestimmten Menge V_{def} an Messfluid in der Ballonsonde 46 durchgeführt wird.

Nachdem in diesem Zwischenschritt 630 der vorbestimmte Überdruck op erreicht worden ist, kann in einem folgenden Schritt 640, entweder passiv durch Ausströmenlassen des Messfluids bei geöffnetem Ventil 420 oder durch aktives Abpumpen des Messfluids, so viel Messfluid aus der Ballonsonde 46 entnommen werden, dass ein vorbestimmter Ballondruck p_{def} erreicht wird, der der vorbestimmten Menge V_{def} an Messfluid in der Ballonsonde 46 entspricht.

In einer weiteren Ausgestaltung kann das Ösophagusballonsteuersystem 400 dazu ausgebildet sein, fortlaufend oder wiederholt einen Basis-Ösophagusballondruck Peso_{base} (Schritt 650) zu erfassen, den erfassten Basis-Ösophagusballondruck Peso_{base} mit einem vorgegebenen Bezugsniveau Peso_{PN} zu vergleichen (Schritt 660), und die Befüllungssteuerung 490 zur Durchführung der zuvor beschriebenen Fluid-Befüllungsprozedur 600 zur Befüllung der Ballonsonde 46 mit Messfluid anzusteuern, wenn festgestellt wird, dass der Basis-Ösophagusballondruck Peso_{base} um mindestens einen vorbestimmten Betrag gegenüber dem vorgegebenen Bezugsniveau Peso_{PN} abgesunken ist. Die Befüllung der Ballonsonde 46 kann dabei bei fortlaufender Beatmung vorgenommen werden, wenn als Referent für den Ösophagusballondruck Peso immer der Basis-Ösophagusballondruck Peso_{base} herangezogen wird.

Mit dem Begriff "Basis-Ösophagusballondruck Peso_{base}" ist hierbei ein Druck oder eine Druckdifferenz gemeint, der/die sich über eine Vielzahl aufeinander folgender Beatmungszyklen möglichst nicht verändert und der/die deswegen als Maß für eine Veränderung des Füllungszustands der Ballonsonde dienen kann.

Der Basis-Ösophagusballondruck Peso_{base} sollte insbesondere ein von dem Druckermittlungssystem 300 erfassbarer Ösophagusballondruck Peso sein, dem kein Beatmungsprofil überlagert ist, welches die Inspirationsvorgänge und Exspirationsvorgänge während der Beatmung wiedergibt.

Beispielsweise kann der Ösophagusballondruck Peso_ee, der am Ende der Exspirationsphasen aufeinander folgender Beatmungszyklen gemessen wird, als "Basis-Ösophagusballondruck" Peso_{base} definiert werden. Alternativ kann auch der Ösophagusballondruck Peso_ei, der am Ende der Inspirationsphase aufeinander folgender Beatmungszyklen gemessen wird, als Basis-Ösophagusballondruck Peso_{base} definiert werden.

Eine derart ausgestaltete Befüllungssteuerung 490 überwacht fortlaufend, ob der einmal eingestellte Befüllungszustand der Ballonsonde 46, der beispielsweise durch die zuvor beschriebene Kalibrierungsprozedur 100 ermittelt und eingestellt worden ist, stabil bleibt. Die Befüllungssteuerung 490 reagiert mit einer entsprechenden Korrektur der Fluidbefüllung V in der Ballonsonde 46, wenn der aktuell festgestellte Basis-Ösophagusballondruck Peso_{base} um einen vorbestimmten Wert oder mehr gegenüber von dem vorgegebenen Bezugsniveau Peso_{PN} abweicht.

Auf diese Weise können Verluste an Messfluid in der Ballonsonde 46, wie sie beispielsweise durch eine Leckage entstehen können, ausgeglichen werden, jedenfalls solange der Verlust an Messfluid aus der Ballonsonde 46 gewissen Grenzen nicht überschreitet.

Eine derartige Befüllungssteuerung 490 ermöglicht es auch, eine Verlust- bzw. Leckagerate abzuschätzen, beispielsweise indem überwacht wird, in welchem Zeitraum der gemessene Basis-Ösophagusballondruck Peso um einen vorbestimmten Betrag oder mehr absinkt, oder indem überwacht wird, wie stark der gemessene Basis-Ösophagusballondruck Peso_{base} nach einer vorbestimmten Zeitdauer abgesunken ist.

Ist die Verlustmenge oder Leckagerate an Messfluid bekannt, kann der Verlust an Messfluid in der Ballonsonde 46 dadurch kompensiert werden, dass eine vorgegebene Menge an Messfluid in die Ballonsonde 46 nachgefüllt wird. Die vorgegebene Menge kann sich aus der zuvor bestimmten Verlustmenge oder Leckagerate ergeben, die vorgegebene Menge kann sich insbesondere daraus ergeben, dass die zuvor bestimmte Verlustmenge oder Leckagerate, mit der zugehörigen Zeitdauer, über welche der Verlust festgestellt worden ist, multipliziert wird.

Das Nachfüllen von Messfluid kann auch derart erfolgen, dass ein mit dem Einleiten von Messfluid in die Ballonsonde 46 einhergehender Anstieg des Ösophagusballondrucks Peso überwacht wird und das Nachfüllen beendet wird, sobald ein vorbestimmter Ösophagusballondruck Pesoₛₒₗₗ erreicht worden ist.

Übersteigt die bestimmte Verlustmenge oder Leckagerate eine vorgegebene Schwelle, die nicht mehr ersetzt werden kann, zeigt das Ösophagusballonsteuersystem 400 an, dass der Ösophagusballonkatheter 45 ersetzt werden muss.

Wie erwähnt, sind die Beatmungssteuerung 180 und das Ösophagusballonsteuersystem 400 einer offenbarungsgemäßen Beatmungsvorrichtung 10 so ausgebildet,
dass die Beatmungssteuerung 180 das Ösophagusballonsteuersystem 400 automatisch ansteuern kann und/oder so, dass das Ösophagusballonsteuersystem 400 die Beatmungssteuerung 180 automatisch ansteuern kann.

Die Beatmungssteuerung 180 und das Ösophagusballonsteuersystem 400 können insbesondere dazu ausgebildet sein, bidirektional miteinander zu kommunizieren und insbesondere zu interagieren.

Dass die Beatmungssteuerung 180 zur automatisierten Ansteuerung des Ösophagusballonsteuersystems 400 ausgebildet ist, beinhaltet, dass die Beatmungssteuerung 180 neben ihrer bestimmungsgemäßen steuernden und/oder regelnden Funktion hinsichtlich der Beatmung an sich auch eine steuernde und/oder regelnde Funktion mit Blick auf den Ösophagusballonkatheter 45, insbesondere mit Blick auf dessen Ballonsonde 46, hat.

Dies umfasst insbesondere, dass die Beatmungssteuerung 180 das Ösophagusballonssteuersystem 400 derart ansteuert, dass das Ösophagusballonssteuersystem 400 vorbestimmte Prozeduren startet. Diese vorbestimmten Prozeduren können insbesondere Prozeduren zum Kalibrieren der Ballonsonde 46 (Kalibriesteuerung 450), Prozeduren zur Befüllung der Ballonsonde 46 mit Messfluid (Befüllungssteuerung 490), und/oder Prozeduren zur laufenden Überwachung eines Füllungszustands der Ballonsonde (Füllmengenüberwachung 470) umfassen, wie sie zuvor beschrieben worden sind.

Zusätzlich oder alternativ kann die Beatmungssteuerung 180 auf den Ablauf der Prozeduren, die von dem Ösophagusballonssteuersystem 400 abgearbeitet werden, steuernden und/oder regelnden Einfluss nehmen. Ein solcher steuernder bzw. regelnder Einfluss kann beispielsweise umfassen, dass die Beatmungssteuerung 180 dem Ösophagusballonsteuersystem 400 Werte von aktuell eingestellten Beatmungsparametern zur Verfügung stellt und das Ösophagusballonsteuersystem 400 auf deren Grundlage dieser Werte vorbestimmte Steuerprozeduren, insbesondere wenigstens einer der oben genannten Steuerprozeduren, die auf den Ösophagusballon der Ballonsonde 46 einwirken, startet, steuert, regelt und/oder beendet.

Durch eine bidirektionale Kommunikation und/oder Interaktion zwischen der Beatmungssteuerung 180 und dem Ösophagusballonsteuersystem 400 kann ein optimales Zusammenspiel zwischen der Beatmungssteuerung 180 und dem Ösophagusballonsteuersystem 400 erreicht werden, wobei die jeweils ausgeführten Prozeduren aufeinander abgestimmt und synchronisiert ablaufen. Insbesondere können die von dem Ösophagusballonsteuersystem 400 ausgeführten Steuerprozeduren, die auf den Ösophagusballonkatheter 45 einwirken, auf diese Weise durchgeführt werden, ohne dabei eine laufende Beatmung zu unterbrechen.

Über die bidirektionale Verbindung 150 kann die Beatmungssteuerung 180 dem Ösophagusballonsteuersystem 400 auch Informationen zur Verfügung stellen, die für den Ablauf von bestimmten Steuerprozeduren für den Ösophagusballonkatheter 45 von Bedeutung sind. Dies erlaubt es, Veränderungen von Beatmungsparametern oder erfassten Größen, die während der Beatmung auftreten, beim Ablauf der Steuerprozeduren im Ösophagusballonsteuersystem 400 zu berücksichtigen. Die Steuerprozeduren können insbesondere die zuvor erwähnten Steuerprozeduren zum Kalibrieren der Ballonsonde 46, zum Befüllen der Ballonsonde 46 und zur Überwachung der Fluidmenge in der Ballonsonde 46 umfassen.

Ein Beispiel dafür, wie Veränderungen von Beatmungsparametern oder Größen, die während der Beatmung erfasst werden, beim Ablauf der Steuerprozeduren im Ösophagusballonsteuersystem 400 berücksichtigt werden können, ist, dass bei manchen Beatmungsmodi die Beatmungssteuerung 180 das im Laufe der maschinellen Beatmung applizierte Tidalvolumen V_{tidal} von Atemzug zu Atemzug anpasst bzw. neu einstellt. In diesen Beatmungsmodi ist das Tidalvolumen V_{tidal} daher kein über die gesamte Zeit der Beatmung konstanter Beatmungsparameter, sondern eine Größe, die sich von Atemzug zu Atemzug verändert.

Je nach Beatmungsmodus spricht man anstelle von Atemzug auch von Beatmungszug, Atemzyklus oder Beatmungszyklus. Im Folgenden werden die Begriffe Atemzug, Beatmungszug, Atemzyklus und Beatmungszyklus daher synonym verwendet.

Das im jeweiligen Atemzug applizierte Tidalvolumen V_{tidal} hat auch einen Einfluss auf andere Größen, etwa den Tidaldruck p_{tidal}, der die Druckdifferenz in der Lunge zwischen dem Ende der Inspirationsphase und dem Ende der Exspirationsphase beschreibt. Das jeweils applizierte Tidalvolumen V_{tidal} kann auch Einfluss auf die Durchführung der Steuerprozeduren des Ösophagusballonsteuersystems 400 haben.

Beispielsweise wird bei einer in vivo erfolgender Kalibrierung der optimalen Menge Vₒₚₜ an Messfluid in der Ballonsonde 46 des Ösophagusballonkatheters 45 vorausgesetzt, dass sich der Tidaldruck p_{tidal} während der Kalibrierung nicht verändert. Daher sollte während der Kalibrierung auch das in jedem Atemzug applizierte Tidalvolumen V_{tidal} möglichst konstant bleiben.

Aus diesem Grund wäre eine Kalibrierung der Ballonsonde 46 des Ösophagusballonkatheters 45 an sich nur in Beatmungsmodi möglich, in denen das applizierte Tidalvolumen V_{tidal} konstant gehalten wird.

Eine Beatmungsvorrichtung 10, die gemäß einem Ausführungsbeispiel der Offenbarung mit einer bidirektionalen Kombination zwischen der Beatmungssteuerung 180 und dem Ösophagusballonsteuersystem 400 ausgebildet ist, erlaubt es jedoch der Beatmungssteuerung 180, die Größe des in jedem Atemzug applizierten Tidalvolumens V_{tidal} an das Ösophagusballonsteuersystem 400 zu übermitteln. Folglich können Veränderungen des der Ballonsonde 46 jeweils zugeführten Tidalvolumens V_{tidal} bei der Kalibrierung des Ösophagusballonkatheters 45 berücksichtigt werden.

Dies kann beispielsweise dadurch geschehen, dass ein jeweils erfasster Wert für die Differenz zwischen dem Ösophagusballondruck Peso_ei am Ende der Inspirationsphase und dem Ösophausballondruck am Ende der Exspirationsphase Peso_ee auf das im jeweiligen Atemzug applizierte Tidalvolumen V_{tidal} bezogen wird. Insbesondere kann der Quotient aus der Druckdifferenz und dem Tidalvolumen V_{tidal} als Bezugsgröße verwendet werden. Dies ermöglicht eine Kalibrierung der Ballonsonde 46 auch während weiterlaufender Beatmung in Beatmungsmodi, in denen das Tidalvolumen V_{tidal} nicht konstant ist.

Neben der automatisierten Einstellung von Beatmungsparametern, wie dem positiven endexpiratorischen Druck PEEP oder dem maximalen Atemwegsdruck Paw_max, kann auch vorgesehen sein, dass die Beatmungsvorrichtung 10 zur weitgehend automatisierten Darstellung von für die Beatmung relevanten Informationen ausgebildet ist. Die Darstellung solcher Informationen kann die Anzeige bestimmter Beatmungsparameter wie z.B. dem transpulmonalen Druck Ptp auf einem Display der Beatmungsvorrichtung 10 umfassen. Optional können noch weitere Informationen angezeigt werden, z.B. die Information, ob der Ösophagusballonkatheter 45 im normalen Modus betrieben wird, oder in einem Sondermodus, insbesondere einer Kalibrierung 100, einer Fluidmengen-Überwachungsprozedur 500 und/oder einer Fluid-Befüllungsprozedur 600.

Die Beatmungssteuerung 180 kann insbesondere dazu ausgelegt sein, das Ösophagusballonsteuersystem 400 nach Maßgabe von Druckwerten zu steuern und/ oder regeln, die von dem Druckermittlungssystem 300 geliefert worden sind. Diese Interaktion kann insbesondere bidirektional erfolgen. Dabei kann die Intelligenz des Gesamtsystems, das die Beatmungssteuerung 180 und das Ösophagusbal-Ionsteuersystem 400 umfasst, weitestgehend auf Seiten der Beatmungssteuerung 180 liegen, so dass die Beatmungssteuerung 180 in diesem Fall alle Steuer- und/ oder Regelprozeduren durchführt, einschließlich der den Ösophagusballonkatheter 45 betreffenden Steuer- und/oder Regelprozeduren wie z.B. das Kalibrieren 100 des Ösophagusballonkatheters 45, das Überwachen der Fluidmenge im Ösophagusballon der Ballonsonde 46 (Fluidmengen-Überwachungsprozedur 500) und ein mögliches Nachfüllen bzw. Befüllen der Fluidmenge im Ösophagusballon der Ballonsonde 46 (Fluid-Befüllungsprozedur 600). In einer solchen Konfiguration ist das Ösophagusballonsteuersystem 400 lediglich zum Ausführen von Steuer- und/oder Regelbefehlen ausgebildet, die von der Beatmungssteuerung 180 an das Ösophagusballonsteuersystem 400 geliefert werden.

In anderen Ausführungsbeispielen kann ein größerer Teil der Intelligenz des Gesamtsystems 10 im Ösophagusballonsteuersystem 400 realisiert sein. Insbesondere kann in solchen Ausführungsbeispielen das Ösophagusballonsteuersystem 400 den Ösophagusballonkatheter 45 betreffende Steuer/Regelprozeduren wie z.B. die Kalibrierung 100 des Ösophagusballonkatheters 45, das Überwachen 500 der Fluidmenge im Ösophagusballon der Ballonsonde 46 durch die Fluidmengenüberwachung 470 und ein mögliches Nachfüllen 600 der Fluidmenge im Ösophagusballon der Ballonsonde 46 durch die Befüllungssteuerung 490 weitestgehend selbstständig durchführen, nachdem es von der Beatmungssteuerung 180 dazu aufgefordert ("getriggert") worden ist.

Dies kann insbesondere beinhalten, dass, wie zuvor beschrieben, Parameter und Messgrößen von der Beatmungssteuerung 180 an das Ösophagusballonsteuersystem 400 übertragen und von diesem bei der Ausführung der den Ösophagusballonkatheter 45 betreffende Steuer- und/oder Regelprozeduren berücksichtigt werden. Umgekehrt können Parameter und Messgrößen von dem Ösophagusbal-Ionsteuersystem 400 an die Beatmungssteuerung 180 übertragen werden, um die Beatmung auf Grundlage der von dem Ösophagusballonsteuersystem 400 an die Beatmungssteuerung 180 übertragenen Parameter und Messgrößen zu steuern und bei Bedarf anzupassen, wie es zuvor beschrieben worden ist.

In Ausführungsbeispielen, in denen das Druckermittlungssystem 300 wenigstens teilweise dem Ösophagusballonsteuersystem 400 zugeordnet ist, kann das Ösophagusballonsteuersystem 400 auch dazu ausgebildet sein, Signale an die Beatmungssteuerung 180 zu liefern, die den in der Ballonsonde 46 erfassten Ösophagusballondruck pₑₛₒ wiedergeben.

In weiteren Ausführungsbeispielen kann ein noch größerer Teil der Intelligenz und Steuerung des Gesamtsystems im Ösophagusballonsteuersystem 400 realisiert sein.

So wird mit der Formulierung, dass das Ösophagusballonsteuersystem 400 auch zur automatisierten Ansteuerung der Beatmungssteuerung 180 ausgebildet ist, zum Ausdruck gebracht, dass das Ösophagusballonsteuersystem 400 über die gegebenenfalls vorgesehene Übermittlung von Signalen, die den in der Ballonsonde 46 erfassten Ösophagusballondruck pₑₛₒ repräsentieren, hinaus eine steuernde/regelnde Funktion auf die Beatmungssteuerung 180 ausübt.

Das Ösophagusballonssteuersystem 400 kann zum Beispiel die Beatmungssteuerung 180 so ansteuern, dass sie vorbestimmte Prozeduren startet. Zusätzlich oder alternativ kann das Ösophagusballonssteuersystem 400 den Ablauf von bestimmten Prozeduren, die von der Beatmungssteuerung 180 ausgeführt werden, steuernd und/oder regelnd beeinflussen. Zum Beispiel kann das Ösophagusballonsteuersystem 400 die Beatmungssteuerung 180 anweisen, Werte von aktuell eingestellten Beatmungsparametern für eine bestimmte Zeit "einzufrieren" bzw. festzuhalten.

Durch "Einfrieren" oder Festhalten aktuell eingestellter Beatmungsparametern verhindert das Ösophagusballonsteuersystem 400 für eine bestimmte Zeit, dass die eingefrorenen/festgehaltenen Beatmungsparameter von der Beatmungssteuerung 180 verändert werden können, während das Ösophagusballonsteuersystem 400 bestimmte Prozeduren, insbesondere eine der zuvor erwähnten Prozeduren zum Kalibrieren 100 der Ballonsonde 46, zum Befüllen der Ballonsonde 46 (Fluid-Befüllungsprozedur 600) und/oder zum Überwachen der Fluidmenge in der Ballonsonde 46 (Fluidmengen-Überwachungsprozedur 500), ausführt.

Das Ösophagusballonssteuersystem 400 kann dazu die Beatmungssteuerung 180 so ansteuern, dass sie nach einer Aktivierung bzw. nach dem Beginn einer Ösophagusballonsteuerprozedur 100, 500, 600 keine Veränderung vorbestimmter Beatmungsparameter und/oder Beatmungsmodi zulässt, bis die Ösophagusballonsteuerprozedur beendet ist. Auf diese Weise können unerwünschte Wechselwirkungen zwischen der ausgeführten Ösophagusballonsteuerprozedur 100, 500, 600 und einer gleichzeitig vorgenommenen Änderung von Beatmungsparametern und/ oder Beatmungsmodi, welche die Ausführung der Ösophagusballonsteuerprozedur 100, 500, 600 und/oder der fortlaufenden Beatmung beeinträchtigen können, vermieden werden.

Die Beatmungssteuerung 180 kann auch so konfiguriert sein, dass sie ab einem vorbestimmten Zeitpunkt vor der geplanten Aktivierung oder dem geplanten Beginn einer Ösophagusballonsteuerprozedur 100, 500, 600 keine Veränderungen vorbestimmter Beatmungsparameter 192A, 192B, 192C, 192D und/oder Beatmungsmodi 190A, 190B, 190C, 190D zulässt, bis die geplante Ösophagusballonsteuerprozedur 100, 500, 600 beendet ist. Um ein Blockieren der Beatmungssteuerung 180 zu verhindern, kann die Beatmungssteuerung 180 wieder eine Veränderung der vorbestimmten Beatmungsparameter 192A, 192B, 192C, 192D und/oder Beatmungsmodi 190A, 190B, 190C, 190D zulassen, wenn die Ösophagusballonsteuerprozedur 100, 500, 600 nicht innerhalb einer vorbestimmten Zeit begonnen hat.

Wenn ab einem vorbestimmten Zeitpunkt vor Beginn einer Ösophagusballonsteuerprozedur 100, 500, 600, beispielsweise durch entsprechende Benutzereingaben, eine Änderung vorbestimmter Beatmungsparameter 192A, 192B, 192C, 192D und/ oder Beatmungsmodi 190A, 190B, 190C, 190D erzwungen wird, kann die Beatmungssteuerung 180 und/oder das Ösophagusballonsteuersystem 400 eine Warnung erzeugen. Alternativ oder zusätzlich kann das Ösophagusballonsteuersystem 400 den Beginn der Ösophagusballonsteuerprozedur 100, 500, 600 verzögern, um einen von Änderungen der Beatmungsparameter 192A, 192B, 192C, 192D und/ oder Beatmungsmodi 190A, 190B, 190C, 190D nicht gestörten Ablauf der Ösophagusballonsteuerprozedur 100, 500, 600 zu ermöglichen.

Die Beatmungssteuerung 180 und/oder das Ösophagusballonsteuersystem 400 können insbesondere so konfiguriert ist, dass sie zu einem oder mehreren vorbestimmten Zeitpunkten oder in vorbestimmten Zeitintervallen eine Ösophagusballonsteuerprozedur 100, 500, 600 starten. Auf diese Weise kann der Betrieb der Beatmungsvorrichtung 10 noch weiter automatisiert werden und die Betriebssicherheit kann verbessert werden.

Die Beatmungssteuerung 180 und/oder das Ösophagusballonsteuersystem 400 können insbesondere so konfiguriert sein, in dem Fall, in dem innerhalb einer vorbestimmten Zeitdauer vor Beginn einer Ösophagusballonsteuerprozedur 100, 500, 600 eine Änderung eines vorbestimmten Beatmungsparameters 192A, 192B, 192C, 192D und/oder Beatmungsmodus 190A, 190B, 190C, 190D erfolgt, der Beginn der Ösophagusballonsteuerprozedur 100, 500, 600 solange verschoben wird, bis die vorgenommene Änderung des Beatmungsparameters 192A, 192B, 192C, 192D und/oder des Beatmungsmodus 190A, 190B, 190C, 190D abgeschlossen ist, damit die Ösophagusballonsteuerprozedur 100, 500, 600 durchgeführt werden kann, ohne dabei von den Änderungen der Beatmungsparameter 192A, 192B, 192C, 192D und/oder Beatmungsmodi 190A, 190B, 190C, 190D beeinflusst und/ oder gestört zu werden.

Auch können die Beatmungssteuerung 180 und/oder das Ösophagusballonsteuersystem 400 Änderungen wenigstens eines vorbestimmten Beatmungsparameters 192A, 192B, 192C, 192D und/oder des Beatmungsmodus 190A, 190B, 190C, 190D, die in einem vorbestimmten Zeitintervall vor oder während einer ausgewählten Ösophagusballonsteuerprozedur 100, 500, 600 vorgenommen werden sollen, zeitlich verschieben, bis eine ausgewählte, und ggf. bereits gestartete, Ösophagusballonsteuerprozedur 100, 500, 600 abgeschlossen ist, um eine Beeinflussung der Ösophagusballonsteuerprozedur 100, 500, 600 durch Änderungen wenigstens eines vorbestimmten Beatmungsparameters 192A, 192B, 192C, 192D und/oder des Beatmungsmodus 190A, 190B, 190C, 190D zu vermeiden.

Eine Kommunikation zwischen dem Ösophagusballonssteuersystem 400 und der Beatmungssteuerung 180, durch welche das Ösophagusballonssteuersystem 400 auf die Beatmungssteuerung 180 einwirkt, verbessert das Zusammenspiel zwischen der Beatmungssteuerung 180 und dem Ösophagusballonsteuersystem 400, da sie bewirkt, dass die von der Beatmungssteuerung 180 und dem Ösophagusballonsteuersystem 400 ausgeführten Prozeduren jeweils aufeinander abgestimmt sind und synchronisiert ablaufen.

Eine offenbarungsgemäß ausgebildete Beatmungsvorrichtung 10 ermöglicht auch eine genauere Berücksichtigung des im Pleuraspalt 44 herrschenden Drucks Ppl durch den Ösophagusballonkatheter 45 und eine flexible Anpassung der von dem Ösophagusballonkatheter 45 gelieferten Messwerte an sich verändernde Umgebungsbedingungen während fortlaufender Beatmung, insbesondere während einer weitgehend automatisiert ablaufenden fortlaufenden Beatmung.

Wenn die zur Beatmungsvorrichtung 10 gehörende Beatmungssteuerung 180 und das zum Ösophagusballonkatheter 45 gehörende Ösophagusballonsteuersystem 400 offenbarungsgemäß dazu ausgelegt sind, bidirektional miteinander zu kommunizieren, ist eine fortlaufende gegenseitige Überwachung der Beatmungssteuerung 180 und des Ösophagusballonsteuersystems 400 möglich. Dadurch können Fehlfunktionen frühzeitig erkannt und korrigiert werden.

Darüber hinaus können Parameter, Kontroll- und Steuergrößen der Beatmung während einer laufenden Beatmung an sich verändernde Umgebungsbedingungen angepasst werden, ohne dass dazu manuelle Eingriffe erforderlich wären. Insbesondere ist es bei einer offenbarungsgemäßen Beatmungsvorrichtung 10 nicht erforderlich, die Beatmung zu unterbrechen, um bestimmte Funktionen und/oder Prozeduren der Beatmung neu zu kalibrieren und/oder neu zu starten. Es ist auch möglich, die Füllmenge V des Messfluids in der Ballonsonde 46 anzupassen, ohne eine laufende Beatmung zu unterbrechen.

Die erwähnte Anpassung der Beatmung und des die Beatmung durchführenden Gesamtsystems schließt auch die Möglichkeit ein, den Ösophagusballonkatheter 45 im Rahmen einer Kalibrierungsprozedur 100 vollständig neu zu kalibrieren, ohne eine laufende Beatmung dafür zu unterbrechen. Eine solche Neukalibrierung ohne Unterbrechung der laufenden Beatmung ist mit einer offenbarungsgemäßen

Beatmungsvorrichtung 10 auch dann möglich, wenn die Beatmung mittels vollautomatischer Beatmungsmodi erfolgt, beispielsweise bei einer Beatmung mittels geschlossener Regelkreise, wie zum Beispiel bei der von der Anmelderin entwickelten "Adaptive Support Ventilation" ("ASV-Beatmung") und der ebenfalls von der Anmelderin entwickelten INTELLiVENT-ASV.

## Patentansprüche

1. Vorrichtung (15) zur automatisierten Einstellung eines durch eine Beatmungsvorrichtung (10) vorgegebenen Beatmungsparameters (192A, 192B, 192C, 192D), insbesondere eines Drucks, insbesondere eines positiven endexpiratorischen Drucks (PEEP) und/oder eines maximalen Atemwegsdrucks (Paw_max), und/oder zur automatisierten Darstellung von für maschinelle Beatmung relevanter Information, insbesondere eines transpulmonalen Drucks (Ptp), umfassend:
- ein Druckermittlungssystem (300) zum Ermitteln eines transpulmonalen Drucks (Ptp), insbesondere eines transpulmonalen Drucks (Ptp_ee) am Ende einer Exspirationsphase und/oder eines transpulmonalen Drucks (Ptp_ei) am Ende einer Inspirationsphase, auf Grundlage eines in den Ösophagus (34) einführbaren Ösophagusballonkatheters (45) mit Ballonsonde (46) zur Bestimmung eines Ösophagusballondrucks (Peso);
- ein Ösophagusballonsteuersystem (400) mit einem Flusssensor (410) und einer Pumpanordnung (430) zur automatisierten Überwachung und/oder Einstellung einer betriebsgemäßen Befüllung der Ballonsonde (46) des Ösophagusballonkatheters (45) in vivo, wobei die betriebsgemäße Befüllung der Ballonsonde (46) eine Befüllung der Ballonsonde (46) mit Messfluid ist, bei der die Ballonsonde (46) ein optimales Signal zur Erfassung des Ösophagusballondrucks (Peso) liefert; und
- eine Beatmungssteuerung (180),
wobei die Beatmungssteuerung (180) zur Einstellung des durch die Beatmungsvorrichtung (10) vorgegebenen Beatmungsparameters (192A, 192B, 192C, 192D) und/oder zur Darstellung der für maschinelle Beatmung relevanten Information auf Grundlage des durch das Druckermittlungssystem (300) ermittelten transpulmonalen Drucks (Ptp) ausgebildet ist; und
wobei die Beatmungssteuerung (180) auch zur automatisierten Ansteuerung des Ösophagusballonsteuersystems (400) ausgebildet ist und/oder das Ösophagusballonsteuersystem (400) auch zur automatisierten Ansteuerung der Beatmungssteuerung (180) ausgebildet ist;
**dadurch gekennzeichnet, dass**
die Beatmungssteuerung (180) derart konfiguriert ist, dass sie ab einem vorbestimmten Zeitpunkt vor Aktivierung oder Beginn einer Ösophagusballonsteuerprozedur (100, 500, 600) für vorbestimmte Beatmungsparameter (192A, 192B, 192C, 192D) und/oder Beatmungsmodi (190A, 190B, 190C, 190D) keine Veränderung zulässt, bis die Ösophagusballonsteuerprozedur (100, 500, 600) beendet ist oder nicht innerhalb einer vorbestimmten Zeit begonnen hat; und/oder
wobei die Beatmungssteuerung (180) und/oder das Ösophagusballonsteuersystem (400) derart konfiguriert ist, dass eine Ösophagusballonsteuerprozedur (100, 500, 600) zu einem oder mehreren vorbestimmten Zeitpunkten gestartet wird, wobei dann, wenn innerhalb einer vorbestimmten Zeitdauer vor Beginn einer Ösophagusballonsteuerprozedur (100, 500, 600) eine Änderung eines vorbestimmten Beatmungsparameters (192A, 192B, 192C, 192D) und/oder Beatmungsmodus (190A, 190B, 190C, 190D) erfolgen soll, die Beatmungssteuerung (180) und/oder das Ösophagusballonsteuersystem (400) dazu konfiguriert ist, den Beginn der Ösophagusballonsteuerprozedur (100, 500, 600) solange zu verschieben, bis die Änderung des Beatmungsparameters (192A, 192B, 192C, 192D) und/oder des Beatmungsmodus (190A, 190B, 190C, 190D) abgeschlossen ist, und/oder wobei die Beatmungssteuerung (180) und/oder das Ösophagusballonsteuersystem (400) derart konfiguriert ist, dass eine Änderung eines vorbestimmten Beatmungsparameters (192A, 192B, 192C, 192D) und/oder Beatmungsmodus (190A, 190B, 190C, 190D), die nach Aktivierung oder Beginn einer Ösophagusbal-Ionsteuerprozedur (100, 500, 600) erfolgen soll, verschoben wird, bis die Ösophagusballonsteuerprozedur (100, 500, 600) abgeschlossen ist, und/ oder
wobei die Beatmungssteuerung (180) und/oder das Ösophagusballonsteuersystem (400) derart konfiguriert ist, dass eine Ösophagusballonsteuerprozedur (100, 500, 600) nach Maßgabe von vorbestimmten Zeitintervallen gestartet wird, wobei dann, wenn innerhalb einer vorbestimmten Zeitdauer vor Beginn einer Ösophagusballonsteuerprozedur (100, 500, 600) eine aufschiebbare Änderung eines vorbestimmten Beatmungsparameters (192A, 192B, 192C, 192D) und/oder Beatmungsmodus (190A, 190B, 190C, 190D) erfolgen soll, die Beatmungssteuerung (180) so konfiguriert ist, dass sie den Beginn der Änderung des Beatmungsparameters (192A, 192B, 192C, 192D) und/oder des Beatmungsmodus (190A, 190B, 190C, 190D) solange verschiebt, bis die Ösophagusballonsteuerprozedur (100, 500, 600) abgeschlossen ist.

2. Vorrichtung (15) nach Anspruch 1,
wobei die Beatmungssteuerung (180) zur Steuerung/Regelung des Ösophagusballonsteuersystems (400) nach Maßgabe von durch das Druckermittlungssystem (300) gelieferten Druckwerten ausgebildet ist und/oder
wobei das Ösophagusballonsteuersystem (400) derart konfiguriert ist, dass sie nach Maßgabe von durch die Beatmungssteuerung (180) gelieferten Steuerbefehlen Ösophagusballonsteuerprozeduren (100, 500, 600) abarbeitet und/oder
wobei das Ösophagusballonsteuersystem (400) dazu ausgebildet ist, vorbestimmte Ösophagusballonsteuersignale an die Beatmungssteuerung (180) zu senden, und die Beatmungssteuerung (180) derart konfiguriert ist, dass sie vorbestimmte Beatmungsparameter (192A, 192B, 192C, 192D) und/oder Beatmungsmodi (190A, 190B, 190C, 190D) nach Maßgabe dieser Ösophagusballonsteuersignale einstellt/steuert/regelt/anzeigt.,

3. Vorrichtung (15) nach Anspruch 1 oder 2, wobei die Beatmungssteuerung (180) und/oder das Ösophagusballonsteuersystem (400) derart konfiguriert ist, dass eine Warnung erzeugt wird, wenn ab einem vorbestimmten Zeitpunkt vor dem Beginn einer Ösophagusballonsteuerprozedur (100, 500, 600) eine Veränderung vorbestimmter Beatmungsparameter (192A, 192B, 192C, 192D) und/oder Beatmungsmodi (190A, 190B, 190C, 190D) erzwungen wird, wobei die Veränderung insbesondere durch entsprechende Benutzereingaben erzwungen wird.

4. Vorrichtung (15) nach einem der Ansprüche 1 bis 3, wobei das Druckermittlungssystem (300) eine zur Bestimmung des Ösophagusballondrucks ausgelegte Sensorik aufweist, wobei die Sensorik insbesondere einen in den Ösophagus (34) einführbaren Ösophagusballonkatheter (45) mit Ballonsonde (46) und eine Vorrichtung zum Erfassen eines Drucks in der Ballonsonde (46) in vivo umfasst, und/oder
wobei das Ösophagusballonsteuersystem (400) eine Anordnung (430), insbesondere eine Pumpanordnung, zum Befüllen der Ballonsonde (46) mit einem Messfluid und/oder zum Entnehmen von Messfluid aus der Ballonsonde (46), nachdem die Ballonsonde (46) im Ösophagus (34) platziert worden ist, umfasst, und/oder
wobei das Ösophagusballonsteuersystem (400) zum schrittweisen Verändern einer jeweils eingestellten Menge an Messfluid in der Ballonsonde (46) ausgebildet ist, wobei das Ösophagusballonsteuersystem (400) insbesondere derart ausgebildet ist, dass es die Menge an Messfluid in der Ballonsonde (46) in einem Schritt oder in mehreren Schritten verändert und bei jeder der auf diese Weise schrittweise als Messpunkte (S1, S2, M1, M2,..., M7, E1, E2, E3) eingestellten Mengen an Messfluid in der Ballonsonde (46) einen Ösophagusballondruck (Pesoi) erfasst und der jeweils eingestellten Menge (Vi) an Messfluid in der Ballonsonde (46) zuordnet.

5. Vorrichtung (15) nach einem der Ansprüche 1 bis 4, wobei das Ösophagusballonsteuersystem (400) und/oder die Beatmungssteuerung (180) wenigstens eines aus einer Füllmengenüberwachung (470), einem Fluidbefüllungssystem mit einer Befüllungssteuerung (490) und einer Kalibriersteuerung (450) umfasst und wobei jedenfalls für den Fall, dass das Ösophagusballonsteuersystem (400) das wenigstens eine aus einem Füllmengenüberwachung (470), einer Befüllungssteuerung (490) und einer Kalibriersteuerung (450) umfasst, die Beatmungssteuerung (180) zu bidirektionaler Interaktion mit der wenigstens einen aus der Füllmengenüberwachung (470), der Befüllungssteuerung (490) und der Kalibriersteuerung (450) ausgebildet ist, derart dass die Beatmungssteuerung (180) das Ösophagusballonsteuersystem (400) zum Start von vorbestimmten Fluidmengen-Überwachungsprozeduren (500), Fluid-Befüllungsprozeduren (600) und/oder Kalibrierprozeduren (100) ansteuert und/oder den Ablauf von Fluidmengen-Überwachungsprozeduren (500), Fluid-Befüllungsprozeduren (600) und/oder Kalibrierprozeduren (100) steuert, und/oder
wobei die Beatmungssteuerung (180) und/oder das Ösophagusballonsteuersystem (400) in Reaktion auf eine Änderung eines der folgenden Parameter um mehr als einen vorbestimmten Wert den Start einer Kalibrierprozedur (100), einer Fluidmengen-Überwachungsprozedur (500) und/oder einer Fluid-Befüllungsprozedur (600) veranlasst:
(i) Sollwert-Änderungen: Änderung des positiven endexpiratorischen Drucks (PEEP), Änderung des vorgegebenen maximalen Atemwegsdrucks (paw-max), Änderung des Tidalvolumens (Vt), Änderung des Tidalhubs (IE), Änderung der Beatmungsrate (BR);
(ii) Istwert-Änderungen: endexpiratorischer Druck (Pes_ee), Differenz zwischen endinspiratorischem Druck und endexpiratorischem Druck (Pes_ei-Pes_ee), Ösophagus (34)-Druckschwankung pro Atemzug, Nachgiebigkeit der Thoraxwand (chest wall compliance, Ccw = Vt/(Pes_ei-Pes_ee), und/ oder
wobei das Ösophagusballonsteuersystem (400) eine Kalibriersteuerung (450) umfasst, die derart ausgebildet ist, dass sie die Menge an Messfluid in der Ballonsonde (46) schrittweise verändert, wobei die Kalibriersteuerung (450) bei jeder der auf diese Weise schrittweise als Messpunkte (S1, S2, M1, M2,..., M7, E1, E2, E3) eingestellten Mengen (Vᵢ) an Messfluid in der Ballonsonde (46) einen von dem Drucksensor erfassten Ösophagusballondruck (Pesoᵢ) aufnimmt und der jeweils eingestellten Menge an Messfluid in der Ballonsonde (46) zuordnet, und/oder
wobei das Ösophagusballonsteuersystem ein Befüllungssteuerung (490) umfasst, das derart ausgebildet ist, dass sie nach Ansteuerung eine Fluid-Befüllungsprozedur (600) zum Befüllen der Ballonsonde (46) mit einer vorbestimmten Menge an Messfluid durchführt.

6. Vorrichtung (15) nach Anspruch 5, wobei die Befüllungssteuerung (490) derart ausgebildet ist, dass sie vor dem Start der Fluid-Befüllungsprozedur zum Befüllen der Ballonsonde (46) eine Entleerungsprozedur (610) zur Entnahme von in der Ballonsonde (46) befindlichem Messfluid durchführt, und/oder
wobei die Entleerungsprozedur (610) eine Prozedur (620a, 620b) zur Einstellung eines Nullvolumens (V_{Null}) umfasst, und/oder
wobei die Prozedur (620a) zur Einstellung eines Nullvolumens (V_{Null}) Folgendes umfasst:
(i) Entnehmen (621a) von Messfluid aus der Ballonsonde (46), bis ein erster vorbestimmter Unterdruck (up,) in der Ballonsonde (46) erreicht ist;
(ii) Einleiten (622a) von Messfluid in die Ballonsonde (46), bis ein zweiter vorbestimmter Unterdruck (up₂) in der Ballonsonde (46) erreicht ist, der größer ist als der erste vorbestimmte Unterdruck (up₁), und
(iii) Einleiten (623a) eines vorbestimmten Volumens an Messfluid in die Ballonsonde (46), und/oder
wobei die Befüllungssteuerung insbesondere derart ausgebildet ist, dass sie nach der Entleerungsprozedur (610) zur Entnahme von in der Ballonsonde (46) befindlichem Messfluid eine Prozedur (630) zum Einleiten von Messfluid in die Ballonsonde (46) durchführt, bis ein vorbestimmter Überdruck (op) in der Ballonsonde (46) erreicht ist, und/oder
wobei das Ösophagusballonsteuersystem (400) dazu ausgebildet ist, einen Basis-Ösophagusballondruck (Peso_{base}) zu erfassen und die Befüllungssteuerung zur Durchführung der Fluid-Befüllungsprozedur (600) zum Befüllen der Ballonsonde (46) mit Messfluid anzusteuern, wenn der Basis-Ösophagusballondruck (Peso_{base}) um einen vorbestimmten Betrag oder mehr absinkt.

7. Vorrichtung (15) nach Anspruch 5 oder 6, wobei das Ösophagusballonsteuersystem (400) eine Fluidmengenüberwachung (470) umfasst, die derart ausgebildet ist, dass sie nach Ansteuerung eine Fluidmengen-Überwachungsprozedur (500) zur Überprüfung einer gewünschten Befüllung an Messfluid in der Ballonsonde (46) durchführt.

8. Vorrichtung (15) nach Anspruch 7, wobei die Fluidmengenüberwachung (470) dazu ausgebildet ist, nach Start der Prozedur zur Überprüfung einer gewünschten Befüllung an Messfluid in der Ballonsonde (46) die vorhandene Menge an Messfluid in der Ballonsonde (46) schrittweise zu verändern, bei einer jeweiligen auf diese Weise als Vergleichs-Messpunkt eingestellten Vergleichsmenge an Messfluid in der Ballonsonde (46) einen zugehörigen Vergleichs-Ösophagusballondruck zu erfassen und der jeweiligen Vergleichsmenge an Messfluid in der Ballonsonde (46) zuzuordnen, und/oder
wobei die Fluidmengenüberwachung (470) nach Start der Fluidmengen-Überwachungsprozedur (500) zur Überprüfung einer gewünschten Befüllung an Messfluid in der Ballonsonde (46) die vorhandene Menge (V₀) an Messfluid in der Ballonsonde (46) auf eine erste als erster Vergleichs-Messpunkt (M₁) eingestellte Vergleichsmenge (V₁) an Messfluid verändert, die größer ist als die vorhandene Menge (V₀) an Messfluid, und/oder
wobei die Fluidmengenüberwachung insbesondere nach Start der Fluidmengen-Überwachungsprozedur (500) zur Überprüfung einer gewünschten Befüllung an Messfluid in der Ballonsonde (46) die vorhandene Menge (V₀) an Messfluid in der Ballonsonde (46) auf eine zweite als Vergleichs-Messpunkt (M₂) eingestellte Vergleichsmenge (V₂) an Messfluid verändert, die kleiner ist als die vorhandene Menge (V₀) an Messfluid, wobei die Fluidmengenüberwachung insbesondere feststellt, dass die vorhandene Menge an Messfluid in der Ballonsonde (46) der gewünschten Menge an Messfluid in der Ballonsonde (46) entspricht, wenn der bei der ersten Vergleichsmenge (V₁) an Messfluid erfasste Vergleichs-Ösophagusballondruck (Peso₁) und der bei der zweiten Vergleichsmenge (V₂) an Messfluid in der Ballonsonde (46) erfasste Vergleichs-Ösophagusballondruck (Peso₂) innerhalb eines vorbestimmten Bereichs um den der vorhandenen Menge an Messfluid in der Ballonsonde (46) zugeordneten Ösophagusballondruck (Peso₀) liegt, und/oder
wobei die Fluidmengenüberwachung feststellt, dass die vorhandene Menge an Messfluid in der Ballonsonde (46) der gewünschten Menge an Messfluid in der Ballonsonde (46) entspricht, wenn ein erster Gradient (grad,), der durch den bei der ersten Vergleichsmenge (V₁) an Messfluid erfassten ersten Vergleichs-Ösophagusballondruck (Peso₁) und den bei der vorhandenen Menge (V₀) an Messfluid in der Ballonsonde (46) erfassten Ösophagusballondruck (Peso₀) definiert ist, und ein zweiter Gradient (grad₂), der durch den bei der zweiten Vergleichsmenge (V₂) an Messfluid erfassten Ösophagusballondruck (Peso₂) und den bei der vorhandenen Menge (V₀) an Messfluid in der Ballonsonde (46) erfassten Ösophagusballondruck (Peso₀) definiert ist, einen vorbestimmten ersten Schwellenwert (gradₗᵢₘᵢₜ₁) nicht überschreitet.

9. Vorrichtung (15) nach Anspruch 7 oder 8, wobei die Füllmengenüberwachung (470) derart ausgebildet ist, dass sie die Befüllungssteuerung (490) des Fluidbefüllungssystems zur Durchführung der Fluid-Befüllungsprozedur (600) zum Befüllen der Ballonsonde (46) mit einer vorbestimmten Menge an Messfluid nach einem der Ansprüche 5 oder 6 ansteuert, wenn die Fluidmengenüberwachung (470) feststellt, dass wenigstens einer von dem ersten Gradienten (grad₁) und dem zweiten Gradienten (grad2) größer als der erste Schwellenwert (gradₗᵢₘᵢₜ₁) ist, wobei eine neue vorbestimmte Befüllung eingestellt wird, die gegenüber der vorhandenen Menge (V₀) an Messfluid in der Ballonsonde (46) in Richtung zu der derjenigen Befüllung aus erster und zweiter Vergleichsmenge (V₁, V₂) verschoben ist, für die der größere der beiden Gradienten (grad₁, grad₂) bestimmt worden ist, und/oder
wobei die Kalibriersteuerung (450) dazu ausgebildet ist, eine Kalibrierprozedur (100) nach Anspruch 16 durchzuführen, wenn die Fluidmengenüberwachung (470) festgestellt hat, dass wenigstens einer von dem erstem Gradienten (grad₁) und dem zweitem Gradienten (grad₂) größer als ein zweiter Schwellenwert (gradₗᵢₘᵢₜ₂) ist, wobei der zweite Schwellenwert (gradₗᵢₘᵢₜ₂) größer als der erste Schwellenwert (gradₗᵢₘᵢₜ₁) ist, und/oder
wobei das Ösophagusballonsteuersystem (400) dazu ausgebildet ist, in Reaktion auf eine Veränderung eines Beatmungsparameters (192A, 192B, 192C, 192D) die Fluidmengenüberwachung (470) zum Durchführen einer Fluidmengen-Überwachungsprozedur (500) zur Überprüfung einer vorbestimmten Befüllung an Messfluid in der Ballonsonde (46) anzusteuern.

10. Beatmungsvorrichtung (10), aufweisend ein Atemgassystem (200), das dazu vorgesehen und ausgebildet ist, zur Beatmung eines Patienten Atemgas unter einem Überdruck in die Lunge (28, 30) des Patienten zu leiten, und eine Vorrichtung (15) nach einem der vorangehenden Ansprüche.

## Claims

1. A device (15) for automated adjustment of a ventilation parameter (192A, 192B, 192C, 192D) specified by a ventilation device (10), in particular a pressure, especially a positive end-expiratory pressure (PEEP) and/or a maximum airway pressure (Paw_max), and/or for automated display of information relevant for mechanical ventilation, in particular a transpulmonary pressure (Ptp), comprising
- a pressure ascertaining system (300) for ascertaining a transpulmonary pressure (Ptp), in particular a transpulmonary pressure (Ptp_ee) at the end of an expiration phase and/or a transpulmonary pressure (Ptp_ei) at the end of an inspiration phase, on the basis of an esophageal balloon catheter (45) which can be introduced into the esophagus (34) and has a balloon probe (46), for ascertaining an esophageal balloon pressure (Peso);
- an esophageal balloon control system (400) having a flow sensor (410) and a pump arrangement (430) for automated monitoring and/or adjustment of an operational filling of the balloon probe (46) of the esophageal balloon catheter (45) in vivo, wherein the operational filling of the balloon probe (46) is a filling of the balloon probe (46) with measurement fluid at which the balloon probe (46) delivers an optimum signal for sensing the oesophageal balloon pressure (Peso); and
- a ventilation controller (180),
wherein the ventilation controller (180) is configured to adjust the ventilation parameter (192A, 192B, 192C, 192D) specified by the ventilation device (10) and/or to display the information relevant for mechanical ventilation on the basis of the transpulmonary pressure (Ptp) ascertained by the pressure ascertaining system (300); and
wherein the ventilation controller (180) is also configured to automatically control the esophageal balloon control system (400) and/or the esophageal balloon control system (400) is also configured to automatically control the ventilation controller (180),
**characterized in that** the ventilation controller (180) is configured such that, from a predetermined time prior to activation or commencement of an esophageal balloon control procedure (100, 500, 600), it does not permit any changes to predetermined ventilation parameters (192A, 192B, 192C, 192D) and/or ventilation modes (190A, 190B, 190C, 190D) until the esophageal balloon control procedure (100, 500, 600) has ended or has not commenced within a predetermined time; and/or
wherein the ventilation controller (180) and/or the esophageal balloon control system (400) is configured such that an esophageal balloon control procedure (100, 500, 600) is started at one or more predetermined times, wherein, if a change in a predetermined ventilation parameter (192A, 192B, 192C, 192D) and/or ventilation mode (190A, 190B, 190C, 190D) is to occur within a predetermined time period before the commencement of an esophageal balloon control procedure (100, 500, 600), the ventilation controller (180) and/or the esophageal balloon control system (400) is configured to postpone the commencement of the esophageal balloon control procedure (100, 500, 600) until the change in the ventilation parameter (192A, 192B, 192C, 192D) and/or the ventilation mode (190A, 190B, 190C, 190D) is completed, and/or
wherein the ventilation controller (180) and/or the esophageal balloon control system (400) is configured such that a change in a predetermined ventilation parameter (192A, 192B, 192C, 192D) and/or ventilation mode (190A, 190B, 190C, 190D), which is to take place after activation or commencement of an esophageal balloon control procedure (100, 500, 600), is postponed until the esophageal balloon control procedure (100, 500, 600) is completed, and/or
wherein the ventilation controller (180) and/or the esophageal balloon control system (400) is configured such that an esophageal balloon control procedure (100, 500, 600) is started according to predetermined time intervals, wherein, if a deferrable change in a predetermined ventilation parameter (192A, 192B, 192C, 192D) and/or ventilation mode (190A, 190B, 190C, 190D) is to take place within a predetermined time period before the commencement of an esophageal balloon control procedure (100, 500, 600), the ventilation controller (180) is configured such that it delays the commencement of the change in the ventilation parameter (192A, 192B, 192C, 192D) and/or the ventilation mode (190A, 190B, 190C, 190D) until the esophageal balloon control procedure (100, 500, 600) is completed.

2. A device (15) according to claim 1,
wherein the ventilation controller (180) is configured to control/regulate the esophageal balloon control system (400) according to pressure values supplied by the pressure detection system (300), and/or
wherein the esophageal balloon control system (400) is configured to execute esophageal balloon control procedures (100, 500, 600) according to control commands supplied by the ventilation controller (180), and/or
wherein the esophageal balloon control system (400) is configured to send predetermined esophageal balloon control signals to the ventilation controller (180), and the ventilation controller (180) is configured to adjust/control/regulate/display predetermined ventilation parameters (192A, 192B, 192C, 192D) and/or ventilation modes (190A, 190B, 190C, 190D) according to these esophageal balloon control signals.

3. A device (15) according to claim 1 or 2,
wherein the ventilation controller (180) and/or the esophageal balloon control system (400) is configured such that a warning is generated if, from a predetermined time before the commencement of an esophageal balloon control procedure (100, 500, 600), a change in predetermined ventilation parameters (192A, 192B, 192C, 192D) and/or ventilation modes (190A, 190B, 190C, 190D) is enforced, with the change being enforced in particular by corresponding user input.

4. A device (15) according to any of claims 1 to 3,
wherein the pressure ascertaining system (300) has a sensor system designed to determine the esophageal balloon pressure, wherein the sensor system in particular comprises an esophageal balloon catheter (45) with balloon probe (46) that can be introduced into the esophagus (34) and a device for detecting a pressure in the balloon probe (46) in vivo, and/or
wherein the esophageal balloon control system (400) comprises an arrangement (430), in particular a pump arrangement, for filling the balloon probe (46) with a measuring fluid and/or for removing measuring fluid from the balloon probe (46) after the balloon probe (46) has been placed in the esophagus (34), and/or
wherein the esophageal balloon control system (400) is configured for stepwise changing a respectively set quantity of measuring fluid in the balloon probe (46), wherein the esophageal balloon control system (400) is in particular configured such that it changes the quantity of measuring fluid in the balloon probe (46) in one step or in several steps and, at each of the quantities of measuring fluid in the balloon probe (46) set stepwise in this way as measuring points (S1, S2, M1, M2,..., M7, E1, E2, E3), detects an esophageal balloon pressure (Pesoi) and assigns the same to the respectively set quantity (Vi) of measuring fluid in the balloon probe (46).

5. A device (15) according to any of claims 1 to 4,
wherein the esophageal balloon control system (400) and/or the ventilation controller (180) comprises at least one of a filling quantity monitoring system (470), a fluid filling system with a filling control (490) and a calibration control (450), and wherein, in any case in which the esophageal balloon control system (400) comprises the at least one of a filling quantity monitoring system (470), a filling control (490) and a calibration control (450), the ventilation controller (180) is configured for bidirectional interaction with the at least one of the filling quantity monitoring system (470), the filling control (490) and the calibration control (450) such that the ventilation controller (180) controls the esophageal balloon control system (400) to start predetermined fluid volume monitoring procedures (500), fluid filling procedures (600) and/or calibration procedures (100) and/or controls the execution of fluid volume monitoring procedures (500), fluid filling procedures (600) and/or calibration procedures (100), and/or
wherein the ventilation controller (180) and/or the esophageal balloon control system (400) initiates the commencement of a calibration procedure (100), a fluid volume monitoring procedure (500) and/or a fluid filling procedure (600) in response to a change in one of the following parameters by more than a predetermined value:
(i) setpoint changes: change in positive end-expiratory pressure (PEEP), change in preset maximum airway pressure (paw-max), change in tidal volume (Vt), change in tidal stroke (IE), change in ventilation rate (BR);
(ii) actual value changes: end-expiratory pressure (Pes_ee), difference between end-inspiratory pressure and end-expiratory pressure (Pes_ei-Pes_ee), esophageal (34) pressure fluctuation per breath, chest wall compliance (Ccw = Vt/(Pes_ei-Pes_ee), and/or
wherein the esophageal balloon control system (400) comprises a calibration control (450) configured to stepwise change the quantity of measuring fluid in the balloon probe (46), wherein the calibration control (450), at each of the quantities (Vᵢ) of measuring fluid in the balloon probe (46) set stepwise in this way as measuring points (S1, S2, M1, M2,..., M7, E1, E2, E3), records an esophageal balloon pressure (Peso) detected by the pressure sensor and assigns the same to the respectively set quantity of measuring fluid in the balloon probe (46), and/or
wherein the esophageal balloon control system comprises a filling control (490) configured such that, upon activation, it performs a fluid filling procedure (600) to fill the balloon probe (46) with a predetermined quantity of measuring fluid.

6. A device (15) according to claim 5,
wherein the filling control (490) is configured such that, prior to the start of the fluid filling procedure for filling the balloon probe (46), it performs an emptying procedure (610) to remove measuring fluid present in the balloon probe (46), and/or
wherein the emptying procedure (610) comprises a procedure (620a, 620b) for setting a zero volume (Vₙᵤₗₗ), and/or
wherein the procedure (620a) for setting a zero volume (V_{Null}) comprises the following:
(i) removing (621a) measuring fluid from the balloon probe (46) until a first predetermined negative pressure (up₁) is reached in the balloon probe (46);
(ii) introducing (622a) measuring fluid into the balloon probe (46) until a second predetermined negative pressure (up₂) is reached in the balloon probe (46) which is greater than the first predetermined negative pressure (up₁), and
(iii) introducing (623a) a predetermined volume of measuring fluid into the balloon probe (46), and/or
wherein the filling control is designed in particular such that, after the emptying procedure (610) for removing measuring fluid present in the balloon probe (46), it performs a procedure (630) for introducing measuring fluid into the balloon probe (46) until a predetermined positive pressure (op) is reached in the balloon probe (46), and/or
wherein the esophageal balloon control system (400) is configured to detect a base esophageal balloon pressure (Peso_{base}) and to control the filling control for carrying out the fluid filling procedure (600) to fill the balloon probe (46) with measuring fluid when the base esophageal balloon pressure (Peso_{base}) drops by a predetermined quantity or more.

7. A device (15) according to claim 5 or 6,
wherein wherein the esophageal balloon control system (400) comprises a fluid quantity monitoring system (470) which is designed such that, after activation, it performs a fluid quantity monitoring procedure (500) to check a desired filling of measuring fluid in the balloon probe (46).

8. A device (15) according to claim 7,
wherein the fluid quantity monitoring system (470) is configured for stepwise changing the existing quantity of measuring fluid in the balloon probe (46) after the start of the procedure for checking a desired filling of measuring fluid in the balloon probe (46), to detect an associated comparative esophageal balloon pressure at a respective comparative quantity of measuring fluid in the balloon probe (46) set in this way as a comparative measuring point and to assign the same to the respective comparative quantity of measuring fluid in the balloon probe (46), and/or
wherein the fluid quantity monitoring system (470), after the start of the fluid quantity monitoring procedure (500) to check a desired filling of measuring fluid in the balloon probe (46), changes the existing quantity (V₀) of measuring fluid in the balloon probe (46) to a first comparative quantity (V₁) of measuring fluid set as first comparative measuring point (M₁), which is larger than the existing quantity (V₀) of measuring fluid, and/or
wherein the fluid quantity monitoring system, in particular after the start of the fluid quantity monitoring procedure (500) to check a desired filling of measuring fluid in the balloon probe (46), changes the existing quantity (V₀) of measuring fluid in the balloon probe (46) to a second comparative quantity (V₂) of measuring fluid set as a comparative measuring point (M₂), which is smaller than the existing quantity (V₀) of measuring fluid, wherein the fluid quantity monitoring system in particular determines that the existing quantity of measuring fluid in the balloon probe (46) corresponds to the desired quantity of measuring fluid in the balloon probe (46) when the comparative esophageal balloon pressure (Peso₁) recorded at the first comparative quantity (V₁) of measuring fluid and the comparative esophageal balloon pressure (Peso₂) recorded at the second comparative quantity (V₂) of measuring fluid in the balloon probe (46) lie within a predetermined range around the esophageal balloon pressure (Peso₀) assigned to the existing quantity of measuring fluid in the balloon probe (46), and/or
wherein the fluid quantity monitoring system determines that the quantity of measuring fluid present in the balloon probe (46) corresponds to the desired quantity of measuring fluid in the balloon probe (46) when a first gradient (grad₁) defined by the first comparative esophageal balloon pressure (Peso₁) recorded at the first comparative quantity (V₁) of measuring fluid and the esophageal balloon pressure (Peso₀) recorded at the existing quantity (V₀) of measuring fluid in the balloon probe (46), and a second gradient (grad₂) defined by the esophageal balloon pressure (Peso₂) recorded at the second comparative quantity (V₂) of measuring fluid and the esophageal balloon pressure (Peso₀) recorded at the existing quantity (V₀) of measuring fluid in the balloon probe (46), do not exceed a predetermined first threshold (gradₗᵢₘᵢₜ₁).

9. A device (15) according to claim 7 or 8,
wherein the filling quantity monitoring system (470) is configured such that it controls the filling control (490) of the fluid filling system for carrying out the fluid filling procedure (600) for filling the balloon probe (46) with a predetermined quantity of measuring fluid according to any of claims 5 or 6, when the fluid quantity monitoring system (470) determines that at least one of the first gradient (grad₁) and the second gradient (grad₂) is greater than the first threshold value (gradₗᵢₘᵢₜ₁), wherein a new predetermined filling is set which, compared to the existing quantity (V₀) of measuring fluid in the balloon probe (46), is shifted in the direction towards that filling of the first and second comparative quantity (V₁, V₂) for which the larger of the two gradients (grad₁, grad₂) has been determined, and/or
wherein the calibration control (450) is configured to perform a calibration procedure (100) according to claim 16 when the fluid quantity monitoring system (470) has determined that at least one of the first gradient (grad₁) and the second gradient (grad₂) is greater than a second threshold value (gradₗᵢₘᵢₜ₂), wherein the second threshold value (gradₗᵢₘᵢₜ₂) is greater than the first threshold (gradₗᵢₘᵢₜ₁), and/or
wherein the esophageal balloon control system (400) is configured to control the fluid quantity monitoring system (470) in response to a change in a ventilation parameter (192A, 192B, 192C, 192D) to perform a fluid quantity monitoring procedure (500) to check a predetermined filling of measuring fluid in the balloon probe (46).

10. A ventilation device (10) comprising a breathing gas system (200) that it configured and designed to supply breathing gas at a positive pressure to the lungs (28, 30) of a patient for ventilation of the patient, and a device (15) according to any of the preceding claims.

## Revendications

1. Dispositif (15) pour le réglage automatisé d'un paramètre d'assistance respiratoire (192A, 192B, 192C, 192D) prédéfini par un dispositif d'assistance respiratoire (10), notamment d'une pression, notamment d'une pression expiratoire positive (PEP) et/ou d'une pression maximale des voies respiratoires (Paw_max), et/ou pour la représentation automatisée d'informations relevant de la respiration artificielle, en particulier d'une pression transpulmonaire (Ptp), comprenant :
- un système de mesure de pression (300) destiné à mesurer une pression transpulmonaire (Ptp), en particulier une pression transpulmonaire (Ptp_ee) à la fin d'une phase d'expiration et/ou une pression transpulmonaire (Ptp_ei) à la fin d'une phase d'inspiration, sur la base d'un cathéter à ballonnet œsophagien (45) pouvant être introduit dans l'œsophage (34) et muni d'une sonde à ballonnet (46) pour déterminer une pression du ballonnet œsophagien (Peso) ;
- un système de commande de ballonnet œsophagien (400) comprenant un capteur de débit (410) et un dispositif de pompage (430) destiné à la surveillance et/ou au réglage automatisés d'un remplissage conforme aux spécifications de la sonde à ballonnet (46) du cathéter à ballonnet œsophagien (45) in vivo, le remplissage conforme aux spécifications de la sonde à ballonnet (46) consistant à remplir la sonde à ballonnet (46) avec un fluide de mesure de manière à ce que la sonde à ballonnet (46) fournisse un signal optimal pour la détection de la pression du ballonnet œsophagien (Peso) ; et
- une commande d'assistance respiratoire (180),
la commande d'assistance respiratoire (180) étant réalisée de manière à régler le paramètre d'assistance respiratoire (192A, 192B, 192C, 192D) prédéfini par le dispositif d'assistance respiratoire (10) et/ou à représenter les informations relevant de la respiration artificielle sur la base de la pression transpulmonaire (Ptp) déterminée par le système de mesure de pression (300) ; et
la commande d'assistance respiratoire (180) étant également conçue pour commander automatiquement le système de commande du ballonnet œsophagien (400) et/ou le ballonnet œsophagien (400) étant également conçu pour commander automatiquement la commande d'assistance respiratoire (180) ;
**caractérisé en ce que**
la commande d'assistance respiratoire (180) est configurée de telle sorte qu'à partir d'un moment prédéfini avant l'activation ou le début d'une procédure de commande du ballonnet œsophagien (100, 500, 600), elle ne permet aucune modification des paramètres d'assistance respiratoire (192A, 192B, 192C, 192D) et/ou des modes d'assistance respiratoire (190A, 190B, 190C, 190D) prédéfinis jusqu'à ce que la procédure de commande du ballonnet œsophagien (100, 500, 600) soit terminée ou n'ait pas débuté dans un délai prédéfini ; et/ou
dans lequel la commande d'assistance respiratoire (180) et/ou le système de commande du ballonnet œsophagien (400) sont configurés de telle sorte qu'une procédure de commande du ballonnet œsophagien (100, 500, 600) est lancée à un ou plusieurs moments prédéfinis, sachant que si, au cours d'un intervalle de temps prédéfini avant le début d'une procédure de commande du ballonnet œsophagien (100, 500, 600), une modification d'un paramètre d'assistance respiratoire (192A, 192B, 192C, 192D) et/ou d'un mode d'assistance respiratoire (190A, 190B, 190C, 190D) prédéfinis doit avoir lieu, la commande d'assistance respiratoire (180) et/ou le système de commande du ballonnet œsophagien (400) sont configurés de manière à reporter le début de la procédure de commande du ballonnet œsophagien (100, 500, 600) jusqu'à ce que la modification du paramètre d'assistance respiratoire (192A, 192B, 192C, 192D) et/ou du mode d'assistance respiratoire (190A, 190B, 190C, 190D) soit achevée, et/ou dans lequel la commande d'assistance respiratoire (180) et/ou le système de commande du ballonnet œsophagien (400) sont configurés de telle sorte qu'une modification d'un paramètre d'assistance respiratoire (192A, 192B, 192C, 192D) et/ou d'un mode d'assistance respiratoire (190A, 190B, 190C, 190D) prédéfinis qui doit avoir lieu après l'activation ou le lancement d'une procédure de commande du ballonnet œsophagien (100, 500, 600), est reportée jusqu'à ce que la procédure de commande du ballonnet œsophagien (100, 500, 600) soit achevée, et/ou
dans lequel la commande d'assistance respiratoire (180) et/ou le système de commande du ballonnet œsophagien (400) sont configurés de telle sorte qu'une procédure de commande du ballonnet œsophagien (100, 500, 600) est lancée selon des intervalles de temps prédéfinis, sachant que si,
au cours d'un intervalle de temps prédéfini avant le début d'une procédure de commande du ballonnet œsophagien (100, 500, 600), une modification reportable d'un paramètre d'assistance respiratoire (192A, 192B, 192C, 192D) et/ou d'un mode d'assistance respiratoire (190A, 190B, 190C, 190D) prédéfinis doit avoir lieu, la commande d'assistance respiratoire (180) est configurée de manière à reporter le début de la modification du paramètre d'assistance respiratoire (192A, 192B, 192C, 192D) et/ou du mode d'assistance respiratoire (190A, 190B, 190C, 190D) jusqu'à ce que la procédure de commande du ballonnet œsophagien (100, 500, 600) soit achevée.

2. Le dispositif (15) selon la revendication 1,
dans lequel la commande d'assistance respiratoire (180) est conçue pour commander/régler le système de commande du ballonnet œsophagien (400) selon des valeurs de pression fournies par le système de mesure de pression (300) et/ou
dans lequel le système de commande du ballonnet œsophagien (400) est configuré de manière à exécuter des procédures de commande du ballonnet œsophagien (100, 500, 600) conformément aux instructions de commande fournies par la commande d'assistance respiratoire (180) et/ou
dans lequel le système de commande du ballonnet œsophagien (400) est conçu de manière à envoyer des signaux de commande prédéfinis du ballonnet œsophagien à la commande d'assistance respiratoire (180), et où la commande d'assistance respiratoire (180) est configurée de manière à configurer/commander/régler/afficher des paramètres d'assistance respiratoire (192A, 192B, 192C, 192D) et/ou des modes d'assistance respiratoire (190A, 190B, 190C, 190D) prédéfinis en fonction de ces signaux de commande du ballonnet œsophagien.

3. Le dispositif (15) selon la revendication 1 ou 2, dans lequel la commande d'assistance respiratoire (180) et/ou le système de commande du ballonnet œsophagien (400) sont configurés de manière à générer un avertissement lorsque, à partir d'un moment prédéfini avant le début d'une procédure de commande du ballonnet œsophagien (100, 500, 600), une modification de paramètres d'assistance respiratoire (192A, 192B, 192C, 192D) et/ou de modes d'assistance respiratoire (190A, 190B, 190C, 190D) est déclenchée,
la modification étant en particulier déclenchée par des saisies correspondantes de l'utilisateur.

4. Le dispositif (15) selon l'une quelconque des revendications 1 à 3, dans lequel le système de mesure de pression (300) comporte un système de capteurs conçu pour déterminer la pression du ballonnet œsophagien, ledit système de capteurs comprenant notamment un cathéter à ballonnet œsophagien (45) pouvant être introduit dans l'œsophage (34) et muni d'une sonde à ballonnet (46), ainsi qu'un dispositif permettant de mesurer in vivo la pression dans la sonde à ballonnet (46), et/ou
dans lequel le système de commande du ballonnet œsophagien (400) comprend un dispositif (430), en particulier un dispositif de pompage, destiné à remplir la sonde à ballonnet (46) d'un fluide de mesure et/ou à enlever du fluide de mesure de la sonde à ballonnet (46), une fois que la sonde à ballonnet (46) a été mise en place dans l'œsophage (34), et/ou
dans lequel le système de commande du ballonnet œsophagien (400) est conçu pour modifier progressivement une quantité de fluide de mesure configurée dans la sonde à ballonnet (46), le système de commande du ballonnet œsophagien (400) étant notamment conçu de manière à modifier la quantité de fluide de mesure dans la sonde à ballonnet (46) en une ou plusieurs étapes et, pour chacune des quantités de fluide de mesure ainsi configurées par étapes en tant que points de mesure (S1, S2, M1, M2, ..., M7, E1, E2, E3) dans la sonde à ballonnet (46), à saisir une pression du ballonnet œsophagien (Pesoᵢ) et à l'associer à la quantité respective (Vᵢ) de fluide de mesure configurée dans la sonde à ballonnet (46).

5. Le dispositif (15) selon l'une quelconque des revendications 1 à 4, dans lequel le système de commande du ballonnet œsophagien (400) et/ou la commande d'assistance respiratoire (180) comprennent au moins l'un des éléments suivants : un système de surveillance du volume de remplissage (470), un système de remplissage de fluide comprenant une commande de remplissage (490) et une commande d'étalonnage (450), et dans lequel, en tout cas pour le cas où le système de commande du ballonnet œsophagien (400) comprend au moins un système de surveillance du volume de remplissage (470), une commande de remplissage (490) ou une commande d'étalonnage (450), la commande d'assistance respiratoire (180) est conçue pour interagir de manière bidirectionnelle
avec au moins l'un des éléments parmi le dispositif de surveillance du volume de remplissage (470), la commande de remplissage (490) ou la commande d'étalonnage (450), de telle sorte que la commande d'assistance respiratoire (180) déclenche sur le système de commande du ballonnet œsophagien (400) le lancement de procédures de surveillance de la quantité de fluide (500), de procédures de remplissage de fluide (600) et/ou de procédures d'étalonnage (100) prédéfinies, et/ou commande le déroulement de procédures de surveillance de la quantité de fluide (500), de procédures de remplissage de fluide (600) et/ou de procédures d'étalonnage (100), et/ou
dans lequel la commande d'assistance respiratoire (180) et/ou le système de commande du ballonnet œsophagien (400) déclenchent, en réponse à une variation supérieure à une valeur prédéfinie de l'un des paramètres suivants, le lancement d'une procédure d'étalonnage (100), d'une procédure de surveillance de la quantité de fluide (500) et/ou d'une procédure de remplissage de fluide (600) :
(i) Modifications des valeurs de consigne : modification de la pression expiratoire positive (PEP), modification de la pression maximale prédéfinie des voies respiratoires (paw-max), modification du volume courant (Vₜ), modification du rapport I/E, modification de la fréquence respiratoire (FR) ;
(ii) Modifications des valeurs réelles : pression expiratoire (Pes_ee), différence entre la pression inspiratoire finale et la pression expiratoire finale (Pes_ei-Pes_ee), variation de la pression œsophagienne (34) par cycle respiratoire, compliance de la paroi thoracique (chest wall compliance, Ccw = Vt/(Pes_ei-Pes_ee), et/ou
dans lequel le système de commande du ballonnet œsophagien (400) comprend une commande d'étalonnage (450) conçue pour modifier progressivement la quantité de fluide de mesure dans la sonde à ballonnet (46), la commande d'étalonnage (450) enregistrant, pour chacune des quantités (Vᵢ) de fluide de mesure dans la sonde à ballonnet (46) ainsi réglées progressivement en tant que points de mesure (S1, S2, M1, M2, ..., M7, E1, E2, E3), une pression du ballonnet œsophagien (Pesoᵢ) détectée par le capteur de pression et l'associe à la quantité de fluide de mesure respectivement réglée dans la sonde à ballonnet (46), et/ou
dans lequel le système de commande du ballonnet œsophagien comprend une commande de remplissage (490) conçue pour exécuter,
sur commande, une procédure de remplissage de fluide (600) afin de remplir la sonde à ballonnet (46) d'une quantité prédéfinie de fluide de mesure.

6. Le dispositif (15) selon la revendication 5, dans lequel la commande de remplissage (490) est conçue de manière à exécuter, avant le lancement de la procédure de remplissage de fluide destinée à remplir la sonde à ballonnet (46), une procédure de vidange (610) visant à extraire le fluide de mesure se trouvant dans la sonde à ballonnet (46), et/ou
dans lequel la procédure de vidange (610) comprend une procédure (620a, 620b) de réglage d'un volume zéro (V_{Zéro}), et/ou
dans lequel la procédure (620a) de réglage d'un volume zéro (V_{Zéro}) comprend les étapes suivantes :
(i) extraction (621a) de fluide de mesure de la sonde à ballonnet (46) jusqu'à l'obtention d'une première dépression prédéfinie (up₁) dans la sonde à ballonnet (46) ;
(ii) introduction (622a) de fluide de mesure dans la sonde à ballonnet (46) jusqu'à l'obtention d'une deuxième dépression prédéfinie (up₂) dans la sonde à ballonnet (46), qui est supérieure à la première dépression prédéfinie (up₁), et
(iii) introduction (623a) d'un volume prédéfini de fluide de mesure dans la sonde à ballonnet (46), et/ou
dans lequel la commande de remplissage est notamment conçue de telle sorte qu'après la procédure de vidange (610) visant à extraire le fluide de mesure se trouvant dans la sonde à ballonnet (46), elle exécute une procédure (630) visant à introduire du fluide de mesure dans la sonde à ballonnet (46) jusqu'à ce qu'une surpression prédéfinie (op) soit atteinte dans la sonde à ballonnet (46), et/ou
dans lequel le système de commande du ballonnet oesophagien (400) est conçu de manière à détecter une pression de base du ballonnet œsophagien (Peso_{base}) et à amener la commande le remplissage à exécuter la procédure de remplissage de fluide (600) visant à remplir la sonde à ballonnet (46) de fluide de mesure lorsque la pression de base du ballonnet oesophagien (Peso_{base}) diminue d'une valeur prédéfinie ou plus.

7. Le dispositif (15) selon la revendication 5 ou 6, dans lequel le système de commande du ballonnet œsophagien (400) comprend un dispositif de surveillance de la quantité de fluide (470) conçu pour exécuter, une fois activé, une procédure de surveillance de la quantité de fluide (500) visant à vérifier que la sonde à ballonnet (46) est remplie de la quantité souhaitée de fluide de mesure.

8. Le dispositif (15) selon la revendication 7, dans lequel le système de surveillance de la quantité de fluide (470) est conçu de manière à modifier progressivement, après le lancement de la procédure de vérification d'un remplissage souhaité en fluide de mesure dans la sonde à ballonnet (46), la quantité de fluide de mesure présente dans la sonde à ballonnet (46), puis à enregistrer, pour chaque quantité de référence de fluide de mesure ainsi définie dans la sonde à ballonnet (46), une pression de référence correspondante du ballonnet œsophagien, et à associer cette pression à la quantité de référence respective de fluide de mesure dans la sonde à ballonnet (46), et/ou
dans lequel le système de surveillance de la quantité de fluide (470), après le lancement de la procédure de surveillance de la quantité de fluide (500) visant à vérifier un remplissage souhaité en fluide de mesure dans la sonde à ballonnet (46), modifie la quantité présente (V₀) de fluide de mesure dans la sonde à ballonnet (46) pour l'amener à une première quantité de référence (V₁) de fluide de mesure, définie comme premier point de référence (M₁), qui est supérieure à la quantité présente (V₀) de fluide de mesure, et/ou
dans lequel le système de surveillance de la quantité de fluide, après le lancement de la procédure de surveillance de la quantité de fluide (500) visant à vérifier un remplissage souhaité en fluide de mesure dans la sonde à ballonnet (46), modifie en particulier la quantité présente (V₀) de fluide de mesure dans la sonde à ballonnet (46) pour l'amener à une deuxième quantité de référence (V₂) de fluide de mesure, définie comme deuxième point de référence (M₂), qui est inférieure à la quantité présente (V₀) de fluide de mesure, le système de surveillance de la quantité de fluide constatant notamment que la quantité de fluide de mesure présente dans la sonde à ballonnet (46) correspond à la quantité souhaitée de fluide de mesure dans la sonde à ballonnet (46) lorsque la pression de référence du ballonnet œsophagien (Peso₁) enregistrée pour la première quantité de référence (V₁) de fluide de mesure et la pression de référence du ballonnet œsophagien (Peso₂) enregistrée pour la deuxième quantité de référence (V₂) de fluide de mesure dans la sonde à ballonnet (46) se situent dans une
plage prédéfinie autour de la pression du ballonnet œsophagien (Peso₀) associée à la quantité de fluide de mesure présente dans la sonde à ballonnet (46), et/ou
dans lequel le système de surveillance de la quantité de fluide constate que la quantité de fluide de mesure présente dans la sonde à ballonnet (46) correspond à la quantité souhaitée de fluide de mesure dans la sonde à ballonnet (46) lorsqu'un premier gradient (grad₁), défini par la première pression de référence du ballonnet œsophagien (Peso₁) mesurée pour la première quantité de référence (V₁) de fluide de mesure et la pression du ballonnet œsophagien (Peso₀) mesurée pour la quantité présente (V₀) de fluide de mesure dans la sonde à ballonnet (46), et un deuxième gradient (grad₂), défini par la pression du ballonnet œsophagien (Peso₂) mesurée pour la deuxième quantité de référence (V₂) de fluide de mesure et la pression du ballonnet œsophagien (Peso₀) mesurée pour la quantité présente (V₀) de fluide de mesure dans la sonde à ballonnet (46), ne dépasse pas une première valeur seuil prédéfinie (gradₗᵢₘᵢₜ₁).

9. Le dispositif (15) selon la revendication 7 ou 8, dans lequel le système de surveillance du volume de remplissage (470) est conçu de telle sorte qu'il dirige la commande de remplissage (490) du système de remplissage de fluide pour exécuter la procédure de remplissage de fluide (600) visant à remplir la sonde à ballonnet (46) d'une quantité prédéfinie de fluide de mesure selon l'une quelconque des revendications 5 ou 6, lorsque le système de surveillance de la quantité de fluide (470) constate qu'au moins l'un du premier gradient (grad₁) ou du deuxième gradient (grad₂) est supérieur à la première valeur seuil (gradₗᵢₘᵢₜ₁), une nouvelle quantité de remplissage prédéfinie étant alors définie, qui est décalée par rapport à la quantité présente (V₀) de fluide de mesure dans la sonde à ballonnet (46) en direction de celle des première et deuxième quantités de référence (V₁, V₂) pour laquelle le plus grand des deux gradients (grad₁, grad₂) a été défini, et/ou
dans lequel la commande d'étalonnage (450) est conçue de manière à exécuter une procédure d'étalonnage (100) selon la revendication 16 lorsque le système de surveillance de la quantité de fluide (470) a constaté qu'au moins l'un du premier gradient (grad₁) ou du deuxième gradient (grad₂) est supérieur à une deuxième valeur seuil (gradₗᵢₘᵢₜ₂), la deuxième valeur seuil (gradₗᵢₘᵢₜₑ) étant supérieure à la première valeur seuil (gradₗᵢₘᵢₜ₁), et/ou
le système de commande du ballonnet œsophagien (400) est conçu de manière à commander, en réponse à une modification d'un paramètre d'assistance respiratoire (192A, 192B, 192C, 192D), le système de surveillance de la quantité de fluide (470) afin qu'il exécute une procédure de surveillance de la quantité de fluide (500) visant à vérifier un niveau de remplissage prédéfini de fluide de mesure dans la sonde à ballonnet (46).

10. Dispositif d'assistance respiratoire (10) comprenant un système de gaz respiratoire (200) prévu et conçu pour acheminer du gaz respiratoire en surpression vers les poumons (28, 30) d'un patient, en vue de l'assistance respiratoire du patient, et dispositif (15) selon l'une quelconque des revendications précédentes.
